# EUROPEAN PATENT SPECIFICATION

(11) **EP 1 934 377 B1**
(45) Date of publication and mention of the grant of the patent: **07.03.2012**
(21) Application number: 06814191.0
(22) Date of filing: 05.09.2006
(51) Int. Cl.: C12Q 1/68, G01N 33/53, G01N 33/574

(54) **METHODS FOR IDENTIFYING BIOMARKERS USEFUL IN DIAGNOSIS OF BIOLOGICAL STATES**
VERFAHREN ZUR IDENTIFIZIERUNG VON BIOMARKERN MIT EIGNUNG BEI DER DIAGNOSE BIOLOGISCHER ZUSTÄNDE
PROCÉDÉS PERMETTANT D'IDENTIFIER DES BIOMARQUEURS UTILES AU DIAGNOSTIC D'ÉTATS BIOLOGIQUES

(30) Priority: 02.09.2005 US 713628 P; 02.09.2005 US 713629 P; 02.09.2005 US 714138 P
(43) Date of publication of application: 25.06.2008
(73) Proprietor: The University of Toledo, Toledo, OH 43614 (US)
(72) Inventor: WILLEY, James, C., Toledo, OH 43614 (US); CRAWFORD, Erin, L., Rossford, OH 43460 (US); MULLINS, D'Anna, N., Maumee, OH 43537 (US)
(74) Representative: Vossius & Partner
(86) International application number: PCT/US2006/034594
(87) International publication number: WO 2007/028161

(56) References cited:
- EP-A- 1 304 377
- WO-A1-95/24223
- US-A1- 2001 051 344
- US-A1- 2002 032 319
- US-A1- 2002 044 941
- US-A1- 2002 044 941
- US-A1- 2005 048 480
- PHELPS MONIKA ET AL: "p53-independent activation of the hdm2-P2 promoter through multiple transcription factor response elements results in elevated hdm2 expression in estrogen receptor alpha-positive breast cancer cells." CANCER RESEARCH 15 MAY 2003, vol. 63, no. 10, 15 May 2003 (2003-05-15), pages 2616-2623, XP002518536 ISSN: 0008-5472
- YAN ZHEN ET AL: "Highly coordinated gene regulation in mouse skeletal muscle regeneration." THE JOURNAL OF BIOLOGICAL CHEMISTRY 7 MAR 2003, vol. 278, no. 10, 7 March 2003 (2003-03-07), pages 8826-8836, XP002518537 ISSN: 0021-9258
- VOLM M. ET AL.: 'Prognostic significance of the expression of c-fos, c-jun and c-erbB-1 oncogene products in human squamous cell lung carcinomas' J. CANCER RES. CLIN. ONCOL. vol. 119, 1993, pages 507 - 510, XP008126641
- WILLEY J C ET AL: "Expression measurement of many genes simultaneously by quantitative RT-PCR using standardized mixtures of competitive templates.", AMERICAN JOURNAL OF RESPIRATORY CELL AND MOLECULAR BIOLOGY JUL 1998 LNKD- PUBMED:9651175, vol. 19, no. 1, July 1998 (1998-07), pages 6-17, ISSN: 1044-1549
- WEAVER DAVID A ET AL: "ABCC5, ERCC2, XPA and XRCC1 transcript abundance levels correlate with cisplatin chemoresistance in non-small cell lung cancer cell lines", MOLECULAR CANCER, BIOMED CENTRAL, LONDON, GB, vol. 4, no. 1, 9 May 2005 (2005-05-09), page 18, XP021008239, ISSN: 1476-4598, DOI: DOI:10.1186/1476-4598-4-18

## Description

### BACKGROUND OF THE INVENTION

Assessing the correlation between a particular variation in DNA sequence, or polymorphism, and risk for a particular condition has been a dominant paradigm for many years. A common limitation of such studies, however, is that they involve assessment of a single polymorphism or occasionally, a few polymorphisms. Further, although the polymorphism assessed typically resides within a gene associated with a particular biological state, the selection of a polymorphism for study can be largely empiric, e.g., not being based on known function. As multiple infrequent polymorphisms at different sites may all contribute to risk, and key polymorphisms may not have been identified through functional tests, a statistically valid assessment may require very large study populations, so large as to be impractical. Thus, there remains a need for new approaches to identify biomarkers that can diagnose undesirable conditions and serve as therapeutic targets. Bronchogenic carcinoma (BC) is an example of such a condition. BC is the leading cause of cancer-related death in the United States, causing 28% of all cancer deaths. While cigarette smoking is the primary risk factor, only some heavy smokers acquire the disease. Due to the employment of increasingly effective methods to reduce the prevalence of cigarette smoking (Samet, 1991) there are increasing numbers of ex-smokers in the population. Importantly, although the risk of BC decreases over time following smoking cessation, it never reaches the level observed among those who never smoked (Halpern et al, 1993; Lubin and Blot, 1993; Sobue et al, 1993) and BC now occurs most commonly among ex-smokers (Strauss et al, 1994; Tong et al, 1996). Because BC usually is advanced and not amenable to surgery at the time of diagnosis and it is poorly responsive to any therapy other than surgical removal, the rate of cure is very low (Sekido, 2001). Thus, in this context, there have been multiple efforts over the years to develop means for preventing BC in ex-smokers or to detect it early enough to enable surgical cure (Cohen and Khuri, 2003; Hong and Spom, 1997), such as screening with regular chest X-rays or sputum analysis (Fontana et al, 1986; Tockman, 2000). Based on data obtained thus far, these promising early detection or chemoprevention approaches would not be cost effective (Wagner and Ruckdeschel, 1996). Screening with helical CT appears to be more promising and a long-term study is underway (Jett, J.R., 2005; Mulshine, 2005).

One important way to improve the efficacy of early screening and chemoprevention studies will be to include in the study population a higher fraction of the individuals at highest risk (Greenwald, 1996; Kelloff et al, 1996). Presently, such studies generally include individuals over the age of 50 who have smoked an average of one pack of cigarettes per day for more than 20 years. Yet, although cigarette smoking is the primary risk factor for BC, only 5-10% of heavy (greater than 20 pack years) smokers develop BC. It is possible to identify a sub-population with somewhat higher risk for BC by including those with greater age and heavier smoking history who started smoking at a younger age, but the incidence of BC among this smaller group rises only to a maximum of 15-20% (Bach et al, 2003).

Cigarette smoking is also the primary cause of other pulmonary conditions such as chronic obstructive pulmonary disease (COPD). COPD is one of the most common chronic conditions and the fourth leading cause of death in the United States. Identifying those at greater risk for BC and/or COPD can enhance development of methods and compositions for early detection, as well as methods and compositions for treating and/or preventing the disease. The instant disclosure relates to such methods and compositions for identifying individuals at risk for BC and/or COPD, as well as other biological states, including e.g., other cancer and/or other lung-related conditions.

### BRIEF DESCRIPTION OF THE INVENTION

A first aspect is a method of identifying a transcription factor biomarker that indicates a biological state, comprising assaying in a plurality of control samples expression levels of a transcription factor and of a first gene, said first gene being associated with said biological state; assaying in a plurality of case samples expression levels of said transcription factor and of said.first gene; and deducing whether said expression levels of said transcription factor are correlated with said expression levels of said first gene in said control samples but not correlated in said case samples, thereby identifying a transcription factor biomarker for said biological state.

A second aspect is a method of identifying a biomarker that indicates a biological state, comprising assaying in a plurality of control samples expression levels of a gene and of a first transcription factor, said first transcription factor being associated with said biological state; assaying in a plurality of case samples expression levels of said gene and of said first transcription factor; and deducing whether said expression levels of said gene are correlated with said expression levels of said first transcription factor in said control samples but not correlated in said case samples, thereby identifying a biomarker for said biological state.

A third aspect is a method of identifying a polymorphism that indicates a biological state, comprising obtaining a plurality of control samples wherein expression levels of a transcription factor are correlated with expression levels of a gene; obtaining a plurality of case samples wherein expression levels of said transcription factor are not correlated with expression levels of said gene; and identifying a nucleotide variation in said transcription factor and/or in said gene in one or more of said case samples compared with one or more of said control samples, thereby identifying a polymorphism that indicates said biological state.

### BRIEF DESCRIPTION OF THE FIGURES

The novel features of the invention are set forth with particularity in the appended claims.

Figure 1 illustrates the overall process for identifying biomarkers.

Figure 2 illustrates the overall process for diagnosing a biological state.

Figure 3(a-1) illustrates correlation of each of 6 TFs ((a) CEBPB, (b) CEBPG, (c) E2F1, (d) E2F3, (e) E2F6, (t) EVI1) with each of 5 genes XRCC1, ERCC5, GSTP1, SOD1, or GPX1; and (g- h) illustrate CEBPG/XRCC1 data of Figure 3b presented as scatter plots for (g) NBCI and (h) BCI.

Figure 4 (a-b) illustrates bivariate analysis between CEBPG with XRCC1 in (a) NBCI and (b) BCI.

Figure 5 illustrates the lack of correlation of CEBPB with XRCC1 in either NBCI or BCI.

Figure 6 illustrates a schematic bivariate analysis of a TG/CEBPG expression levels in one NBCI (NBCI1) and 5 BCI (BCI₁₋₅).

Figure 7 illustrates Scatter plot representation of bivariatecorrelation of CEBPG with ERCC5 in the NBEC of non-BC (diamonds) or BC (squares) individuals. Regression line represents linear correlation of data from non-BC individuals. Outliers are circled.

Figure 8 illustrates ERCC5 promoter region cloned into pGL2-Basic luciferase vector (Promega Corp.. Madison, WI); A five hundred eighty-nine base pair section of the ERCC5 promotor region originally analyzed for transcription factor binding sites was cloned between XhoI and HindIII restriction enzyme sites of the pGL2-Basic vector.

Figure 9 illustrates Western blot analysis of transfected H23 cells. H23 cells transfected with CEBPG and/or CEBPB or empty vector were analyzed for the presence of CEBPG. 10 µg of lysate from each condition were loaded. Total protein concentration was determined colorimetrically using the Bio-Rad Protein Assay (Bio-Rad Laboratories, Inc., Hercules, CA). Purified CEBPG protein was used as a positive control (Abnova.)

Figure 10 illustrates analysis of endogenous ERCC regulation by transfecting truncated ERCC5-Luc into 2 NSCLC lines.

### DETAILED DESCRIPTION OF THE INVENTION

The present disclosure relates to methods and compositions for identifying biomarkers that indicate a biological state, in particular transcription factor biomarkers and genes that can be regulated by such transcription factor biomarkers. The disclosure also relates to identifying polymorphisms in such transcription factors and regulated genes indicative of the biological state. The biomarkers and polymorphisms identified find use in diagnostic and treatment approaches, e.g., in some examples the disclosure provides methods and kits for detecting bronchogenic carcinoma and risks thereof.

### 1. Methods and Compositions for Identifying Biomarkers

### A. Lack of Correlation Approach

In one aspect, the disclosure relates to methods for identifying biomarkers that indicate a biological state. In some examples, the method involves identifying lack of correlation between expression levels of a transcription factor and another gene in a given biological state. In some examples, the other gene is a gene known to be associated with a given biological state and the method involves identifying new transcription factor biomarkers. In some examples, the transcription factor is known to be associated with a given biological state and the method involves identifying new biomarkers that are other genes.

A "biological state" as used herein can refer to any phenotypic state, for e.g., a clinically relevant phenotype or other metabolic condition of interest. Biological states can include, e.g., a disease phenotype, a predisposition to a disease state or a non-disease state; a therapeutic drug response or predisposition to such a response, an adverse drug response (e.g. drug toxicity) or a predisposition to such a response, a resistance to a drug, or a predisposition to showing such a resistance, etc. In some examples, the drug may be and anti-tumor drug.

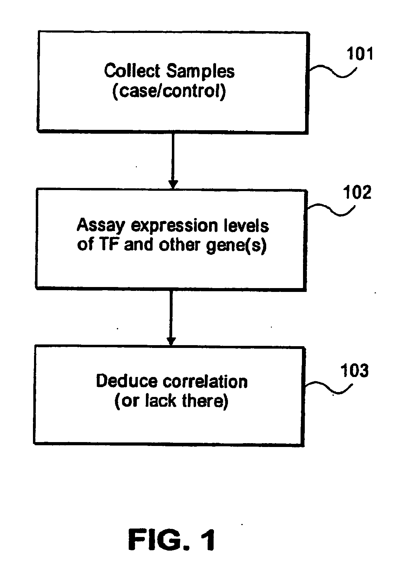

Figure 1 illustrates the overall process for identifying biomarkers in some examples disclosed herein. At step 101, a representative sample set of case samples and control samples are collected. The control samples are samples that correspond to a particular normal biological state. For example, a control sample may be obtained from an individual that exhibits a particular normal state. For example, the control sample may be obtained from the normal bronchial epithelium of a patient with low risk for bronchogenic carcinoma or COPD. Conversely, a case sample may be obtained from the normal bronchial epithelium of a patient at high risk for bronchogenic carcinoma or COPD and therefore has a biological state that does not correspond to the biological state observed in control individuals who are at low risk. Alternatively, a control sample may be obtained from a cancer tissue with a biological state that corresponds to lack of response to a drug, while a case sample may be obtained from a cancer tissue with a biological state that corresponds to response to the drug.

In some examples, a plurality of case samples and control samples are used. A plurality refers to, e.g., 2 or more. Preferably more than about 10 case and more than about 10 control samples are collected for use. Preferably more than about 20 case samples and more than about 20 control samples, preferably more than about 50 case samples and more than about 50 control samples, preferably more than about 100 case samples and more than about 100 control samples are collected for use.

Case/control samples can include, e.g., a swab of culture, a brush of epithelial cells, a pinch of tissue, a biopsy extraction, or a vial of a biological fluid. Tissue can include, e.g., organs, tumors, lymph nodes, arteries, aggregates of cells and/or individual cells, e.g. Biological fluids can include, e.g., saliva, tears, mucus, lymph fluids, sputum, stool, pleural fluid, pericardial fluid, lung aspirates, exudates, peritoneal fluid, plasma, blood, serum, white blood cells, cerebral spinal fluid, synovial fluid, amniotic fluid, milk, semen, urine, and the like, as well as cell suspensions, cell cultures, or cell culture supernatants. Samples may be crude samples or processed samples, e.g., obtained after various processing or preparation steps. For example, various cell separation methods, e.g., magnetically activated cell sorting, may be applied to separate or enrich analytes of interest in a biological fluid, such as blood. A sample may also comprise a dilution, e.g., diluted serum or dilutions of other complex and/or protein-rich mixtures. Preferred examples of the present disclosure can be practiced using small starting materials to yield quantifiable results.

At step 102, expression levels of a transcription factor and at least one other gene are assayed. The expression levels can be determined by measuring abundance of a nucleic acid transcript and/or protein translation product using any techniques known in the art. For example, in some embodiments, expression levels are assayed by assaying abundance of an mRNA transcript. In preferred examples, transcript levels are assayed using one or more methods described in U.S. Patent Nos. 5,639,606; U.S. 5,643,765; U.S. 5,876,978; U.S. Patent Application Serial No. 11/072,700; and U.S. Provisional Application Serial No. 60/646,157.

For example, assaying mRNA transcript abundance comprises measuring a nucleic acid corresponding to a transcription factor relative to its competitive template; co-measuring a nucleic acid corresponding to another gene with its competitive template; and obtaining a relation comparing values obtained from the co-measurements. The nucleic acid corresponding to the transcription factor (or other gene) can refer to an mRNA transcript of the transcription factor (or other gene) or a cDNA obtained from the mRNA. The relation obtained can be a comparison of values for the transcription factor, its competitive template, the other gene, and its competitive template. In preferred examples the transcription factor and/or other gene is measured relative to a reference nucleic acid, e.g., as described in U.S. Patent Application Serial Nos. 11/072,700 and 11/103,397.

This may entail co-amplifying a nucleic acid corresponding to a transcription factor with its competitive template; co-amplifying a nucleic acid corresponding to another gene with its competitive template; and obtaining a
relation comparing amplified products obtained from the co-amplifications. The nucleic acid corresponding to the transcription factor (or other gene) can refer to an mRNA transcript of the transcription factor (or other gene) or a cDNA obtained from the mRNA. The relation obtained can be a comparison of amplified amounts of the transcription factor, its competitive template, the other gene, and its competitive template. In preferred embodiments, the transcription factor and/or other gene is measured relative to a reference nucleic acid, e.g., as described in U.S. Patent Application Serial Nos. 11/072,700 and 11/103,397. Alternatively, co-measurement may involve amplifying signal from each nucleic acid and corresponding internal standard through binding of a sequence-specific probes, such as those used in branched chain-amplification.

At least one of the other nucleic acids being analyzed can serve as the reference nucleic acid. "Reference nucleic acid" as used herein can refer to a nucleic acid that is amplified as well the nucleic acid to be analyzed. The nucleic acid can be "normalized" to a reference nucleic acid. In some examples, the reference nucleic acid serves as a control for loading, e.g., to control for cDNA loaded into the reaction. For example, , the reference nucleic acid comprises a nucleic acid that is not expected to vary (or to vary significantly) among given biological specimen and/or in response to certain stimuli. For example, mRNA from a constitutively expressed gene may provide the reference nucleic acid. In some examples, known or potential housekeeping genes may provide the reference nucleic acid, including but not limited to human, mouse and/or rat glyceraldehydes-3-phospate dehydrogenase (GAPD or GAPDH), β-actin, 28S RNA, 18S RNA, and/or other ribonuclear protein genes. Other housekeeping genes that have been used as internal standards in Northern analyses of gene expression may also be used. See, e.g., Devereux et al., Nucleic Acids Res. 12:387 (1984); Barbu et al., Nucleic Acids Res. 17:7115 (1989). In some examples, a competitive template for a reference nucleic acid may comprise a nucleic acid having a sequence similar to either strand of cDNA of a housekeeping gene, but having a distinguishable feature as described above.

Many different genes can provide reference nucleic acids. The choice of reference nucleic acid may depend on the tissues to be assayed and/or the biological states being studied. For example, β-action varies little among different normal bronchial epithelial cell samples (see, e.g., Crawford, E. L., Khuder, S. A., Durham, S. J., et al. (2000) Normal bronchial epithelial cell expression of glutathione transferase P1, glutathione transferase M3, and glutathione peroxidase is low in subjects with bronchogenic carcinoma. Cancer Res. 60, 1609-1618), but it may vary over about 100-fold in samples from different tissues, such as bronchial epithelial cells compared to lymphocytes. In some examples, the reference nucleic acid corresponds to a gene that is expressed in all or nearly all or the majority of all tissues; and/or is expressed at a high, substantially high or relatively high level

In some examples, the competitive templates are provided in a standardized mixture. A "standardized mixture" as used herein can refer to a mixture comprising a number of internal standards, e.g., a number of competitive templates. In still some examples, a series of serially-diluted standardized mixtures is used to assay analytes in a mixture. "Serially-diluted standardized mixtures" can refer to two or more standardized mixtures in which one or more of the reagents in the standardized mixtures is serially-diluted. In some examples, one or more reagents in the standardized mixtures is serially-diluted relative to a different one or more of the reagents in the mixtures. For example, the series of standardized mixtures can provide competitive template for a transcription factor at a series of known concentrations relative to competitive template for another gene. Preparation and use of standardized mixtures are described in U.S. Patent Application Serial Nos. 11/072,700 and 11/103,397.

Other methods for assaying mRNA transcript abundance can also be used. For example, real-time RT-PCR and/or hybridization assays can be used in some embodiments. For example, specific oligonucleotide probes for the relevant transcription factors and other genes can be used in hybridization techniques, as is known in the art. Any hybridization format for determining specific RNA levels can be used, including but not limited to Northern blots, slot blots, dot blots, and hybridization to oligonucleotide arrays, micro-arrays and other solid-phase approaches. Specificity of hybridization can be assessed by varying degrees of stringency of the hybridization conditions.

In some examples, expression levels are assayed by assaying abundance of a protein. To assess specific translation product (protein) expression levels, antibodies specific for the protein can be used readily. Again, any format known in the art for measuring specific protein levels can be used, including sandwich assays, ELISAs, immunoprecipitations, and Western blots. Any of monoclonal antibodies, polyclonal antibodies, single chain antibodies, and antibody fragments may be used in such assays.

Further, in some examples, methods provided in U.S. Patent Application Serial No. 11/103,397 can be used. The patent application describes standardized immuno-PCR methods and compositions that can be used to measure protein copy number, protein-DNA hybrids, and/or protein-protein hybrids. Briefly, , internal standards can be used that comprise a known number of molecules of antigen (e.g. transcription factor protein) hybridized in equimolar amount to a highly specific, high affinity monoclonal antibody that in turn is covalently bound to a double stranded DNA molecule that serves as a template for PCR. A known quantity of internal standard for each of multiple genes can be combined in a standardized mixture of internal standards (SMIS). Due to the signal amplification power of PCR, a 1 mg batch of this SMIS can serve the world's needs for 5-10 years.

At step 103, correlation or lack thereof is deduced. That is, the method involves deducing whether or not expression levels of the transcription factor are correlated with expression levels of the other gene in control and/or case samples. In some examples, transcription factor expression levels represent the total amount of both wild type and mutant transcription factor transcripts. Where the biological state of interest is a disease state, e.g., a cancer-related condition, expression levels of the transcription factor and the other gene generally are correlated in control samples but not correlated in case samples.

Those of skill in the art will recognize that more than one transcription faction and/or other genes can be assayed. For example, in searching for a transcription factor biomarker, the expression levels of one or more additional genes associated with a biological state can be assayed. In searching for other genes (putatively regulated genes) that can serve as biomarkers, the expression levels of one or more transcription factors associated with a biological state can be assayed.

"Correlated" can refer to positive or negative correlation, preferably positive correlation. A correlation can be based on statistical significance, e.g, using one of tests described the Examples. Conversely, "not correlated" can be based on a lack statistical significance, e.g., a lack of statistically significant correlation between expression level of a transcription factor and expression level of at least one other gene in case samples. "Not correlated," "lack of correlation" and other grammatical variations thereof, will refer to a lesser or reduced degree of correlation between the expression levels of two genes, e.g., in case samples compared to controls, e.g., a low or relatively low correlation. By detecting loss of correlation, a new biomarker can be identified. For example, where a gene is known to be associated with a given biological state, loss of correlation between expression levels of the gene and a given transcription factor in case samples can identify the transcription factor as a biomarker for the alternative biological state. As another example, where a transcription factor is known to be associated with a given biological state, loss of correlation between expression levels of the transcription factor and a given gene in case samples can identify the gene as a biomarker for the alternative biological state.

Without being limited to a particular theory or hypothesis, the loss of correlation in a disease state, e.g., in a cancer-related condition, may indicate loss of functional regulation of the gene by the transcription factor. "Transcription factor" or "TF" as used herein can refer to a gene or gene product that can influence the level of expression of another gene or gene product. In some examples, a transcription factor is a nucleic acid binding protein, e.g., a protein that can bind regulatory elements of other genes. Transcription factors can include, e.g., trans-acting factors, e.g., proteins that bind to cis-regulatory elements (eg. an enhancer or a TATA box) and thereby, directly or indirectly, affect the initiation of transcription. Common transcription factors include eukaryotic proteins that aid RNA polymerase to recognize promoters, as well as prokaryotic sigma factors. Transcription factors can activate and/or repress gene expression, resulting in up- or down-regulation.

Generally, the transcription factor regulates a given gene in control samples but not in case samples. Such genes may be referred to as "normally-regulated genes" or "putatively regulated genes," and grammatically similar variations and can also be referred to as "target genes" (TG). Regulation may be direct or indirect by various mechanistic bases. Methods of the instant disclosure facilitate exploration of various mechanistic bases, as described in the Examples below.

According to the paradigm used in this study, a) a normal phenotype results from regulated transcription of a group of genes by one or more TFs, b) the corresponding risk-conferring or disease phenotype results from sub-optimal interaction among those same genes, and c) each phenotype is identifiable and distinguishable by assaying expression levels. Accordingly, methods and compositions provided herein involve quantifying a) regulated transcription of a group of genes by one or more TFs that is associated with a normal phenotype, b) sub-optimal interaction among those same genes that is responsible for corresponding risk-conferring or disease phenotype, and c) using an expression level profile that identifies the normal from diseased or at-risk phenotype. The data presented here support the utility of this paradigm in identifying genes associated with risk for BC, as provided below.

### Biomarkers for Bronchogenic Carcinoma and other Cancer-related conditions

Transcription factor biomarkers can be identified for bronchogenic carcinoma (BC). Genes associated with BC include antioxidant (AO) and DNA repair (DNAR) genes. Such genes are expressed in the progenitor cells for BC, normal bronchial epithelial cells (NBEC), and are believed to protect against harmful effects of cigarette smoke (Willey JC, et al, American Journal of Respiratory Cell and Molecular Biology, 19, 16-24, 1998). Inherited inter-individual variation in function of these genes has been shown to play a role in determining risk for BC (Spitz MR, Wei Q, Dong Q, Amos CI, Wu X, Cancer Epidemiol Biomarkers Prev., 12, 689-98, 2003). For example, transcript abundance of AO genes may be lower in NBEC of bronchogenic carcinoma individuals (BCI) compared to non-BCI (NBCI), suggesting that BCI are selected on the basis of poor antioxidant protection (Crawford, E.L. et al, Cancer Research, 60, 1609-1618, 2000). In the Crawford study, for example, there was a tendency towards correlation in transcript abundance between several pairs of AO or DNAR genes in NBCI, but not in BCI. Gene pairs included in that observation were GSTP1/GPX1, CAT/GPX3, and GPX3/SOD1.

Without being limited to a particular hypothesis and/or theory, there may be inter-individual variation in regulation of such key AO and DNAR genes by one or more TFs and individuals with sub-optimal regulation may be selected for development of BC if they are smokers. Inter-individual variation in risk for a disease that does not display a familial pattern, e.g., can be explained in that an individual must be heterozygous or homozygous for a risk bearing allele at a threshold number of genes from a group of genes that have redundant function in protecting cells from DNA damage. This may explain why only a fraction of smokers develop BC or other cancer-related and/or lung- related conditions. For example, genetic risk for BC may be inversely proportional to coordinate regulation of AO and DNAR genes in NBECs.

"Smokers" as used herein includes individuals who use or have used one or more products associated with conditions of the lung, including, e.g., tobacco products, such as cigarettes and/or chewing tobacco, as well as individuals who are or have been exposed to such products second-hand, such as being exposed to second-hand smoke. Smokers can include heavy smokers and light smokers or a range in between. For example, smokers include those who smoke 1 cigarette/day, 5 cigarettes/day, a pack of cigarettes/day or more. In some examples, individuals that are likely to have maximal difference in genetically determined risk can be compared. For example, case samples can be obtained from younger, light smokers or non-smokers who develop BC; while control samples can be obtained from older, heavy smokers without BC. Other factors considered can include individual airway anatomy, type of cigarette, inhalation technique, function of the cilia and mucosal cells in the bronchial epithelium, and intermittent chronic bronchitis exacerbations. Identified biomarkers can indicate BC, risk ofBC, extent of BC (e.g., metastasizing or non-metastasizing) and/or prognosis (e.g., likelihood and/or degree of responsiveness to a particular chemotherapy).

The methods provided herein show that transcript abundance of CEBPG transcription factor is significantly (p < 0.01) correlated with key antioxidant (AO) or DNA repair (DNAR) genes in NBEC of NBCI but not correlated in BCI. Further, for several key genes, this correlation is significantly lower in the NBEC of BCI. Details of these methods are provided in the Examples below. Briefly, TF recognition sites common to genes associated with BC (e.g., GSTP1, GPX1, CAT, GPX3, and SOD1) can be identified through sequence analysis, e.g., *in silico* DNA sequence analysis. Such sequence analysis using Genomatix Software GmbH, Munich, Germany, http://genomatix.de/cgi-bin/eldoradol) (Quandt K, Frech K, Karas H, Wingender E, and Werner T, NAR, 23, 4878-4884. 1995), for example, yields sites for 11 TFs, including EV11 and members of the C/EBP and E2F families.

Expression levels of the 11 identified TFs can be assayed in NBEC case samples from patients with BC and in control NBEC samples obtained from healthy individuals. For example, standardized RT-PCR reagents can be prepared and preferentially optimized for the TFs and other genes, e.g., as provided in Willey JC, et al, in Methods in Molecular Biology (ed. Shimkets, R.A.) 13-41 (Humana Press, Inc., Totowa, N.J., 2004). TFs found to be expressed at low and/or invariant levels among multiple NBEC samples can be excluded from further analysis. Remaining TFs can be evaluated for correlation with an expanded group of AO and/or DNAR genes, including e.g., XRCC1, ERCC5, GSTP1, SOD1, GPX3, ERCC1, CAT, GSTZ1, and ERCC2.

As detailed in the Examples below, expression levels of XRCC1, ERCC5, GSTP1, SOD1 and GPX1 are significantly or nearly significantly correlated with expression levels of CEBPG in NBCI compared in BCI. Loss of correlation in BCI compared to NBCI can also be observed between expression levels of E2F1 with expression levels of ERCC5, GSTP1 and SOD1.

Other AO and/or DNAR genes can also be assayed. Examples of AO genes include those encoding enzymes (such as glutathione transferases (GSTs, e.g., GSTT1) and glutathione peroxidases (GSHPxs, e.g., GSHPxA)) that are capable of preventing or reducing injury from carcinogens. There are several classes of GSTs, including one microsomal class (mGST) and at least five cytosolic classes: GSTA, GSTM (e.g., GSTM1, GSTM3), GSTP (e.g., GSTP1), GSTT, and GSTZ. See also, e.g., Crawford et al., Cancer Res 60: 1609-1618 (2000); Hackett et al., American Journal of Respiratory Cell and Molecular Biology 29: 331-343 (2003); and Willey et al., ILSI Press, Washington, D.C., U. Heinrich and U. Mohr (Eds), pp. 79-96 (2000).

Examples of DNAR genes include those encoding enzymes that can recognize and/or repair specific nucleotide alterations, base mispairs, and double-strand breaks. DNAR pathways that have been identified in mammalian cells and which play major roles in protection against mutation are: 1) DNA mismatch repair (MMR), 2) nucleotide excision repair (NER), 3) base excision repair (BER), 4) damage reversal by 06-methylguanine DNA methyltransferase (MGMT), 5) homologous recombination (HR), and 6) non-homologous end joining (NHEJ).

Without being limited to a given theory and/or hypothesis, it appears that smokers are selected to develop BC at least in part due to sub-optimal AO and/or DNAR gene regulation by CEBPG. That is, in NBCI, CEBPG may regulate transcription of key AO and/or DNAR genes in NBEC and in smokers who develop BC, CEBPG regulation may be sub-optimal for a sufficient number of AO and/or DNAR genes to cause increased risk. For example, one possible explanation for loss of correlation in BCI is alteration in the function of one or more TFs responsible for correlation in NBCI. Methods provided herein may improve understanding of risk for lung cancer and enable early screening and chemoprevention for those at the highest risk.

One of skill in the art will recognize that the methods provided herein can be applied to the identification of biomarkers for other cancer-related conditions. Examples of other cancer-related conditions include, but are not limited to, breast cancer, skin cancer, bone cancer, prostate cancer, liver cancer, lung cancer, brain cancer, cancer of the larynx, gallbladder, pancreas, rectum, parathyroid, thyroid, adrenal, neural tissue, head and neck, colon, stomach, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumor, small-cell lung tumor, gallstones, islet cell tumor, primary brain tumor, acute and chronic lymphocytic and granulocytic tumors, hairy-cell tumor, adenoma, hyperplasia, medullary carcinoma, pheochromocytoma, mucosal neuronms, intestinal ganglloneuromas, hyperplastic corneal nerve tumor, marfanoid habitus tumor, Wilm's tumor, seminoma, ovarian tumor, leiomyomater tumor, cervical dysplasia and in situ carcinoma, neuroblastoma, retinoblastoma, soft tissue sarcoma, malignant carcinoid, topical skin lesion, mycosis fungoide, rhabdomyosarcoma, Kaposi's sarcoma, osteogenic and other sarcoma, malignant hypercalcemia, renal cell tumor, polycythermia vera, adenocarcinoma, glioblastoma multiforma, leukemias, lymphomas, malignant melanomas, epidermoid carcinomas, and other carcinomas and sarcomas.

Case and control samples may be obtained from different stages of cancer. Cells in different stages of cancer, for example, include non-cancerous cells vs. non-metastasizing cancerous cells vs. metastasizing cells from a given patient at various times over the disease course. Cancer cells of various types of cancer may be used, including, for example, a bladder cancer, a bone cancer, a brain tumor, a breast cancer, a colon cancer, an endocrine system cancer, a gastrointestinal cancer, a gynecological cancer, a head and neck cancer, a leukemia, a lung cancer, a lymphoma, a metastases, a myeloma, neoplastic tissue, a pediatric cancer, a penile cancer, a prostate cancer, a sarcoma, a skin cancer, a testicular cancer, a thyroid cancer, and a urinary tract cancer. In preferred embodiments, biomarkers can be developed to predict which chemotherapeutic agent can work best for a given type of cancer, e.g., in a particular patient.

The methods for identifying biomarkers for BC can be applied to identifying biomarkers for these other cancer-related conditions. For example, TF recognition sites common to genes associated with one of these other cancer-related conditions can be identified through sequence analysis. Examples of genes associated with cancer-related conditions include, but are not limited to, antioxidant (AO), xenobiotic metabolism enzyme genes (XME) and DNA repair (DNAR) genes. Examples of XME genes include those expressed in human NBEC that metabolize carcinogens and/or pro-carcinogens present in cigarette smoke, such as, but not limited to, cytochromes p450 (CYP) 1A1, 1B1, and 2B6, which metabolize polycyclic aromatic hydrocarbon procarcinogens in cigarette smoke, epoxide hydrolase, NAPD oxidoreductase and phenolosulfotransferases, which also metabolize polycyclic aromatic hydrocarbons; and CYP2A6/7 and CYP2E1, which metabolize nitroso compounds, such as nitrosamines. See, e.g., Willey et al., Am J Respir Cell Mol Biol 17(1): 114-124 (1997); and Willey et al., Am J. Respir Cell Mol Biol 14(3): 262-271 (1996).

Expression levels of the identified TFs can be assayed in case samples from patients with the cancer-related condition and in control samples obtained from healthy individuals or obtained from different stages of cancer. In preferred examples, standardized RT-PCR reagents can be prepared and preferentially optimized for, the TFs and other genes, e.g., as provided in Willey JC, et al, in Methods in Molecular Biology (ed. Shimkets, R.A.) 13-41 (Humana Press, Inc., Totowa, N.J., 2004). TFs found to be expressed at low and/or invariant levels among multiple control samples can be excluded from further analysis. Remaining TFs can be evaluated for correlation with an expanded group of genes known to be associated with the cancer-related condition. Additional details are provided in the Examples below.

### Biomarkers for Chronic Obstructive Pulmonary Disease and other Lung-related Conditions

Transcription factor biomarkers can be identified for COPD. COPD includes, e.g., emphysema (including both heterogeneous emphysema and homogenous emphysema), asthma, bronchiectais, and chronic bronchitis. Genes associated with COPD also include AO and DNAR genes. Without being limited to a particular hypothesis and/or theory, there may be inter-individual variation in regulation of key AO and DNAR genes by one or more TFs and individuals with sub-optimal regulation may be selected for development of COPD, especially if they are smokers. Identified biomarkers can indicate COPD, risk of COPD, extent of COPD (e.g., metastasizing or non-metastasizing) and/or prognosis (e.g., likelihood and/or degree of responsiveness to a particular therapy).

The methods provided herein may show that transcript abundance of CEBPG transcription factor is significantly (p < 0.01) correlated with key antioxidant (AO) or DNA repair (DNAR) genes in control samples obtained from healthy individuals but not correlated in case samples obtained from COPD patients. Without being limited to a given theory and/or hypothesis, smokers may be selected to develop COPD on the basis of sub-optimal AO and/or DNAR gene regulation by the transcription factor CEBPG.

TF recognition sites common to genes associated with a COPD (e.g., GSTP1, GPX1, CAT, GPX3, and SOD1) can be identified through sequence analysis, e.g., using Genomatix Software GmbH, Munich, Germany, http://genomatix.de/cgi-bin/eldorado/) (Quandt K, Frech K, Karas H, Wingender E, and Werner T, NAR, 23, 4878-4884. 1995). Expression levels of identified TFs can be assayed in NBEC case samples from patients with COPD and in NBEC control samples obtained from healthy individuals. For example, standardized RT-PCR reagents can be prepared and preferentially optimized for the TFs and other genes, e.g., as provided in Willey JC, et al, in Methods in Molecular Biology (ed. Shimkets, R.A.) 13-41 (Humana Press, Inc., Totowa, N.J., 2004). TFs found to be expressed at low and/or invariant levels among multiple control samples can be excluded from further analysis. Remaining TFs can be evaluated for correlation with an expanded group of AO and/or DNAR genes, including e.g., XRCC1, ERCC5, GSTP1, SOD1 or GPX1. Similar findings may be obtained for the transcription factor E2F1.

One of skill in the art will recognize that the methods provided herein can be applied to the identification of biomarkers for other lung-related conditions. Examples of lung-related conditions include, e.g., sarcoidosis, pulmonary fibrosis, pneumothorax, fistulae, bronchopleural fistulae, cystic fibrosis, inflammatory states, and/or other respiratory disorders. Lung-related conditions can also include smoking-related and/or age-related changes to the lung, as well as lung damage caused by a traumatic event, infectious agents (e.g., bacterial, viral, US2005/0048480 A1 discloses a method of identifying a deregulation of a marker by correlating it to the level of the marker in healthy tissue, both correlated to the level of the responsible transcription factor. fungal, tuberculin and/or viral agents), exposure to toxins (e.g., chemotherapeutic agents, environmental pollutants, exhaust fumes, and/or insecticides), and/or genetic factors (e.g., alpha-1 antitrypsin deficiency and other types of genetic disorders which involve elastic and/or connective tissues degradation and/or impaired synthesis of elastic and/or connective tissues and/or impaired repair of elastic and/or connective tissues of the lungs).

For example, TF recognition sites common to genes associated with one of these other lung-related conditions can be identified through sequence analysis. Expression levels of the identified TFs can be assayed in case samples from patients with the lung-related condition and in control samples obtained from healthy individuals or obtained from different stages of the lung-related condition. standardized RT-PCR reagents can be prepared and preferentially optimized for the TFs and other genes, e.g., as provided in Willey JC, et al, in Methods in Molecular Biology (ed. Shimkets, R.A.) 13-41 (Humana Press, Inc., Totowa, N.J., 2004). TFs found to be expressed at low and/or invariant levels among multiple control samples can be excluded from further analysis. Remaining TFs can be evaluated for correlation with an expanded group of genes known to be associated with the lung-related condition.

### B. Identification of Polymorphisms

In another aspect, the disclosure relates to methods for identifying polymorphisms that indicate a biological state. the method involves identifying a nucleotide variation between case samples and control samples in a transcription factor or other gene, where the transcription factor and/or the other gene are identified using methods provided herein. Some examples can comprise obtaining a plurality of control samples wherein expression levels of a transcription factor are correlated with expression levels of another gene; obtaining a plurality of case samples wherein expression levels of the transcription factor are not correlated with expression levels of the gene; and identifying a nucleotide variation in the transcription factor and/or in the gene in one or more of said case samples compared with one or more of said control samples.

Some examples provide a method for identifying DNA sequence variation associated with disease and/or risk for disease involving a) determining expression levels of genes involved in i) conferring the phenotype or ii) regulating transcription of the genes involved in conferring the phenotype, b) identifying a transcription factor responsible for regulating the relevant genes for which expression levels are correlated with regulated gene expression levels in low risk and/or non-diseased individuals/tissues, but is not correlated with normally-regulated genes in at risk individuals and/or diseased individuals/tissues, and c) identifying one or more DNA sequence variations responsible for determining regulation of the involved genes.

"Polymorphism" or "DNA sequence variation" as used herein can refer to any one of a number of alternative forms of a given locus (position) on a chromosome. The alternative form may involve a single base pair difference, such as a single nucleotide polymorphism (SNP). The polymorphism may involve more than one base pair change, e.g., it may involve at least about 2, at least about 3, or least about 10 nucleotide differences. the polymorphism may involve less than about 50, less than about 100, less than about 200, or less than about 500 nucleotide differences. The term polymorphism may also be used to indicate a particular combination of alleles, e.g., two or more SNPs, in a given gene or chromosomal segment. In some embodiments, for example, identification of more than one nucleotide variation identifies a biological state, e.g., a specific combination of alleles at particular genes may indicate risk for a disease condition.

Identifying the nucleotide variation can be achieved by any methods known in the art, e.g., using various methods for determining sequence information of nucleic acids. Examples include the dideoxy termination method of Sanger (see, e.g., Sanger et al., Proc. Natl. Acad. Sci. U.S.A. 74: 563-5467 (1977)); the Maxam-Gilbert chemical degradation method (see, e.g., Maxam and Gilbert, Proc. Natl. Acad. Sci. U.S.A. 74: 560-564 (1977);
Sanger-extension method using dyes associated with terminal nucleotides, gel electrophoresis and automated fluorescent detection; techniques using mass spectroscopy instead of electrophoresis; pyrophosphate release techniques (see, e.g., Ronaghi et al., "A Sequencing Method Based on Real-Time Pyrophosphate," Science 281: 363-365 (1998) and Hyman, "A New Method of Sequencing DNA," Anal. Biochem. 174: 423-436 (1988); single molecule sequencing techniques utilizing exonucleases to sequentially release individual fluorescently labeled bases (see, e.g., Goodwin et al., "Application of Single Molecule Detection to DNA Sequencing," Nucleos. Nucleot. 16: 543-550 (1997); techniques pulling DNA through a thin liquid film as it is digested in order to spatially separate the cleaved nucleotides (see, e.g., Dapprich et al., "DNA Attachment to Optically Trapped Beads in Microstructures Monitored by Bead Displacement," Bioimaging 6: 25-32 (1998)); techniques determining the spatial sequence of fixed and stretched DNA molecules by scanned atomic probe microscopy (see, e.g., Hansma et al., "Reproducible Imaging and Dissection of Plasmid DNA Under Liquid with the Atomic Force Microscope," Science 256: 1180-1184 (1992)); techniques described in U.S. Pat. No. 5,302,509 to Cheeseman and in U.S. 2003/0044781 (Korlach); and technique using hybridization of (substantially) complementary probes as described, e.g., in U.S. Pat. Publication Nos. 2005/0142577 and 2005/0042654 (Affymetrix). Identified polymorphisms can comprise certain alleles that are represented at higher or significantly higher rates in a disease condition, e.g., as described in more detail below.

In some examples, the pattern of expression levels of a transcription factor and its normally-regulated gene allows focus on a particular region of the transcription factor and/or gene, in order to identify polymorphisms indicative of the biological state. Methods further comprise obtaining a first relation comparing expression levels of the gene to expression levels of the transcription factor in control samples; obtaining a second relation comparing an expression level of the gene to an expression level of the transcription factor in one of the case samples; comparing first and second relations; and analyzing a region of said transcription factor and/or said gene based on the comparison in order to identify said nucleotide variation.

"Region" as used herein can refer to a nucleic acid sequence that preferably involves fewer base pairs than the entire gene. A region can include coding and non-coding, transcribed and non-transcribed, and/or translated and un-translated regions. For example, a region of a gene can include the regulatory elements 5' of the coding region, e.g., recognition sites for TF. A region of a TF can include 5' regulatory regions, 3' UTR and/or coding regions of the TF. Methods provided herein teach specific region to focus on in identification of polymorphisms indicative of a biological state. In some examples the region spans at least about 5, at least about 10, at least about 20, at least about 30, at least about 50, at least about 80, or at least about 100 bases. In some examples, the region spans less than about 150, less than about 200, less than about 250, less than about 300, or less than about 500 bases.

First and second relations can refer to any mathematical, graphical, statistical relationship between values. In some examples, expression levels of the transcription factor can be plotted against expression levels of the other gene, where the expression levels are assayed in control samples. The control sample values can be used to obtain a regression line as the first relation. The second relation can comprise a coordinate point, e.g., plotted with the regression line, of transcription factor expression level versus the expression level of the other gene, where the expression levels are assayed in a given case sample. In such examples, first and second relations can be compared in terms of whether the case sample coordinate point falls on, above, or below said regression line.

In some examples, where the coordinate point falls above the regression line, focus is directed to the 5' regulatory region and/or 3' UTR of the transcription factor. In some examples, where the coordinate
point falls below the regression line, focus is directed to the coding region of the transcription factor and/or recognition sites for the transcription factors in other genes. The coordinate point may fall above, on, or below the line for different individuals, and where the coordinate falls indicates the region(s) to focus on when analyzing nucleic acids from those individuals. The frequency of polymorphisms in case samples can be determined and compared to frequency in controls. Additional explanation, discussion and details are provided in the Examples below, specifically in relation to NBCI and BCI.

### Polymorphisms for Bronchogenic Carcinoma and other Cancer-related conditions

In some examples, polymorphisms that indicate BC can be identified using the methods provided herein. For example, the nucleotide sequences of CEBPG and E2F1 transcription factors can be analyzed to determine variation between case and control samples. For, the 5' regulatory region, 3' UTR and/or coding regions of the TFs can be analyzed. In some examples, the nucleotide sequences of XRCC1, ERCC5, GSTP1, SOD1 or GPX1 can be analyzed to determine variation between case and control samples. Preferably, TF recognition sites common to such genes are analyzed, e.g., the 1100 bp regulatory region (1000 upstream and 100 bp downstream of the transcription start site) of XRCC1. The frequency of each allele at each SNP in NCBI can be determined and compared to frequency of each allele at each SNP in BCI. Those of skill in the art will recognize that the methods provided herein can be applied to the identification of polymorphisms for other cancer-related conditions.

Structural knowledge of transcription factor biomarkers can aid in identifying biomarker polymorphisms. For example, it is known that CEBPG is a truncated CEBP TF (Johnson PF, and Williams SC, in Liver Gene Expression (eds Yaniv M and Tronche F) 231-258 (R. G. Landes Company, 1994). CEBPG possesses sequences necessary for DNA binding and heterodimer formation, but lacks sequences necessary for transactivation (Cooper C, Henderson A, Artandi S, Avitahl N, Calame K, NAR, 23, 4371-4377,1995). CEBPG can form heterodimers with other CEBP family members, e.g., leading to increased (Hongwei G, Parkin S, Johnson PF, and Schwartz RC, JBC, 277, 38827-38837, 2002) or decreased (Cooper C, Henderson A, Artandi S, Avitahl N, Calame K, NAR, 23, 4371-4377, 1995) transcription of regulated genes.

In preferred examples, DNA regions analyzed to provide polymorphisms include regions affecting transcription regulation, protein function, post-transcriptional processing, and/or protein-protein binding, including those in the 5' regulatory region, those in the 3' UTR, translated region, and 5' UTR of the coding region. For example, sequences for DNA binding and heterodimer formation can be analyzed for polymorphisms indicative of BC. Specifically, the collinear string of 50 bp in the bZip region of CEBPG can also be analyzed for polymorphisms indicative of BC. Further, regions known to be associated with risk can be evaluated for polymorphism. See, e.g., regions described in Ratnasinghe et al, Anticancer Res. 23: 627-32 (2003); Wang, DNA Repair (Amst). 2(8): 901-8 (2003); and Misra et al, Cancer Lett. 191:171-8 (2003)). Additional details are provided in the Examples below.

Identification of polymorphisms that affect regulation of XRCC1, ERCC5, GSTP1, SOD1, and GPX1 by CEBPG can also yield biomarkers. A biomarker combining polymorphisms that affect regulation with those that affect function of AO and DNAR genes is preferred in some examples for identifying individuals at risk for BC. For example, a biomarker associated with functional alteration in regulation of risk secondary to one or more variations in DNA sequence enables focus on genes that contribute to risk. This can enables marked reduction in the number of individuals that would have to be included in epidemiologic studies.

For example, the polymorphism is at position -77 in XRCC1 gene. (SNP, rs3213245, -77T>C). (See, Hao et al. Oncogene, 2006; 25:3613-20).

As discussed above, the relation of expression levels of TG and TF from a particular BCI can indicate regions to be analyzed for polymorphisms in that particular BCI. As detailed in the Examples below, the 5' regulatory region, coding region and/or 3' UTR of CEBPG are analyzed specifically, as well as CEBPG recognition sites for target genes, such as XRCC1, ERCC5, SOD1, GSTP1 and/or GPX1. Compositions and methods disclosed herein are utilized to identify a cancer biomarker, whereby inheritance of particular alleles at polymorphic sites in CEBPG and ERCC5 contributes to increased risk for BC by causing sub-optimal coordinate regulation of AO and DNAR genes in NBEC. Previous attempts to identify biomarkers for cancer have enjoyed limited success (Caporaso, 2002) and the results have not been sufficiently strong to yield clinically useful biomarkers. Most of these studies involved assessment for risk bearing alleles at polymorphic sites that affect either the function or regulation of one or a few xenobiotic metabolism enzyme (XME), antioxidant (AO), and/or DNA repair (DNAR) genes (Dandara et al, 2002; Wang et al, 2003; Watson et al, 1998). For example, in some epidemiologic studies, individuals with GSTM1 null genotype or certain GSTP1 polymorphisms have an increased BC risk (Seidegard et al, 1990; Nazar-Stewart et al, 1993). However, the results of other studies are contradictory (Zhong et al, 1991). Evidence is mounting that, because the pathways designed to protect cells from mutation and subsequent uncontrolled growth (including DNA repair and antioxidant mechanisms) are redundant, it is likely that multiple genetic variants, each affecting one or more genes of a pathway, are necessary to cause measurable increased risk (Caporaso,2002; Caporaso, 2003; Spitz et al, 2003; Xi et al, 2004). However, even studies that evaluate multiple genes are not guaranteed success (Misra, et al, 2003). One possible reason for limited success in previous studies is that there was little or no biological information to indicate that a particular polymorphic site would be associated with altered gene function, gene regulation, or increased risk. Especially in early studies, the polymorphic sites were chosen for analysis because they were conveniently located and common, not because they were known to have a biological effect that could be expected to affect BC risk.

In contrast to previous studies, an advantage of the present disclosure is that genes and polymorphic sites were chosen for both epidemiologic sequencing analysis and experimental mechanistic investigation based on extensive biological information indicating a role in BC risk. Specifically, extensive transcript abundance profiling was done to compare NBEC, the progenitor cell for BC, from Non-BC individuals to BC idividuals. Based on the findings of these analyses, there is strong rationale to seek biomarkers for BC risk through more detailed evaluation of XRCC1, SOD1, GPX1, GSTP1, and ERCC5 gene regulation by CEBPG in Non-BC individuals compared to BC individuals. In one example, rigorous epidemiologic and experimental evaluation of ERCC5 regulation by CEBPG.

There are several different pathways of DNA repair in mammalian cells, each involving several different proteins and each specific for a certain type of DNA damage (Mohrenweiser and Jones, 1998). ERCC5 participates in the nucleotide excision repair (NER) pathway. The NER pathway involves over 20 proteins and repairs the DNA damage caused by UV radiation and the adducts caused many different chemicals, including platinum drugs (Mohrenweiser and Jones, 1998; Seeberg, 1995). The damaged DNA is identified, and incisions bracketing the area of damage in a 24-32 bp region are made by NER-specific enzymes, including excision repair cross-complementing rodent repair deficiency, complementation group 1 (ERCC1) and ERCC5 (de Laat et al., 1999; Griffin et al., 2005; Ura and Hayes, 2002). General replication factors then fill the gap and ligate it (Sancar et al., 2004). Furthermore, target genes are regulated by selected transcription factors or families of transcription factors.

CCAAT/Enhancer Binding Protein (CEBP) Family of Transcription Factors and CEBPG. The CEBP family of transcription factors (CEBPA, -B, -D, -E, -G and -Z) are basic region-leucine zipper (bZIP) proteins. The bZIP region is characterized at the C-terminal by a positively-charged, basic DNA-binding domain and an N-terminal dimerization domain containing heptad leucine repeats (Landschulz et al, 1988b; Ramji and Foka, 2002;
Vinson et al, 2002). The CEBP proteins share greater than 90% homology in this region, allowing them to bind a consensus palindromic DNA recognition site sequence, with the exception of CEBPZ (Cassel and Nord, 2003; Ramji and Foka, 2002). Dimerization of these proteins produces a "scissors-grip," or inverted Y shape that binds to the palindromic DNA recognition site and is necessary for DNA binding (Landschulz et al., 1989; Ramji and Foka, 2002; Vinson et al., 1989). Heterodimerization has been demonstrated for all CEBP protein combinations in vitro, and cannot occur when leucines in the zipper are mutated (Cooper et al., 1995; Lekstrom-Himes and Xanthopoulos, 1998; Ramji and Foka, 2002). The N-termini of the CEBP proteins share less than 20% homology, and contain the transactivation domains responsible for transcriptional regulation, with the exception of CEBPG which is shortened and lacks the transactivation domain (Lekstrom-Himes and Xanthopoulos, 1998; Ramji and Foka, 2002; Takiguchi, 1998). The CEBP proteins, particularly CEBPB, -D and -Z, have been demonstrated to be regulated by post-translational phosphorylation (Lacorte et al., 1997; Nakajima et al., 1993).

For example, the transcription factor CEBPG is ubiquitously expressed, and differs from the other family members by being truncated, possessing the sequences necessary for DNA binding, but lacking the N-terminal transactivation domain (Cooper et al., 1995; Roman et al., 1990). Because of this, it was originally thought that CEBPG strictly formed heterodimers to perform the function of being a dominant negative regulator of the other CEBP family members (Cooper et al., 1995). However, it has since been determined that: a) CEBPG can act as an activator of transcription by forming heterodimers with other CEBP family members, b) this function is dependent upon heterodimer formation in the bZIP region, and c) this function can be demonstrated in the absence of the activation domain of the other protein (Gao et al., 2002). CEBPB has been shown in several cell types to exist in high quantities as a heterodimer with CEBPG (Pan et al., 2000) (Wall et al., 1996), and CEBPG also seems to preferentially dimerize with CEBPB (Gao et al., 2002). Finally, CEBPG-null mice are healthy at birth but begin to die within 48 hours (Kaisho et al., 1999). These mice exhibit natural killer cell dysfunction, and histological examination reveals emphysematous lungs (Kaisho et al., 1999). In humans, risk for emphysema is associated with antioxidant capacity (Repine et al., 1997), and there is a strong correlation between risk for emphysema and risk for BC (Schabath et al., 2005).

As provided in more detail in Example 7, *infra,* alleles with higher prevalence in BC Individuals are associated with suboptimal CEBPG regulation of AO and/or DNAR genes in NBEC and in peripheral blood cells (PBC). First, sequencing is conducted of ERCC5 in 110 Non-BC and 110 BC individuals to confirm that the ERCC5 -222 G allele is represented at a significantly higher rate in BC Individuals. Sequence information from this sample number enables demonstration of significant difference at p value of 0.05 and 80% power. Second, expression levels were measured for CEBPG and ERCC5 in the normal bronchial epithelial cells and peripheral blood cells (PBC) of the same 220 individuals as in the preceding step. As such, an assessment is provided as to whether the ERCC5-222, -228, and/or other polymorphic sites are associated with lower ERCC5 expression relative to CEBPG. In addition, ERCC5 regulation by CEBPG was examined to determine whether specific alleles at polymorphic sites affect this regulation. Successful completion of the proposed aims should provide candidate biomarkers for BC risk and increase mechanistic understanding of BC risk. *(See,* Example 5, *infra*). Therefore, the present invention enables identification of biomarkers of additional AO or DNAR genes providing better treatment and diagnostic options for BC.

### Polymorphisms for COPD and other Lung-related Conditions

Polymorphisms that indicate COPD can be identified using the methods provided herein. For example, similar regions of TF, AO and/or DNAR genes can be analyzed, as described with respect to BC. Without being limited to a given theory and/or hypothesis, there is a strong correlation between risk
for emphysema and risk for BC, and risk for emphysema is also associated with antioxidant capacity in humans (Repine JE, Bast A, and Lankhorst, I, Am. J. Respir. Crit. Care Med., 156, 341-357, 1997). Also, CEBPG-/- knockout mice begin to die within 24 hours of birth, showing emphysematous lungs on histological examination (Kaisho T, et al, J. Exp. Med, 190,1573-1581, 1999).

For example, the nucleotide sequences of CEBPG and E2F1 transcription factors can be analyzed to determine variation between case samples from COPD patients and control samples. Preferably, the 5' regulatory, coding, and 3' un-translatd regions of the TFs are analyzed. The nucleotide sequences of XRCC1, ERCC5, GSTP1, SOD1 or GPX1 can be analyzed to determine variation between case samples from COPD patients and control samples. Preferably, TF recognition sites common to such genes are analyzed, more preferably CEBPG recognition sites of XRCC1, ERCC5, GSTP1, SOD1 and/or GPX1. As detailed above, DNA regions analyzed to provide polymorphisms include regions affecting transcription regulation, protein function, post-transcriptional processing, and/or protein-protein binding, including those in the upstream regulatory region, and those in the 3' UTR, translated region, and 5' UTR of the coding region. Those of skill in the art will recognize that the methods provided herein also can be applied to the identification of biomarkers for other lung-related conditions.

Some examples also provide probes consisting of relevant nucleic acid sequences for identifying polymorphisms indicative of COPD. Examples of four such probes include (i) a probe comprising a nucleic acid sequence consisting of a 5' regulatory region of CEBPG ± about 100 bases; (ii) a probe comprising a nucleic acid sequence consisting of a 3' untranslated region of CEBPG ± about 100 bases (iii) a probe comprising a nucleic acid sequence consisting of a bZip region of CEBPG ± about 100 bases; and (iv) a probe comprising a nucleic acid sequence consisting of a CEBPG recognition site ± about 100 bases. Such probes can be anchored to a support, e.g., in an array, and/or provided in kits for use in identifying COPD or risk thereof, as well as other lung-related conditions

### II. Methods and Compositions for Diagnosis

### A. Lack of Correlation Approch

In another aspect, the disclosure relates to methods and compositions for diagnosing a biological state by identifying loss of correlation between two or more biomarkers compared to controls. In some examples, the method involves identifying lack of correlation between expression levels of a transcription factor and another gene. Generally, the other gene is a gene associated with the biological state and/or a gene that is regulated by the transcription factor in controls.

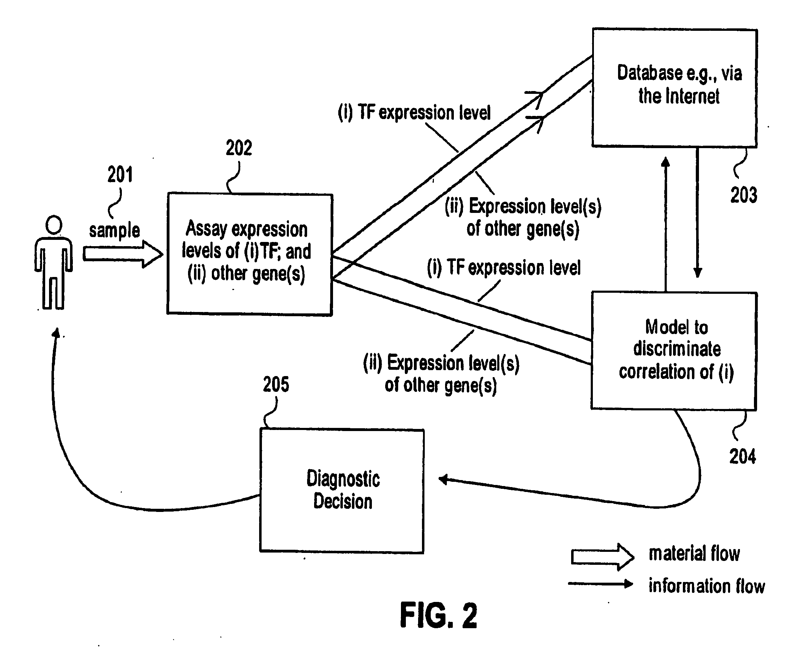

Figure 2 illustrates the overall process for diagnosing a biological state in some examples disclosed herein. At step 201, a sample is collected from a subject, e.g. a patient. The sample may comprise, e.g., any tissue or biological fluid as provided in detail above. The type of sample collected may depend, e.g., on the biological state sought to be identified. For example, NBEC samples may be obtained to test for BC and/or COPD, described in more detail below. In preferred examples, the sample is a readily-accessible sample, e.g., one that can be obtained with a non-invasive or mildly-invasive technique. Such samples include, e.g., urine samples, bloods samples, semen samples, or more preferably saliva samples, buccal and/or nasal epithelial cell samples.

At step 202, expression levels of (i) a transcription factor and (ii) at least one other gene are assayed using the sample obtained from the subject. Any methods for assaying expression levels may be used, e.g., as described above. In preferred examples, the expression levels are measured using standardized mixtures of reagents that comprise known amounts of internal standards as described, e.g., in U.S. Patent Application Serial Nos. 11/072,700 and 11/103,397.

At step 203, expression level values are entered into a database. In some examples, the database can be accessed over the Internet. In some examples, demographic information regarding the subject can be recorded along with the results of gene expression measurements on the collected samples. Preferably, such data is stored in a separate database from the identifying information. For example, the identifying information can be separated from the sample and demographic information as soon as the sample is brought into the laboratory. In preferred examples, the database allows for collection of values for various expression measurements, e.g., measurements obtained from different patients, the same or different patients over time (e.g., over the course of a disease and/or over the course of a treatment regime). In preferred examples, these data are directly comparable within certain CV limits, e.g., as taught in U.S. Provisional Application Serial No. 60/646,157. In some examples, such a database can be used with gene expression data in clinical diagnostic testing, e.g., as described below.

At step 204, the expression levels are mathematically computed using a model that discriminates whether or not the expression levels are correlated with each other. As Figure 2 illustrates, in some examples, assayed expression levels for (i) the transcription factor and (ii) the other gene(s) are entered into a database 203 and data from the database is used in the model. In some examples, assayed expression levels for (i) the transcription factor and (ii) the other gene(s) are used directly in the model. The model allows discrimination of whether or not (i) is correlated with (ii). Lack of correlation in the sample obtained from the subject can indicate the biological state. In still some examples, newly identified biomarkers themselves can be used to identify the biological state, e.g., as discussed in more detail below.

A model that discriminates whether or not expression levels are correlated with each other can be obtained by any means, e.g., using techniques of biostatistics and/or bioinformatics. For example, in some examples, the model was obtained by assaying in a plurality of case samples expression levels of the transcription factor and of the other gene; assaying in a plurality of control samples expression levels of the transcription factor and of the other gene; and using said expression levels to compute the model.

In some examples, the model is computed using at least one technique selected from bivariate analysis, mutivariate analysis, genetic programming software analysis, logarithmic transformation, Pearson's correlation, Bonferroni adjustment, Fisher's Z-transformation test, t-test, two-sided test, ANOVA, and Duncan's test. Models can be derived using a training set of subjects, assessed using expression levels obtained from additional sets of subjects, and suitable models can be refined through analysis of additional genes in the training sets and validation in additional sets. Additional details are provided in the Examples below.

In some examples, the model comprises a relation between expression levels of the transcription factor and other gene, e.g., as described below for diagnosing BC. In some examples, the relation is a ratio, e.g., a gradient of a regression line plotting expression levels of a transcription factor against expression levels of a target gene for controls, e.g., TF/TG or TG/TF. In some examples, where expression levels obtained from a sample coincide with the ratio (e.g., fall along the regression line), correlation is indicated; where expression levels obtained from a sample do not coincide with the ratio (e.g., being significantly above or below the regression line), lack of correlation is indicted. In preferred examples, the TG/TF ratio for each of a plurality of TGs does not coincide with a regression line ratio. For example, the TG/TF ratio does not coincide for at least about 2, at least about 3, at least about 5, at least about 10, at least about 20, at least about 50, at least about 80, or at least about 100 TGs. In
some examples, the TG/TF ratio does not "coincide with a regression line ratio for less than about 150, less than about 200, less than about 300, or less than about 500 TGs. Additional details are provided in the Examples below.

In preferred examples, a high-speed computer program can be used to cause multiple expression level values to interact in a random manner, e.g., according to a multiplicity of linear and non-linear functions. This can (a) enable rapid determination of mathematical rules that best fit the data; and (b) provide information regarding the likely scaling of expression levels to experimentally-observed function for various genes (i.e. based on whether predominantly linear or non-linear mathematical functions relate expression levels for a particular gene to that of other genes). Software that can be used for this purpose include, e.g., Genetics2, Ann Arbor, MI.

At step 205, a diagnosis decision can be made, e.g., based on whether or not (i) and (ii) are correlated, and/or on the level of a biomarker identified. The diagnostic decision may comprise identification of a disease condition, the stage or extent of progression of the condition, and/or the likeliness of response to various treatments. As Figure 2 illustrates, the information regarding diagnosis can be communicated to the subject, e.g., a patient in a clinical setting.

### Diagnosis of Cancer-related Conditions and/or Lung-related Conditions

In some examples, the disclosure provides methods and compositions for diagnosing a cancer-related condition, e.g., BC, and/or a lung-related condition, e.g., COPD. For example, BC and be diagnosed by identifying lack of correlation (compared to controls) between a transcription factor and a gene associated with BC. For example, some examples comprise assaying an expression level of CEBPG and/or E2F1 in a sample obtained from a subject; assaying an expression level of at least one other gene in the sample, where the other gene is associated with BC; and mathematically computing the expression levels using a model that discriminates whether or not the expression levels are correlated with each other. Lack of correlation in the sample obtained from the subject can indicate BC, risk of BC, extent of BC (e.g., metastasizing or non-metastasizing) and/or prognosis (e.g., likelihood and/or degree of responsiveness to a particular chemotherapy). Some examples provide methods for predicting resistance of individual tumors to a chemotherapeutic agents, e.g., by taking samples for individual tumors.

In some examples, COPD and be diagnosed by identifying lack of correlation (compacted to controls) between a transcription factor and a gene associated with COPD. Some examples comprise assaying an expression level of CEBPG and/or E2F1 in a sample obtained from a subject; assaying an expression level of at least one other gene in the sample, where the other gene is associated with COPD; and mathematically computing the expression levels using a model that discriminates whether or not the expression levels are correlated with each other. Lack of correlation in the sample obtained from the subject can indicate COPD, risk of COPD, extent of COPD, and/or prognosis (e.g., likelihood and/or degree of responsiveness to a particular therapy). One of skill in the art will recognize that other cancer-related conditions and/or other lung-related conditions can also be diagnosed using the approaches described herein.

In some examples, the sample obtained comprises bronchial epithelial cells, e.g., NBECs. In preferred examples, as described above, the sample comprises readily-acgssible cells, such as but not limited to, nasal epithelial cells, buccal epithelial cells or blood cells. In some examples, the subject is a smoker, e.g., an individual having a heavy smoking history.

In some examples, the other gene is selected from an AO gene and a DNAR gene, including, but not limited to XRCC1, ERCC5, GSTP1, SOD1, GPX1, ERCC1, CAT, GSTZ1 and/or ERCC2. Generally, the other gene is regulated by CEBPG and/or E2F1 in control samples. Expression levels can be assayed by methods known in the art, e.g., any of the techniques provided above. Preferably, methods of the instant disclosure allow
early detection of BC and/or COPD, improving efficacy of prevention. Methods described herein also can facilitate inclusion of only individuals at higher risk in trials for BC and/or COPD, which in turn can improve the cost-effectiveness of such studies.

In some examples, the model used to identify BC and/or risk thereof involves a relation between expression levels of CEBPG and one or more AO and/or DNAR genes, e.g., one or more AO and/or DNAR genes present at high incidence in NBECs of BCI but not present or present at low incidence in NBCI. For example, in some examples a ratio of TG/TF is used, as discussed above. In some examples, lack of correlation of CEBPG with each AO or DNAR gene is characteristic of NBEC from individuals who are more susceptible to BC. That is lack of correlation of CEBPG with an TG can indicate increased BC risk.

While CEBPG may play a major role in controlling AO and/or DNAR protection in NBEC, in each individual a combination of any of tens or hundreds of different DNA sequence variations may be responsible for decreased correlation between CEBPG and the other (normally-regulated) genes. In preferred examples, risk associated alleles that affect function of many different genes and regions within genes can be detected through this analysis. In some examples, the TG/CEBPG ratio for a set of genes can quantify the effect of functionally significant variants on BC risk, e.g., providing a model for increased risk. In such examples , lack of correlation is associated with individuals indicating high or low (TG/CEBPG) ratios relative to the regression line observed for NBCI.

In some examples, a risk allele occurring in any of a collinear string of 50 bp in the bZip region of CEBPG increases risk. Although the prevalence of the rare allele for each nucleotide may be less than about 0.001, the chance of one of these rare alleles occurring in the 50 bp collinear string would be 50 x about 0.001 or about 0.05 which is consistent with the measured risk for BC among heavy smokers. Some examples of this disclosure allow identification of this risk, e.g., using a ratio of TG/CEBPG. Additional details of determination and application of such models are provided in the Examples below.

In some reference examples, the disclosure provides kits for diagnosing biological states, e.g., kits for identifying a cancer-related condition, e.g., BC, and/or a lung-related condition, e.g., COPD. Such a kit comprises a standardized mixture comprising: a competitive template for a transcription factor and a competitive template for at least one other gene, where the competitive templates are at known concentrations relative to each other. Such a kit can be used to determine expression levels of the transcription factor and/or other gene, and the expression levels computed to diagnosis the condition.

For example, in a kit for diagnosing a cancer-related condition and/or a lung-related condition, the standardized mixture can comprise a competitive template for CEPBG e.g., a competitive template comprising a nucleic acid sequence consisting of SEQ ID NO: 1 ± about 100 bases; and a competitive template for at least one other gene, the other gene being associated with the cancer-related condition and/or lung-related condition. In another kit for diagnosing a cancer-related condition and/or a lung-related condition, the standardized mixture can comprise a competitive template for E2F1, e.g., a competitive template comprising a nucleic acid sequence consisting of SEQ ID NO: 2 ± about 100 bases; and a competitive template for at least one other gene, the other gene being associated with said cancer-related condition and/or lung-related condition. The other gene can be selected from an AO and DNAR genes, e.g., XRCC1, ERCC5, GSTP1, SOD1, GPX1, ERCC1, CAT, GSTZ1, and ERCC2. For example, the competitive template for the other gene can comprise a nucleic acid sequence consisting of at least one sequence selected from SEQ ID NOS: 3-7 ± about 100 bases. In preferred examples , the kit can be used to test for risk for BC and/or COPD.

In some examples, the standardized mixture can further comprise primer pairs, e.g., for amplifying both the transcription factor (from the sample) and its competitive template and/or primer pairs for amplifying both the other gene (from the sample) and its competitive template. In some specific examples, the primer pair can be selected from a primer pair listed in Table 1.

Kits and methods described herein provide more accurate identification of those at risk for BC and/or COPD, compared to traditional methods. More accurate identification of those at risk for BC and inclusion of such individuals in chemoprevention and/or early detection studies can lead to improved efficacy. Those of skill in the art will recognize that the methods provided herein can be applied to the diagnoses of other cancer-related conditions and/or other lung-related conditions, e.g., other cancer-related conditions and lung-related conditions provided herein.

### B. Polymorphism Approach

In yet another aspect, the disclosure relates to methods and compositions for diagnosing a biological state using identified polymorphisms. In preferred examples, the identified polymorphism consists of a specific region of DNA that contains one or more nucleotide differences compared to controls. The nucleotide differences may differ from one subject to the next. However, one or more differences within the region are indicative of a given biological state.

### Diagnosis of Cancer-related Conditions and/or Lung-related Conditions

Some examples provide a method of identifying a cancer-related condition or a lung-related condition in a subject comprising obtaining a sample from a subject, where the sample comprises a nucleic acid region corresponding to relevant polymorphism, comparing the region to a nucleic acid sequence consisting of the sequence found in controls, and identifying a nucleic acid difference. In some examples, the sequence from controls is a consensus sequence, obtained, e.g., from a plurality of healthy individuals. In preferred examples, the sample obtained is a readily-accessible sample, e.g., one that can be obtained with a non-invasive or mildly-invasive technique. Such samples include, e.g., urine samples, bloods samples, semen samples, or more preferably saliva samples, buccal and/or nasal epithelial cell samples. For example, identified polymorphism(s) can allow diagnostic testing using more-readily accessible patient samples, such as peripheral blood and/or buccal smears

In some examples, the nucleic acid region is a 5' regulatory region of CEBPG; and this region is compared to the nucleic acid sequence (of controls) consisting of the 5' regulatory region of CEBPG ± about 10, ± about 50, ± about 100, ± about 150, ± about 200, ± about 500, ± about 800, or ± about 1000 bases, wherein a nucleotide difference indicates a cancer- or lung-related condition.

In some examples, the nucleic acid region is a 3' un-translated region of CEBPG; and this region is compared to the nucleic acid sequence (of controls) consisting of the 3' un-translated region of CEBPG ± about 10, ± about 50, ± about 100, ± about 150, ± about 200, ± about 500, ± about 800, or ± about 1000 bases, wherein a nucleotide difference indicates a cancer- or lung-related condition.

In some examples, the nucleic acid region is a bZip region of CEBPG; and this region is compared to the nucleic acid sequence (of controls) consisting of the bZip region ofCEBPG ± about 10, ± about 50, ± about 100, ± about 150, ± about 200, ± about 500, ± about 800, or ± about 1000 bases, wherein a nucleotide difference indicates a cancer- or lung-related condition.

In some examples, the nucleic acid region is a CEBPG recognition site; and this region is compared to the nucleic acid sequence (of controls) consisting of a CEBPG recognition site ± about 10, ± about 50, ± about 100, ± about 150, ± about 200, ± about 500, ± about 800, or ± about 1000 bases wherein a nucleotide difference indicates a cancer- or lung-related condition. in some examples, the CEBPG recognition site is the CEBPG recognitions site for XRCC1, ERCC5, SOD1, GSTP1 and/or GPX1.

In some examples, the comparison involves identifying at least one base in the nucleic acid region, e.g., using methods as known in the art and/or provided herein. In some examples, the region obtained form the sample and the sequence obtained from controls are compared by contacting the sample with a probe consisting of a the sequence obtained from controls (or a complementary sequence thereof) under conditions allowing hybridization; and
detecting whether or not hybridization occurs. Specificity of hybridization can be assessed by varying degrees of stringency of the hybridization conditions, as known in the art. Under suitable conditions, hybridization can indicate that the sample sequence is the same, sufficiently the same, significantly the same, or substantially the same as the control sequence, indicating lack of the condition or low risk thereof. Reduced hybridization can indicate that the sample sequence was different, sufficiently different, significantly different, or substantially different from the sample sequence (probe), indicating the condition or risk thereof. In addition, comparison of mismatch to perfect match oligonucleotide probes can be used to determine specificity of binding. In some examples, the cancer-related condition is bronchogenic carcinoma, a risk thereof or responsiveness to a chemotherapeutic agent. In some examples, the lung-related condition is COPD or a risk thereof.

In some examples, the nucleotide difference is a single nucleotide polymorphism. In some examples, the nucleotide difference comprises more than one base pair change either consecutively or at various locations within the region, e.g., two or more SNPs within the region. For example, the nucleotide difference can involve at least about two, at least about three, at least about 5, at least about 10, at least about 20, or at least about 50 nucleotide differences. In some examples, the nucleotide difference involves less than about 80, less than about 100, less than about 150, less than about 200, less than about 300 or less than about 500 nucleotide differences.

In yet other examples, more than one region can be compared to that of controls to diagnose a condition (or risk or prognosis thereof). For example, in identifying a cancer- and/or lung-related condition, the method can comprise identifying nucleotide differences in two or more nucleic acid regions selected from a 5' regulatory region of CEBPG, a 3' un-translated region of CEBPG; a bZip coding region of CEBPG; and a CEPBG recognition site of XRCC1, ERCC5, SOD1, GSTP1 and/or GPX1.

Some examples provide probes consisting of relevant nucleic acid sequences for identifying polymorphisms indicative of cancer- and/or lung-related conditions. Examples of four such probes include (i) a probe comprising a nucleic acid sequence consisting of a 5' regulatory region of CEBPG ± about 10, ± about 50, ± about 100, ± about 150, ± about 200, ± about 500, ± about 800, or ± about 1000 bases; (ii) a probe comprising a nucleic acid sequence consisting of a 3' un-translated region of CEBPG ± about 10, ± about 50, ± about 100, ± about 150, ± about 200, ± about 500, ± about 800, or ± about 1000 bases; (iii) a probe comprising a nucleic acid sequence consisting of a bZip region of CEBPG ± about 10, ± about 50, ± about 100, ± about 150, ± about 200, ± about 500, ± about 800, or ± about 1000 bases; and (iv) a probe comprising a nucleic acid sequence consisting of a CEBPG recognition site ± about 10, ± about 50, ± about 100, ± about 150, ± about 200, ± about 500, ± about 800, or ± about 1000 bases. One or more such probes can be anchored to a support, e.g., in an array, and/or provided in kits for use in identifying a cancer and/or lung-related condition (e.g., BC and/or COPD), risk thereof, predicted response to treatment, etc.

### III. Methods and Compositions for Treatment

Yet another aspect of the disclosure relates to methods and compositions for treating a biological state, e.g:, by providing an agent that "makes up" for the lack of correlation between a transcription factor and a gene. For example, some examples comprise administering a therapeutic based on the identified lack of correlation; and/or the identified biomarker or combination of biomarker(s). Generally, the present disclosure provides methods, pharmaceutical compositions, and kits for the treatment of animal subjects. The term "animal subject" as used herein includes humans as well as other mammals. The term "therapeutic" can refer to any agent that can be used to treat animal subjects.

The term "treating" as used herein includes achieving a therapeutic benefit and/or a prophylactic benefit. By therapeutic benefit is meant eradication or amelioration of the underlying disorder being treated. For example, in a BC patient, therapeutic benefit includes eradication or amelioration of the underlying cancer. Also, a therapeutic benefit is achieved with the eradication or amelioration of one or more of the physiological symptoms associated with the underlying disorder such that an improvement is observed in the patient, notwithstanding the fact that the patient may still be afflicted with the underlying disorder. For example, with respect to BC, treatment can provide therapeutic benefit when an improvement is observed in the patient with respect to other disorders and/or discomforts that accompany BC, such as cough, dyspnea, hemoptysis, and/or pso-obstructive pneumonia. For prophylactic benefit, a therapeutic may be administered to a patient at risk of developing a disease condition, e.g., BC and/or COPD, or to a patient reporting one or more of the physiological symptoms of such conditions, even though a diagnosis may not have been made.

### Treatment of Cancer-related Conditions and/or Lung-related Conditions

In some examples, the disclosure provides methods and compositions for treating a cancer-related condition, e.g., BC, and/or a lung-related condition, e.g., COPD. As indicated above, methods and compositions of the disclosure can be used in making a diagnostic decision and a therapeutic can be administered where indicated. Examples of therapeutics that can be administered, e.g., in treating a cancer-related and/or lung-related condition include, but at not limited to cis-platin, alkylating agents, such as busulfan, cis-platin, mitomycin C, and carboplatin; antimitotic agents, such as colchicine, vinblastine, paclitaxel, and docetaxel; topo I inhibitors, such as camptothecin and topotecan; topo II inhibitors, such as doxorubicin and etoposide; RNA/DNA antimetabolites, such as 5-azacytidine, 5-fluorouracil and methotrexate; DNA antimetabolites, such as 5-fluoro-2'-deoxy-uridine, ara-C, hydroxyurea and thioguanine; EGFR inhibitors, such as Iressa® (gefitinib) and Tarceva® (erlotinib); proteosome inhibitors; antibodies, such as campath, Herceptin® (trastuzumab), Avastin® (bevacizumab), or Rituxan® (rituximab). Other therapeutics which may be used include melphalan, chlorambucil, cyclophosamide, ifosfamide, vincristine, mitoguazone, epirubicin, aclarubicin, bleomycin, mitoxantrone, elliptinium, fludarabine, octreotide, retinoic acid, tamoxifen, Gleevec® (imatinib mesylate) and alanosine. See, e.g., U.S. 2005/0137213. Examples of therapeutics that can be administered, e.g., in treating a cancer-related and/or lung-related condition also include, but at not limited to, erlotinib, canertinib, cetuximab, ABX-EGF, trastuzumab, imatinib, SU11274, PHA665752, AP23573, RAD001, CCI-779, bevacizumab, vatalanib, bexarotene, bortezomib, flavopiridol, oblimersen, VEGF inhibitors, selenium, 15-PGDH and/or 15-PGDH activators (e.g., NSAIDs like indomethacin), P13K/Akt inhibitors (e.g., deguelin and *myo*-inositol), PPAR-γ and/or PPAR-γ activators (e.g., p21 up-regulators, E-cadherin up-regulators, gelsolin up-regulators, cyclins D and E down-regulators, MUC1 down-regulators, MMP2 down-regulators, and α5-integrin down-regulators), DNA methyltransferase-1 inhibitors, HDAC and methyltransferase inhibitors, prostaglandin E2 inhibitors, prostacyclin and/or prostacyclin activators, 5-LOX inhibitors, COX inhibitors, LOX-COX inhibitors, 12-LOX inhibitors, EGFR inhibitors, leukotriene A₄ hydrolase modulators, cyclooxygenase 1 modulators, antioxidants, caratinoids, retinoids, such as etretinate, isotretinoin, beta-carotene, fenretinide, anethole dithiolthione, 9-cis-retinoic acid, retinol, budesonide, alpha-tocopherol, retinyl palmitate, and the like. See, e.g., Hahn et al., Hematol Oncol Clin N Am 19: 343-367 (2005) and Hirsch et al, J. Clinical Oncology 23(14): 3186-3179 (2005). See also, e.g., Cohen et al., Cancer Control. 10:315-324 (2003) and Hong et al Science 278: 1073-1077 (1997).

Without being limited to a given design and/or theory, better understanding of AO and DNAR gene transcription regulation can enable design of improved chemo-preventive and chemo-therapeutic pharmaceuticals, as well as accompanying biomarkers that predict response. Performance of both classes of therapeutics can be affected by function of AO and/or DNAR genes. Developing pharmaceuticals that target regulation of AO and/or DNAR genes by CEBPG can be a productive avenue in many areas of health, including cancer prevention, cancer treatment, as well as inflammation and/or immunity.

Identified polymorphisms can also be used to develop therapeutics, e.g., therapeutics for treating and/or preventing cancer-related and/or lung-related conditions. For example, peptide molecules can be developed that have a therapeutic effect by interfering with or enhancing binding of transcription factors to hetero-dimeric proteins and/or to DNA recognition sites. See, e.g., Vassilev et al, Science 6(303): 8440848 (2004). One or more of such identified therapeutics can be administered where indicated, e.g., alone or in combination with other known therapeutics, e.g., other known therapeutics for treating cancer-related and/or lung-related conditions, e.g., as provided above.

### EXAMPLES

### Example 1

### Collection of NBCI and BCI Samples

Normal bronchial epithelial cell (NBEC) and peripheral blood samples can be obtained from patients and a portion of each sample can be used to in the Examples described herein. Individuals can be recruited from among patients who are undergoing diagnostic bronchoscopy. Some indications for bronchoscopy include coughing up of blood, chronic cough, pneumonia resistant to antibiotics, and need to remove a foreign body. Some of these patients may be diagnosed with bronchogenic carcinoma (BCI), while others may have non-neoplastic conditions (NBCI). The age of these patients may range from approximately 20 to approximately 90, with most participants being between the ages of about 60 and about 75. NBEC samples are obtained according to previously described methods. See, e.g, Benhamou S. et al., Carcinogenesis, 8: 1343-1350 (2002).

From each patient, NBEC samples and 20 ml of peripheral blood can be collected and processed, e.g., as previously described (Willey et al, 1997; Crawford et al, 2000). Approximately 10-15 brush biopsies can be obtained from normal appearing mucosa at approximately the tertiary bronchi. If the patient has a local pathological condition, such as pneumonia, trauma, or BC, the brushes can be taken from the opposite side.

After each bronchoscopic brush biopsy, the brush can be swirled in approximately 3 ml of ice cold saline to dislodge and retrieve the cells. Approximately 500,000 to 1 million cells can be obtained with each brush. Thus, 10 brushes can yield about 5-10 million cells. These cells in 3 ml of ice cold saline can be divided up for extraction of RNA and protein and preparation of slides for IHC and FISH. Approximately 5 million cells can be used for nuclear extract protein extraction (see, e.g., Dignam et al, Nucleic Acid Research 11: 1475-1499 (1983)). This can yield approximately 100 ug of nuclear extract for EMSA and Western hybridization analyses. Approximately 1 million cells can be used for RNA extraction for the expression level measurements.

The 20 ml of peripheral blood may contain approximately 1-2 x 10⁸ white blood cells. Most of these cells can be used to produce nuclear extracts for surface plasmon resonance (SPR) experiments described
below. About 1-2 million cells can be used for RNA extraction for expression level measurements, and another about 1-2 million can be used for DNA extraction for sequencing studies.

Buccal and nasal epithelial samples can also be collected from BCI and NBCI providing bronchoscopic brush samples and peripheral blood samples for SNPs. Buccal and nasal epithelial samples can be obtained by brushing of the inside of the mouth or the nose. The buccal epithelial cell samples from BCI and NBCI can be handled in a similar way as the NBEC samples, as provided above.

### Example 2

### (a) Identification of CEBPG and E2F1 as transcription factor biomarkers for BC

TF recognition sites common to GSTP1/GPX1, CAT/GPX3, and GPX3/SOD1 are identified through sequence analysis (Genomatix Software GmbH, Munich, Germany, http://genomatix.de/cgi-bin/eldorado/) (Quandt K, Frech K, Karas H, Wingender E, and Werner T, NAR, 23, 4878-4884. 1995), yielding sites for 11 TFs.

Standardized RT (StaRT)-PCR™ reagents (Willey JC, et al, in Methods in Molecular Biology (ed. Shimkets, R.A.) 13-41 (Humana Press, Inc., Totowa, N.J., 2004) are optimized for ten of these TFs, including CEBPB, CEBPE, CEBPG, E2F1, E2F3, E2F4, E2F5, E2F6, EVI1, and PAX5. Four TFs expressed at low and invariant levels among multiple NBEC samples are evaluated no further. The remaining six, CEBPB, CEBPG, E2F1, E2F3, E2F6, and EVI, are evaluated for correlation with an expanded group often AO and six DNAR genes. Accession numbers for these genes are provided in Table 1 below, along with competitive template sequences for each gene analyzed, forward and reverse primer pair sequences for amplification, the position on the gene at which the primers hybridize, and the size of the PCR-amplified products.

NBEC samples are obtained by bronchial brush biopsy during diagnostic bronchoscopy as detailed above. StaRT-PCR™ is used to generate virtually-multiplexed transcript abundance (VMTA) data (Workman J and Mark H, Spectroscopy, 19, 1-3, 2004). Details of this method, including extensive validation of the method in independent laboratories, were published recently. Willey JC, et al., in Methods in Molecular Biology (ed. Shimkets, R.A.) 13-41 (Humana Press, Inc., Totowa, N.J., 2004). Briefly, total-mRNA samples extracted from NBEC are reverse transcribed using M-MLV reverse transcriptase and oligo dT primers as previously described. Benhamou S. et al., Carcinogenesis, 8: 134301350 (2002). With StaRT-PCR™, an internal standard for each gene within a standardized mixture of internal standards (SMIS™) is included in each measurement. StaRT-PCR™ reagents for each of the measured genes, including primers and standardized mixtures of internal standards (SMIS™) are prepared according to previously described methods (competitive template and primer sequence information provided in Table 1 as discussed above).

VMTA values for the above 22 genes are measured in NBEC samples from 49 individuals including 24 NBCI and 25 BCI. Demographic data of patient providing NBEC samples are provided in Table 2 and VMTA data obtained is provided in Table 3. An internal standard controls for several known sources of variation during PCR, including inhibitors in samples. E.g., the presence of an inhibitor was the primary reason why it was not possible to obtain an E2Fl measurement in sample 147 (see Table 3).

Pearson analysis is performed on the normalized VMTA values for AO and DNAR genes and putative regulatory TFs. Data is provided in Table 4. In NBCI samples, the CEBPG TF is significantly (p < 0.01) correlated with eight of the 16 AO or DNAR genes, specifically XRCC1, ERCC5, GSTP1, SOD1, GPX1, ERCC1, CAT and ERCC2. In contrast, in BCI samples CEBPG is not correlated with any of the AO or DNAR genes tested in this example. Analysis of each VMTA value relationship with age is assessed with Pearson's correlation, with gender by t-test, and with smoking history by ANOVA followed by Duncan's test. All statistical tests are two-sided test and are performed using SAS version 8.0 (SAS Institute, Cary, NC).

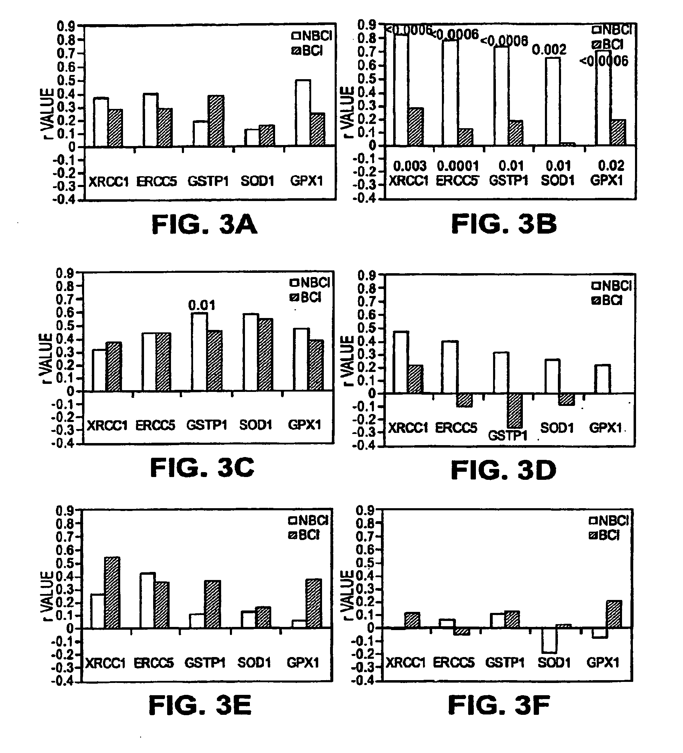

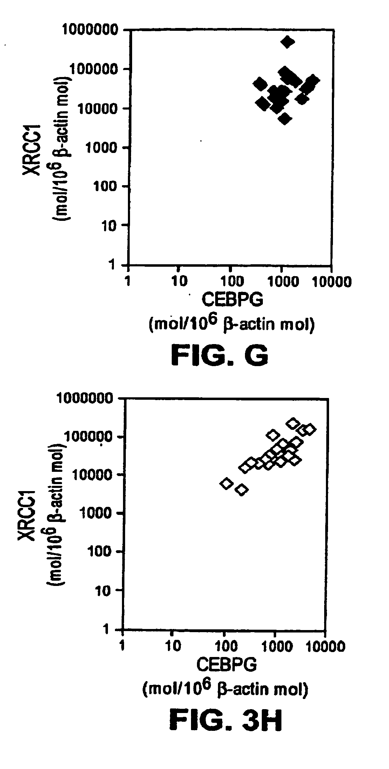

Figure 3 (a-f) illustrate correlation of each of 6 TFs ((a) CEBPB, (b) CEBPG, (c) E2F1, (d) E2F3, (e) E2F6, (f) EVI1) with each of 5 genes XRCC1, ERCC5, GSTP1, SOD1, or GPX1. Each panel presents the correlation coefficients (r values) for one TF in relation to each of the five genes. Correlation is determined by Pearson's correlation following logarithmic transformation. The transformation may be necessary due to the wide range of expression of each gene among the individuals. In Figure 3, the p value for each significant correlation is provided above the bar. Significance level is defined as (p < 0.01) following Bonferroni adjustment for multiple comparisons, specifically comparison of each of the six TFs to each of the AO or DNAR genes. Comparison for significant differences between pairs of correlation coefficients is done by Fisher's Z-transformation test. Workman J and Mark H, Spectroscopy, 19: 1-3 (2004).

For CEBPG, presented in 3(b), the difference in r value between NBCI and BCI is significant or nearly significant for each correlated gene, and the p value for each comparison is provided below the corresponding pair of bars. As Figure 3b illustrates, the correlation between CEBPG and each of XRCC1, ERCC5, GSTP1, and SOD1 was significantly lower in BCI compared to NBC1 and the difference was nearly significant for GPX1.

In NBCI, based on the r² values from Pearson's correlation analysis, CEBPG accounts for much of the variance in expression of XRCC1 (69%), ERCC5 (62%), GSTP1 (55%), SOD1 (44%), and GPX1 (52%). E2F1 accounts for some of the remaining variance. For example, in NBCI, E2F1 is correlated with GSTP1 (Figure 3c) and the correlation is lower in BCI. However, the difference in correlation between NBCI and BCI is not significant. Further, when samples from all 49 NBCI and BCI were assessed as a single group, E2F1 is significantly correlated with ERCC5, GSTP1 and SOD1 (see Table 4). None of the other TFs tested in this Examples are correlated with XRCC1, ERCC5, GSTP1, SOD1, or GPX1 (Figure 3a, d, e, f).

Figures 3(g- h) illustrate CEBPG/XRCC1 data from Figure 3b presented as scatter plots for (g) NBCI and (h) BCI. Scatter plots of the relationship between CEBPG and XRCC1 in NBCI or BCI are representative of the other four genes (ERCC5, GSTP1, SOD1 and GPX1). CEBPG, XRCC1, ERCC5, GSTP1, SOD1 and GPX1 are not significantly correlated with age, gender, or smoking history in NBCI, BCI, or the combined group.

### (b) Additional data Identifying CEBPG as a transcription factor biomarker for BC

Expression levels of 16 selected AO and DNAR genes are found to be correlated in bivariate analysis of 12 NBCI and not correlated in 15 BCI. The NBCI and BCI groups are closely matched for age, gender, smoking history, and disease status and comprise Study 2 in this Example. For any study, candidate genes can be identified through bivariate analysis of transcript abundance values for multiple AO, DNAR, XME, cell cycling, differentiation, apoptosis, and transcription factor genes an comparing results in groups of cancer and non-cancer individuals (e.g., Non-BC vs. BC Individuals).

The correlated genes are subjected to TF recognition site analysis. Specifically, the EI Dorado (Build 35) program from the Genomatix software package is used to search for TF recognition sites in regulatory regions of each of the 16 AO and DNAR genes. First, the software is used to locate the AO and DNAR genes within the genome and define 1101 base pairs of the promoter regions (1000 base pairs upstream of and 100 base pairs into the transcription start site) for each gene (Genomatix Software GmbH, Munich, Germany, http://genomatix.de/cgi-bin/eldorado/). The 1101 base pair sequences obtained from the EI Dorado program are then used as the target sequences for putative TF recognition site identification using the MatInspector Version 42 program (Genomatix Software GmbH, Munich, Germany, http://genomatix.de/cgi-bin/eldorado/). The parameters used are the standard (0.75) core similarity and the optimized matrix similarity. The TF recognition sites identified in each of the correlated AO and DNAR genes are included in the CEBP family, E2F family, EVI1, and PAX5.

StaRT-PCR™ reagents are prepared for each of the TFs in each of these families. VMTA data for each of the TFs that have recognition sites shared among these target genes were collected from the NBEC samples of the 12 NBCI and 15 BCI. Of 11 TFs evaluated, 8 are expressed in NBEC, including CEBPG, CEBPA, CEBPB, E2F1, E2F3, E2F6, and EVII.

The TFs are identified that share the pattern of correlation with target genes in NBCI, and loss of this correlation in BCI. The TF out of the eight assessed that had this characteristic was CEBPG. Each of the eight TFs and each of the 16 AO and DNAR genes can be assayed in an additional 12 NBCI and 13 BCI to test whether CEBPG is responsible for regulation of the 10 AO and DNAR genes in NBCI and loss of correlation in BCI. The 12 NBCI and 13 BCI comprise Study 3.

In Studies 2 and 3, the 24 NBCI had and average age of 55 while the 25 BCI had and average age of 68. There were 18 males and 7 females among the BCI, while there were 12 males and 12 females among the NBCI. An algorithm to predict BC risk based on cigarette smoking history, age, and gender was developed from the demographic information gathered as part of the CARAT study (see e.g., Bach et al, J Natl Cancer Inst. 95: 470 (2003)). Based on this algorithm, among the NBCl the average calculated BC risk is 2.2% (range 1-15%) while among the BCI the average calculated risk is 6.8% (range 1-15%). Thus, although the incidence of BC among the 25 BCI in Studies 2 and 3 is 100%, the incidence ofBC among individuals in the general population who have the same age, gender, and smoking history is only 6.8%. Ostensibly, the 25 BCI may be selected on the basis of genetically determined low protection against the oxidant and DNA damage stress posed by cigarette smoking.

As such, it can be predicted that CEBPG would a) be correlated with each of the 10 genes that it was correlated with in NBCI, b) not be correlated with the 10 genes in BCI, and c) not be correlated with the six genes that it was not correlated with in either NBCI or BCI. It can be further predicted that d) each of the other six TFs. assessed that were not correlated with the 10 AO or DNAR genes would demonstrate this pattern again.

### Results

Each of the above predictions is confirmed in a blinded analysis of VMTA data from Study 3 and further confirmed when VMTA data from Studies 2 and 3 are combined. The combined data from Studies 2 and 3 are presented in Tables 5-8. The data supporting the predictions are as follows:

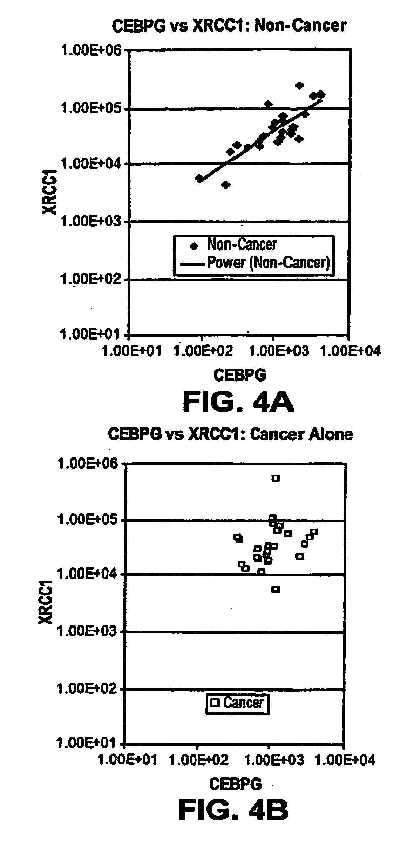

a) For bivariate analysis of CEBPG with each of the AO or DNAR genes, the mean correlation. coefficient and standard deviation in NBCI is 0.69 +/- 0.10 with average P value of 0.003 Table 5. An example of bivariate analysis between CEBPG and XRCC1 in NBCI is presented in Figure 4a.

b) For analysis of CEBPG with the 10 AO and DNAR genes in BCI the correlation coefficient is 0.23 +/- 0.13 with average P value of 0.36, also shown in Table 5. The bivariate plot of CEBPG with XRCC1 in BCI is shown in Figure 4b.

c) In bivariate analysis of CEBPG with each of the six genes that are not correlated in Study 2, there again is no correlation with CEBPG in Study 3 and the combined data are presented in Table 6.

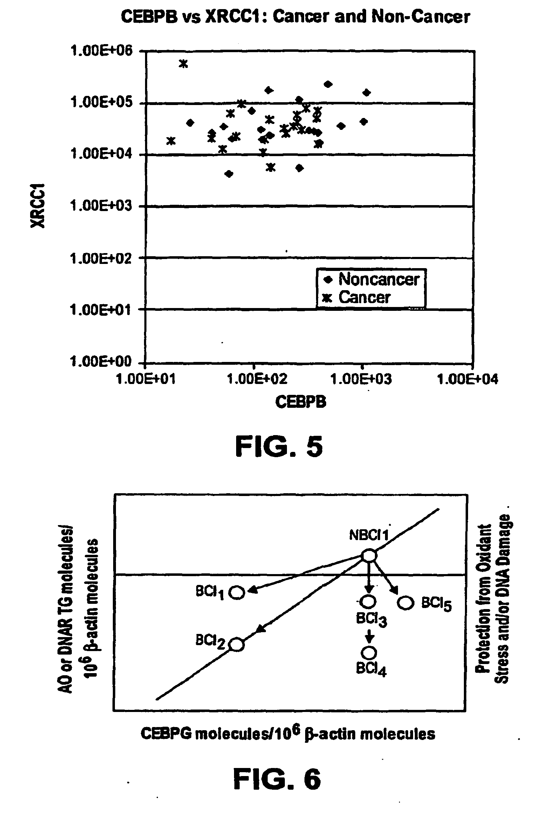

d) In bivariate analysis, none of the seven other TFs, including CEBPA, CEBPB, EVI1, E2F1, E2F3, E2F6, and MYC, demonstrate the pattern observed with CEBPG (i.e. significant correlation with the 10 AO and DNAR genes in NBCI and no correlation in BCI). These findings are represented by results from bivariate analysis of VMTA data for CEBPB with each of the 10 AO and DNAR genes, presented in Table 7. Figure 5 illustrate an example of the lack of correlation of CEBPB with XRCC1 in either NBCI or BCI.

e) Bivariate analysis of expression levels for each of the genes versus age, gender, and recent or cumulative smoking history reveals no correlation. Thus, there is little or no evidence that cigarette smoking affects
regulation of the AO and DNAR genes included in this study. This is important because it can remove a potentially strong, confounding variable that may be difficult to control.

### Additional data relating to E2F1 as a transcription factor biomarker for BC

Of the eight TFs assessed, E2F1 is the only TF other than CEBPG to be significantly correlated with the 10 AO and DNAR genes. Results from bivariate analysis of E2F1 with each of the 10 genes are presented in Table 8. The differences compared to results presented in Table 5 for CEBPG are a) the mean correlation coefficient of bivariate analysis between E2F1 and each of the 10 genes in NBCI (0.49 +/- 0.11) is lower than that observed for CEBPG, b) the mean correlation coefficient for analysis of E2F1 with the 10 genes in BCI is 0.46 +/- 0.11 which is not significantly lower compared to NBCI. These results suggest that E2F1 contributes to regulation of the 10 AO and DNAR genes, and that E2F1 may not play a role in the decreased correlation among the 10 genes in BCI compared to NBCI.

### Example 3

### Identification of a model distinguishing NBCI from BCI

Models that distinguish NBCI from BCI are derived from multivariate analysis and genetic programming software analysis of the VMTA data from a training set of individuals. These models are assessed using VMTA data obtained from a test set of an additional 25 NCBI and 25 BCI, matched for age, gender, and smoking history. Suitable models are refined through analysis of additional genes in the training sets and validation in additional sets.

Where possible, triplicate expression level measurements of each gene are performed. All tests can be performed for the NBCI group alone, the BCI group alone, and the combined groups. Student's t tests are performed to identify statistical differences between NBEC from NBCI and BCI for each gene. Statistical significance is set at p< 0.05. All statistical analyses can be performed using SAS version 6.11 (SAS Institute, Cary, NC).

To confirm statistically significant inter-individual variation in gene expression levels, a one-factor ANOVA is performed. Pearson's correlation test is used to identify significant bivariate correlation between pairs of genes. TG/CEBPG ratio for each gene for each BCI is assessed relative to the confidence limits established for NBCI by Person's correlation test. A cut-off value is identified based on the regression line from bivariate analysis of VMTA data from NBCI. This model is evaluated in a blinded study for its accuracy in determining whether an individual is in the NBCI or BCI group.

The overall concept is that lack of correlation of CEBPG with each AO or DNAR gene is characteristic of NBEC from individuals at risk for BC. High or low (TG/CEBPG) ratios relative to the regression line observed for NBCI indicates lack of correlation and accordingly risk ofBC. Due to the high correlation between CEBPG and TGs in NBCI, it is possible to determine with meaningful confidence the regulated gene expression level predicted to accompany the CEBPG level in NBEC from a particular NBCI. The TG/CEBPG ratio for selected AO and DNAR genes can then be used to predict risk-conferring polymorphic alleles in each individual.

If the TG/CEBPG ratio is above or below a particular level, determined from analysis of the TG/CEBPG for NBCI, a polymorphism that increases risk is indicated. In any particular BCI individual, who by definition is at high risk, the TG/CEBPG for a particular gene may be high, low, or unchanged relative to the regression line observed in NBCI (e.g. Figure 4 CEBPG vs XRCC1). That is, regulation of many of the TGs by CEBPG may be normal in a particular BCI. However, for any particular individual at increased risk for BC, altered regulation of a sufficient number of TGs is expected. This provides models for distinguishing BCI from NBCI.

Upon identifying models that distinguish BCI from NBCI based on expression levels measured in NBEC, the models are assessed in other tissues, e.g., tissues obtainable by non-invasive techniques, including, e.g., buccal and/or nasal epithelial cells. Bivariate correlation patterns observed for NBCI and BCI in NBEC may also be observed in these other tissues. About 50 buccal epithelial samples and peripheral blood cell samples are collected from the same patients providing NBEC. Expression levels are determined for use with the models. The data are compared to data obtained for NBEC and to SNP analysis of peripheral blood cells from the same patients.

### Example 4

### (a) Detection of BC by identifying Loss of Correlation

Discriminate analysis of VMTA data for all 22 genes from Example 2a can be conducted to identify models that identify each individual as NBCI or BCI. Using 36 of the 49 individuals as a training set (19 NBCI and 17 BCI), the best models involve an interaction between CEBPG and one or two other genes. The six best models are then evaluated in a blinded validation set of 6 NBCI and 7 BCI, matched for age, gender, and smoking history. The best model correctly identifies 10/13 individuals as NBCI or BCI, providing 77% accuracy, 100% specificity and 70% sensitivity. The models identified through linear discriminant analysis of VMTA data from NBEC can have sufficient accuracy for the purpose of identifying individuals at risk for BC to improve efficacy of chemoprevention and early detection clinical trials. For example, even 70% specificity is not surprising given that some individuals at risk would not have yet developed BC.

### (b) Additional data for Detecting BC by identifying Loss of Correlation

Multi-variate analysis of the VMTA data for CEBPG and the 10 genes from Example 2b is conducted to identify models that distinguish NBCI from BCI. Analysis is done initially on VMTA data from 34 of the 50 individuals (17 NBCI and 17 BCI) and this yields several models that are 100% accurate. These models can be evaluated using the remaining blinded 16 individuals (8 NBCI and 8 BCI). Several of the models involving CEBPG were at least 75% accurate for distinguishing NBCI from BCI.

### Example 5

### Identification of Polymorphisms

This example describes identification of polymorphisms in the correlated TF, AO and/or DNAR genes for which certain alleles are represented at a significantly higher rate in BCI.

Recognition sites for CEBP and E2F families, PAX5, and EVII are identified as common to the regulatory regions of AO and DNAR genes that are correlated in NBEC of NBCI. These common TF recognition sites are identified through regulatory region sequence analysis with Mattinspector™ software. Of the eight TFs that could bind to the above recognition sites, CEBPG TF expression levels are found to be correlated with expression levels of 10 selected AO and DNAR genes in NBEC of NBCI but not in NBEC of BCI. E2F1 expression levels are correlated with expression levels of AO and DNAR genes in both NBCI and BCI, such genes including GPX1, CAT, GSTZ1, mGST1, SOD1, GSTP1, ERCC1, ERCC2, ERCC5 and XRCC1.

CEBPG, CEBPA, CEBPB, FOS and the 10 correlated AO and DNAR genes can be analyzed for sequence variants. Polymorphisms assessed with priority are those that could affect transcription regulation, protein function, post-transcriptional processing and/or stability, and/or protein-protein binding, including those in the upstream regulatory region, and those in the 3' UTR, translated region, and 5' UTR of the coding region. Initially, groups that are likely to have a maximal difference in genetically determined risk can be compared, including older, heavy smoker NBCI on the one hand and younger, light or non-smoker BCI on the other. Using SNP Consortium databases, polymorphisms, e.g., SNPs, are identified in the 3' untranslated, translated, 5' untranslated, and regulatory regions of correlated genes and of CEBPG and E2F1.

DNA extracted from peripheral blood WBC can be sequenced through a commercial service. Because the C/EBP genes are several thousand bp long, the regions with known function can be assessed with greatest priority. These high priority regions include those responsible for DNA binding, heterodimer formation, and activation, and the 3' UTR. Most of the known SNPs in these genes are in the 3' UTR which may play a role in transcript stability and/or processing (e.g. polyadenylation) See, e.g., Conne, et al, Nature Medicine 6(6): 637-41 (2000).

CEBPA, B, and G proteins are assessed for known SNPs using bioinformatics software available through NCBI. The list of known SNPs is provided below in Table 9. It is evident from this table that most SNPS in CEBPG and A occur in the 3' untranslated region (UTR). For each of the three C/EBP's, there are no known SNPs in the regulatory region or coding region. Although no common SNPs are known in these regions, it is likely that numerous uncommon polymorphisms, e.g., SNPs, exist in the population (Mohrenweiser et al Toxicologic Pathology 32: 1336-45 (2004)) and that a change in any of several nucleotides at a sensitive region can lead to altered function. For CEBPG, A, and B, and their isoleucine heterodimer partners including FOS, the regions of particular interest are those that participate in transactivation, heterodimer formation and/or DNA binding. CEBPG is truncated and lacks the activating domain (Cooper et al, Nucleic Acids Res. 23: 4371-4377 (1995)). However, it binds DNA with the same affinity as the full CEBP proteins (Roman et al, Gene Dev. 4: 1404-1415 (1990)). Therefore, for CEBPG the focus is directed to the bZip region responsible for heterodimer formation and DNA binding.

For the target genes, the primary interest is in the regulatory regions, specifically the regions containing recognition sites for CEBPG. For example, the known SNPs in the 1100 bp regulatory region (1000 upstream and 100 bp downstream of the transcription start site) of XRCC1 are presented in Table 10. The frequency of each allele at each polymorphism in NCBI can be determined and compared to frequency of each allele at each polymorphism in BCI. Below are three of many scenarios that can exist in a particular individual to explain the presumed 5-10% incidence of genetically determined increased risk.

(i) Each of five co-regulated genes contributing to risk has a polymorphism in the recognition site for CEBPG. The prevalence of the risk allele is 0.5 in each recognition site. 0.5 x 0.5 x 0.5 x 0.5 x 0.5 =0.5 x .0625 = 0.0312. If the incidence of the polymorphism for any of them is less than 0.5 or if the low expression phenotype requires homozygosity for any of the genes, the frequency of the phenotype will be less than this.

(ii) There is a polymorphism in a recognition site for a transcription factor that controls all five genes. Either the frequency is 0.25 and homozygosity is required (0.25 x 0.25 = 0.052) or the frequency is 0.05 and heterozygosity is responsible (0.05 x 0.95 = 0.0475).

(iii) As reported by Mohrenwiener (2004), there are many low frequency polymorphisms that affect AO and/or DNAR function. For example, a risk allele occurring in any of a collinear string of 50 bp in the bZip region will increase risk. Although the prevalence of the rare allele for each nucleotide may be less than 0.001, the chance of one of these rare alleles occurring in the 50 bp collinear string would be 50 x 0.001 or 0.05.

Because variation in a different nucleotide may be responsible for the altered AO and/or DNAR function in each individual, among the BCI there may be a higher overall prevalence of rare alleles throughout a functional region of interest compared to the rest of the gene, and this difference may not be observed among NBCI. For example, there may be higher prevalence of rare alleles among the BCI through the bZip region of CEBPG compared to the rest of the CEBPG, while among NBCI there is no difference. Data can be evaluated for significant (p < 0.05) differences with one-way ANOVA.

In assessing high numbers of polymorphic sites in a relatively low number of individuals, a pattern at a polymorphic site may appear to be associated with BC patients by chance. This can be controlled for by initially considering each polymorphic site pattern to be associated with BC as a model. Each of these models can be validated in a subsequent blinded study, e.g., as detailed above.

A power analysis is conducted based on allele frequencies of known SNPs in the correlated TF, AO and/or DNAR genes. A suitable sample size for each gene is calculated assuming an allele frequency of 5% in one group and 95% in the other group. To achieve a power of at least 80%, 5 subjects per group are used. For example, for 10 SNPs, (e.g. one for each gene), 100 subjects are use. Power calculation can be carried out using the Fisher Exact Procedure on nQuery 5.0. Where additional AO and/or DNAR genes that are regulated by CEBPG and/or that contribute to protection are not assessed, sensitivity may be reduced (e.g., high false negatives may be observed), but specificity may not be affected (e.g., false positives remain low).

### Example 6

### Identification of Polymorphisms by Exploring Mechanistic bases for correlation ofAO and DNAR genes with CEBPG in NBCI but not in BCI

The mechanistic bases for correlation of AO and DNAR genes with CEBPG in NBCI but not in BCI can be explored to further identify polymorphic regions indicative ofBC. Established technology can be applied to assess transcript regulation in primary cells/tissues. Measuring transcription regulation can involve three components: (a) VMTA measurements target genes regulated by the TF, (b) analysis if DNA recognition site sequences for the TF that reside in the regulatory region of target genes, and (c) analysis of TF interaction with each recognition site. VMTA data generated by StaRT-PCR™ can be used to measure expression levels of target genes for (a); DNA sequencing and other methods are readily available for high throughput analysis of SNPs for (b); and established EMSA, SPR, and Western blot methods can be used for (c). Due to the small size of NBEC samples obtainable by bronchoscopic brush biopsy, more sensitive methods, e.g., Standardized ImmunoPCR (SiPCR) is preferred. See, e.g., U.S. Patent Application Serial No. 11/103,397. These methods can be used for measuring affinity of TFs for particular DNA sequences, and affinity between different putative TF heterodimer proteins to the small NBEC samples obtained by bronchoscopic biopsy.

Loss of correlation may be due to direct or indirect regulation of TGs by CEBPG. For example, CEBPG expression levels may be correlated with expression levels of AO and DNAR genes, but CEBPG may not regulate them, for example, if a) it is a co-factor necessary but not sufficient for transfection of other genes, or b) it is co-regulated by the same TFs as the co-regulated AO genes, but has no effect on them. To confirm that CEBPG is regulating the AO and DNAR genes, a CEBPG expression vector is introduced into cells that express low levels of CEBPG and a suitable target gene. Cultured NBEC from certain individuals may be suitable for this purpose. See, e.g., Willey et al., Am J Respir Cell Mol Biol. 19(1):6-17 (1998). Also, a reporter construct can be transfected containing the regulatory region for ERCC5 into bronchogenic carcinoma cell lines that express varying endogenous levels of CEBPG. A list of measurable biological correlates to SNPs that have different functional affects is presented in Table 11.

Figure 6 illustrates a schematic bivariate analysis ofTG/CEBPG expression levels in one NBCI (NBCI1) and 5 BCI (BCI₁₋₅). In this schematic, AO or DNAR expression levels are highly correlated with CEBPG expression levels in NBCI individuals. Thus, the bivariate coordinates for NBCI, including individual NCBI1, fall along the thick regression line. The thin horizontal line represents the TG expression level adequate to protect against oxidant and DNA damage stress that occurs in a heavy cigarette smoker. In NBCI, the correlation between
AO or DNAR target gene expression level and CEBPG expression level, e.g., due to regulation by CEBPG, is associated with NBEC protection from oxidant and DNA damage stress (NBCI1 coordinates occur above the thin horizontal line).

In BCI, represented by BCI₁₋₅, there are reduced TG expression levels, indicated by the arrows. Further, there is low or absent correlation between TG and CEBPG expression levels and most of the bivariate coordinates are represented as not being along the regression line. This schematic represents the hypothesis that a) CEBPG regulates transcription of key AO and DNAR TG and decreased correlation is accompanied by reduced protection from oxidant and DNA damage stress and b) because BCI are selected on the basis of reduced protection, they are likely to manifest reduced correlation between CEBPG and TG expression levels in their NBEC.

Whether an individual BCI CEBPG expression level is lower or greater than that in NBCI can indicate possible mechanisms for loss of correlation and thus regions to be analyzed for polymorphism indicative of disease risk. Whether an individual BCI TG/CEBPG ratio falls above, on, or below the regression line can also indicate possible mechanisms for loss of correlation and thus the regions to be analyzed for polymorphisms indicative of disease risk. Table 12 illustrates individual BCI TB/CEBPG data falling above, on, or below an NBCI regression line. Possible mechanisms for decreased correlation include reduced CEBPG transcription and/or reduced functional interaction between CEBPG and TG regulatory regions:

### Reduced CEBPG transcription

In BCI₁ and BCI₂ there is lower CEBPG transcription, resulting in a TG expression level below that adequate for protection against the AO/DNA damage experienced by cigarette smokers. Variation in TG expression level between BCI₁ and BCI₂ may be due to other TFs that regulate TG being induced by stress to different degrees in different individuals. In BCI with reduced CEBPG expression levels, the regulatory region of CEBPG is analyzed for polymorphisms different from NBCI, e.g., that might affect affinity of TFs for recognition site, e.g., as discussed in more detail below.

### Reduced Functional Interaction between CEBPG and TG Regulatory Region

In BCI₃, BCI₄, and BCI₅ the TG expression levels are below that necessary to provide adequate protection against AO and DNA damage stress in heavy cigarette smokers even though CEBPG expression levels are (substantially) the same or higher than that in NBCI1. For example, the CEBPG expression levels are the same as in an individual with higher CEBPG function in BCI₃ and BCI₄. The CEBPG expression levels are higher in BCI₅, e.g., due to feedback signals that insuffcient protection is present In BCI₃, levels of non-CEBPG TFs that regulate the TG are higher than in BCI₄. In each situation, the TG expression level achieved is inadequate to provide adequate protection. In BCI with (substantially) the same or higher CEBPG expression levels, polymorphisms associated with lower function of CEBPG compared to NBCI are searched for, e.g., as discussed in more detail below.

BCI with TG/CEBPG ratios similar to BCI₁, BCI₂ or BCI₃₋₄ are identified for each of the 10 AO or DNAR TGs, as provided in Table 12. According to the mechanisms described herein, BCI with ratios the same or similar to BCI₁ or BCI₂ have polymorphisms that cause reduced transcription of CEBPG relative to NBCI1, while those with ratios the same or similar to BCI₃₋₅ have polymorphisms that cause reduced function of CEBPG. These hypotheses are tested by evaluating BCI with characteristic TG/CEBPG ratios as described below:

### BCI with TG/CEBPG above the NCBI Regression Line

BCI 061102 (Table 12) is analogous to BCI₁ (Figure 6) in that TG/CEBPG ratio falls above the NBCI regression line. As stated in Table 12, the increased TG/CEBPG ratio may be due to is decreased rate of synthesis and/or decreased stability of CEBPG transcripts. Decreased transcription of CEBPG can occur due to a
Polymorphism involving the regulatory region of CEBPG or affecting the function of a TF that regulates CEBPG. Decreased stability may be associated with a polymorphism in the 3' untranslated region (UTR).

In BCI with increased TG/CEBPG values (e.g., BCI 061102), the regulatory region of CEBPG is analyzed for polymorphisms different from NBCI, e.g., that might affect affinity of TFs for recognition site. Where differences are observed, the sequences are synthesized and assessed for affinity with TF (purchased from commercial source) by SPR analysis.

In BCI with.increased TG/CEBPG values (e.g., BCI 061102), the 3' UTR of CEBPG can be analyzed for polymorphisms different from NBCI that account for reduced stability. Reduced stability can be measured by nuclear run-on assays as known in the art.

Also, the function of TFs that regulate CEBPG can be studied, e.g., by transiently transfecting a luciferase reporter construct containing regulatory region of CEBPG into PMNs of NBCI vs BCI.

### BCI with TG/CEBPG below the NBCI regression line

BCI 010902 (Table 12) is analogous to BCI₃₋₅ (Figure 6) in that TG/CEBPG ratio falls below the NBCI regression line. For BCI 010902, TG/CEBPG is significantly reduced relative to the NBCI regression line for 8 of the 10 AO or DNAR TG assessed. For example, the ERCC5/CEBPG ratio predicted from the NBCI regression line is 61, but the value for BCI 010902 is 5.

In some BCI with TG/CEBPG below the NBCI regression, this may be due to a polymorphism (e.g., an SNP) in CEBPG, or in a gene that forms a heterodimer with CEBPG, such as CEBPA, CEBPB, or FOS. In this scenario, the binding efficiency of CEBPG for the CEBP recognition site is less than it is in NBCL To test this, CEBPG, CEBPA, CEBPB, and FOS are isolated from peripheral blood cells of BCI that have TG/CEBPG values like BCI 010902. This is done by first purifying the TF using sequence-specific oligo bound to biotin, mixing with avidin metallic beads then using magnetic separator (Kroeger et al, Analytical Biochemistry 250: 127-129 (1997)). SPR is then used to assess affinity of CEBPG for the CEBP recognition site in the TG and affinity of CEBPG for the recognition site in the presence of CEBPA or CEBPB according to established methods (see, e.g., Rutigliano et al, Int J Oncol 12(2): 337-43 (1998); Linnell et al., J Biol Chem 275: 12231-12236 (2004)). These results are compared to those obtained from NBCI samples for which the TB/CEBPG ratio is on the NBCI regression line.

In BCI with reduced TG/CEBPG that show reduced CEBPG binding efficiency, CEBPG, CEBPA, CEBPB and/or FOS can be analyzed for polymorphisms different form NBCI, e.g., polymorphisms associated with reduced transcription of TG by CEBPG.
In some BCI with TG/CEBPG below the NBCI regression, this may be due to a polymorphism (e.g., an SNP) in the CEBPG recognition site for each of the affected TGs. To test this, recognition sites of the TGs from such BCI are analyzed for polymorphisms different from NBCI. Affinity of CEBPA, B, or G extracted from NBCI or BCI NBEC can also be compared for affinity with the recognition sites with or without the polymorphism(s). Purified CEBPA, B, and G are commercially available (e.g. Abcam, Abnova, or Active Motif) and can be obtained to establish and calibrate the SPR measurement method, e.g., as described above. Standardized immnoPCR, referred to above, can quantify the concentration of bound or free CEBPG in relationship to target gene expression level, in relationship to a recognition site of interest within the small primary NBEC samples available from bronchoscopy. For example, standardized immuno-PCR reagents can be developed for CEBPA, B, G, and FOS, e.g., to enable standardized, numerical measurement of each of the TFs, with lower detection threshold (e.g., less than about 50 molecules in some embodiments, as opposed to greater than about 10 million molecules for Elisa or EMSA). This can enhance understanding of these important interactions among the genes that regulate protection of NBEC from oxidants and DNA damaging agents.

Example 7

Polymorphisms in ERCC5. Sequencing of the ERCC5 regulatory region in Non-BC individuals (NBCI) and BC individuals (BCI) revealed two polymorphic sites in a YY1 transcription factor recognition site in the 5' upstream regulatory region that provide a likely specific cause of lack of correlation of ERCC5 and CEBPG in BC individuals. Specifically, at a previously known polymorphic site in ERCC5 (-222 relative to the transcription start site) the more frequent allele (G) was significantly more prevalent in BC individuals and associated with lower ERCC5 than expected. At a previously unknown site (-228), a rare allele (G) occurred in the only Non-BC individual with lower ERCC5 than expected. (-222 is Position 102296199 on contig. NT_009952.14; ref SNP ID of rs751402; -228 is at position 102296193 on the same contig.).

Sequence analysis of the ERCC5 regulatory region in 11 BC and 12 Non-BC revealed two polymorphic sites and they were both in the YY1 repressor recognition site. Further confirmation can be provided, by sequencing ERCC5 in 110 Non-BC and 110 BC individuals to confirm that the ERCC5 -222 G allele is represented at a significantly higher rate in BC Individuals. Power analysis based on the data indicates that collecting sequence information for this number of individuals will enable demonstration of significant difference at p value of 0.05 and 80% power. Demonstration of significantly higher prevalence of the ERCC5 -222 G allele in BC individuals indicates that individuals with this allele were selected for BC on the basis of increased risk.

ERCC5:229; A common polymorphism in YY1 recognition site with a MF allele (G) frequency of 75% in 12 Non-BC (18/24 alleles) and 91% in 11 BC (20/22). Conversely, the frequency of the LF nucleotide allele (A) in Non-BC individuals was 25% (6/22), while in BC individuals it is 9% (2/22). Further, the one BC individual that the LF allele occurred in had a normal position for ERCC5 relative to the Non-BC CEBPG/ERCC5 regression line. Thus, among five BC individuals with low ERCC5 relative to Non-BC regression line the frequency of the A allele was 0% (0/10). With these allele frequencies, the frequency of heterozygosity or homozygosity for LF allele is 17 % in BC and 44% in Non-BC (a 2.6 -fold difference) compared to an expected frequency among all individuals in a population of 36%. Previous studies, illustrate that among all 21 individuals studied the frequency for the MF allele was 83%, which is close to the 80%.

ERCC5:222; A rare polymorphism in YY1 recognition site. The LF allele was observed in one allele out of 46 evaluated (frequency of 0.02). Certain YY1 recognition site polymorphisms were associated with low ERCC5 expression relative to the regression line generated by bivariate comparison of ERCC5 to CEBPG among Non-BC individuals. For individuals with ERCC5:228 MF allele (GG) homozygosity. 31% (5/16) (including BEP2D) of all individuals and 100% (4/4) [56% or 5/9] ofBC individuals with ERCC5 below regression line had GG homozygosity. The one non-GG individual below the regression line had ERCC5:222 LF allele. 14% of non-GG ERCC5:228 individuals (1/7) had ERCC5 below the regression line. Excluding the single individual with ERCC5:222 LF allele, 0/6 [0/4] with non-GG ERCC5:228 allelotype had ERCC5 below regression line. The single ERCC5:222 LF allele observed occurred in the one Non-BC with ERCC5 below the regression line.

ERCC5 Regulatory Region Deletion Analysis. In H23, deletion through MYB leads to more than 2-fold increased ERCC5-LUC. Further deletion through CEBP1, E2F1, and CEBP2 does not lead to decreased ERCC5-LUC. However, deletion of ELK1 and EVI1 does lead to more than 4-fold reduction beyond the baseline level. Exogenous CEBPG transfection of CMV-CEBPG expression vector into H23 induces ERCC5-LUC 2.5-3.5-fold above the endogenous level.

The data indicates that CEBPG, ELK1, and YY1 each play a role in regulating ERCC5 expression in BEC from Non-BC individuals. The evidence for CEBPG is that there is a correlation between CEBPG expression and ERCC5 expression among 20 Non-BC individuals, and that transfection of CMV-CEBPG into H23 increases
expression of luciferase from ERCC5-LUC by 2.5-3.5-fold. The evidence for ELK1 is that when it is deleted, transcription from ERCC-LUC regulated by endogenous transcription factors decreases markedly. The evidence for YY1 is that there is increased frequency of ERCC5:228 MF (G) allele among BC individuals; increased frequency of ERCC5:228 MF (G) allele or ERCC5:222 LF allele (A) allele among individuals with ERCC5 expression below the regression line generated from bivariate analysis of ERCC5 and CEBPG in Non-BC individuals.

Inheritance of ERCC5:YY1 228 GG or ERCC5:YY1 222 allelotype is associated with ERCC5 transcription regulation that sub-optimally protects BEC from DNA damage. ERCCS is below the Non-BC ERCC5 vs CEBPG regression line in some individuals with one of these allelotypes. (6/16, 38%) [6/17 or 35%]. ERCC5 is not below the regression line in any individual without one of these allelotypes (0/7) [0/6]. The finding that the ERCC5:222 LF allele occurs in an individual with heterozygosity at ERCC5:228 indicate that having both risk determining alleles is high risk for an individual and they are selected against (there was a less than 1:3 chance for this because among non-selected individuals, only 30% are heterozygous or homozygous for ERCC5:228 LF allele). Such an individual may have excessively suppressed ERCC5 and die prior to reproduction. The decrease in heterozygosity or homozygosity of LF allele from 46% [45%] in non cancer to 17% in cancer is consistent with the hypothesis that for regulatory control of some genes, an allele at a polymorphic site that increases resistance to lung cancer may occur in a minority of individuals (e.g.ERCC5:228 LF allele), while an allele at another polymorphic site that increases resistance may occur in a majority of individuals (e.g. ERCC5:228MF allele). The data that 31% of ERCC5:228 MF homozygous (GG) individuals are below the Non-BC ERCC5/CEBPG regression line while 0/6 [1/7] ERCC5:228 heterozygous or LF homozygous individuals are below regression line supports the hypothesis that the MF homozygous allelotype is more likely to be associated with ERCC5 below the regression line and that this is one factor that contributes to lack of correlation between CEBPG and ERCC5 expression in BC individuals.

ERCC5:228 GG allelotype causes ERCC5 to be below the regression line because it causes YY1 to be a stronger repressor of CEBPG and/or ELK1. This allelotype in ERCCS may have survival advantage during age of reproduction due to some other effect, but increases the risk of lung cancer due to sub-optimal ERCC5 transcription regulation in bronchial epithelial cells. Accordingly, the fortunate few with the ERCC5:229 heterozygosity or LF homozygosity and ERCC5:222 MF homozygosity are at reduced risk.

ERCC5 in BC individual 255, and normal ERCC5 in BC individual 99 indicates an alteration in YY1 expression or function. If ERCC5 is not below the regression line, it also could be because CEBPG is low and ERCC5 expression is dominated by other transcription factors or YY1. Low ERCC5 in those with ERCC5:228 GG allelotype or ERCC5:222 AT allelotype indicates that these sequences are permissive of sub-optimal regulation. Putatively, the ERCC5:228 GG allelotype binds the YY1 transcription factor in a way that more effectively downregulates ERCC5, and this combined with less than optimal CEBPG function (e.g. due either to polymorphism in CEBPG or in heterodimer with CEBPG, CEBPZ, or Jun or other) would lead to low ERCC5 relative to regression line.

The ERCC5:222 AT allelotype binds the YY1 transcription factor in a way that more effectively down-regulates ERCC5, and this combined with less than optimal CEBPG function (e.g. due either to polymorphism in CEBPG or in heterodimer with CEBPG, CEBPZ, or Jun or other) would lead to low ERCC5 relative to regression line. In NC individuals, all but the individual with ERCC5:222 heterozygosity have ERCC5 along the regression line, regardless of whether they are ERCC5:228 MF homozygous or heterozygous. Although in ERCC5:229 MF homozygous individuals CEBPG regulation may be sub-optimal, this is overcome by appropriate compensation by optimal YY1 transcription regulation and function, and/or other compensatory responses. The
deletion analysis data indicate, in the absence of CEBPG, ELK1 is the predominant upregulator of ERCC5 expression.

The allelotype data from sequence analysis indicate that YY1 is a downregulator, which downregulation can operate through MYB, CEBPG, ELK1 or some combination. For example, YY1 may interact with the nearest sites, specifically CEBPG and/or ELK1. In cells that express high levels of CEBPG, ERCC5 expression can be modified by YY1 and CEBPG through some interaction, which may also involve interaction with ELK1. Thus, polymorphisms in YY1 result in regulatory modification by CEBPG. The presence of ERCC: 229 homozygous G can by itself indicate an individual is at risk, or can be combined with one or more other markers to indicate risk. However, heterozygosity or AA homozygosity indicates protection from risk. Further, a detectable increase in risk will be determined by a threshold number of risk allelotypes in DNA repair and/or antioxidant genes. This threshold can be reached by combining the AG or AA allelotype with particular polymorphisms in regulatory and/or coding regions of XRCC1, SOD1, GSTP1, GPX1 and/or CAT1.

In order to discover and quantify the altered transcript abundance profiles in BC individuals, it was necessary to develop suitable transcript abundance measurement methods and this was the motivation for developing Standardized RT (StaRT)-PCR. The excellent performance of StaRT-PCR in measuring transcript abundance is clear in a manuscript accepted for publication in Nature Biotechnology (Caneles et al, in press). In this MicroArray Quality Control (MAQC) project, coordinated by the FDA, StaRT-PCR was directly compared to Taqman Gene Expression Assays and Quantigene (branched-chain amplication) (Caneles et al, in press) as well as six microarray platforms (Shi et al, in press). StaRT-PCR and Taqman had equivalent lower detection threshold (10 molecules compared to 6,000 molecules for Quantigene), and similar linear dynamic range among the genes studied (6 log10 for Taqman and StaRT-PCR compared to 4 log10 for Quantigene). StaRT-PCR had the highest signal-to-analyte response of all of the methods. In bivariate analysis of Sample A to B fold-change values for StaRT-PCR relative to Taqman, Quantigene, and each microarray, StaRT-PCR had the highest signal-to-analyte response. That the signal-to-analyte response is 100% and not more than 100% is documented for each gene during quality control preparation of SMIS. StaRT-PCR detected the highest fraction of genes measured compared to all other methods. When compared on the basis of equivalent numbers of genes loaded, the average coefficient of variation (CV) was somewhat higher for StaRT-PCR (3.5%) compared to Taqman (2.5%) or Quantigene (2%), and this is a trade-off resulting from measuring both the native template and corresponding internal standard in each test.

A key advantage of StaRT-PCR is that it generates transcript abundance data that may be compared among multiple patients obtained over many years in the same Standardized Expression Database. The key feature of StaRT-PCR that enables these comparisons is the inclusion of a gene specific internal standard within a standardized mixture of internal standards (SMIS) in each measurement. This provides the data with two important characteristics relevant here. First, each measurement constitutes enumeration of the number of molecules of cDNA in the assay. In the Caneles et al manuscript, the other participants (Taqman, Quantigene, and FDA) saw the advantage of scaling their data to StaRT-PCR because of the molecular enumeration. In the context of the proposed study, this feature facilitates bivariate analysis of the data for altered patterns in BC individuals compared to Non-BC individuals. Second, the presence of an internal standard in each measurement provides intrinsic quality control. There are no false negatives because if the internal standard is not measurable, no value will be recorded, and variation in loading is controlled through measurement of an endogenous reference gene. There are no false positives because enumeration relative to a known number of internal standard molecules allows avoidance of errors related to stochastic phenomena and other artifacts. In contrast, in the MAQC study, both Taqman and Quantigene had documented false positive values for lowly expressed genes, and the evidence suggests that they each had false
positives for highly expressed genes. It is clearly documented that this lack of quality control hampers comparison ofTaqman data from one institution to another (Adams, 2005).

Because of the above clearly documented StaRT-PCR advantages, if a biomarker based on multiple transcript abundance values is developed, samples obtained from individuals in the future may be compared to values in the Standardized Expression Database to determine whether each individual is at increased risk or not. For the Data presented here, it was possible to compare more than 6000 values, obtained in NBEC samples from nearly 50 individuals over more than 6 years. The list of genes for which StaRT-PCR reagents are available is accessible on the worldwide web geneexpressinc.com.

*Statistical Analysis of Virtually Multiplexed Transcript Abundance Data to Identify Co-regulated Genes*

A powerful way to screen for genes that are co-regulated is to measure transcript abundance (TA) of multiple genes in a sample, and to compare the relative TA value of each gene within the same sample to identify those that are correlated according to bivariate analysis. This enables identification of gene pairs with significant correlation. A key finding was that CEBPG transcription factor was correlated with key AO and DNAR genes (i.e. GPX, SOD1, GSTP1, ERCC5, and XRCC1) in NBEC of Non-BC individuals, and not correlated in NBEC ofBC individuals (Mullins et al, 2005).
*Studies to Identify Transcription Factors Responsible for Regulating In Non-BC Individuals the AO and DNAR Genes That Are Dysregulated in BC Individuals: Transcription Factor Recognition Site Analysis*

The E1 Dorado (Build 35) program from the Genomatix software package was used to search for transcription factor recognition sites in the regulatory regions of each of the AO and DNAR genes that were correlated in Non-BC individuals. First, the software was used to locate the AO and DNAR genes within the genome and define 1101 base pairs of the promoter regions (1000 base pairs upstream of and 100 base pairs into the transcription start site) for each gene (Genomatix Software GmbH, Munich, Germany, http://genomatix.de/cgi-bin/eldorado/). The 1101 base pair sequences obtained from the El Dorado program were then used as the target sequences for putative transcription factor recognition site identification using the MatInspector Version 4.2 program (Genomatix Software GmbH, Munich, Germany, http://genomatix.de/cgi-bin/eldorado/). The parameters used were the standard (0.75) core similarity and the optimized matrix similarity. The transcription factor recognition sites that were identified in each of the correlated AO and DNAR genes represented the CEBP family, E2F family, EVI1, and PAX5.

### Transcript Abundance Analysis of Transcription Factors Putatively Responsible for Regulating AO and DNAR Genes

StaRT-PCR reagents were prepared for each of the transcription factors in the CEBP and E2F familes, EVI1 and PAX5 and transcript abundance data were collected in 24 Non-BC and 25 BC samples for which AO and DNAR data had been collected. Of 11 transcription factors evaluated, eight were expressed in NBEC, including CEBPG, CEBPB, CEBPE E2F1, E2F3, E2F6, and EVI1. These eight transcription factors were assessed for correlation with AO and DNAR genes in Non-BC individuals and loss of this correlation in BC individuals. *The only transcription factor out of the eight assessed that had this characteristic was CEBPG* (Mullins et al, 2005).

### Bivariate Analysis to Identify Outliers Relative to the CEBPG/ERCC5 bivariate Plot

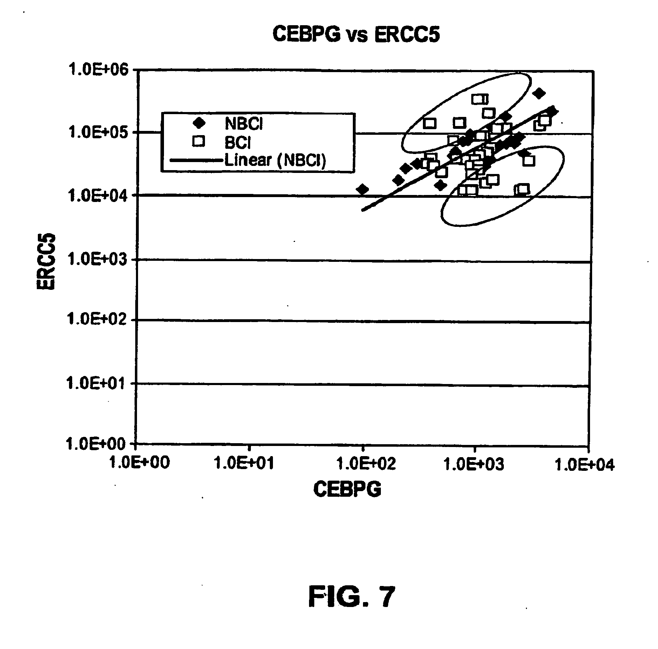

As described above and in Mullins et al (2005) transcript abundance levels for CEBPG and each of the five target genes proposed for study (ERCC5, XRCC1, GSTP1, SOD1, and GPX1) were highly correlated with CEBPG in the Non-BC population (Mullins et al, 2005). An example is the correlation (r = 0.72, p < 0.0001) between CEBPG and ERCC5 among 24 Non-BC individuals (**Figure 7**, diamond symbols). The linear equation for
the regression line defining the relationship between CEBPG and ERCC5 was determined and solved for slope. Any value outside of the regression line slope value +/- 2 standard deviations (SD) was considered a significant outlier. For example, for ERCC5, the slope ([ERCC5 - 6.358]/CEBPG) +/- 2 SD was 0.69 +/- 0.14. The slope value of CEBPG vs ERCC5 in each BC sample (square symbols in Figure 7) then was calculated to determine whether it was a significant outlier. For CEBPG vs ERCC5, there were nine outliers (circled in Figure 7) among BC samples, and only 1 outlier among Non-BC samples. Four of the BC outliers were below the Non-BC regression line.

Summary. Thus, extensive analysis of gene expression in NBEC over many years, using a method in which all of the data could be directly compared, led to the conlusion that key AO and DNAR genes are controlled primarily by CEBPG transcription factor and that this regulation is sub-optimal in BC individuals. Regulation of ERCC5 by CEBPG was selected for detailed investigation to determine the value of this approach to identifying biomarkers for BC risk, as described below and in Experimental Design Section.

### ERCC5 Sequencing in Patient Samples and H23 and BEP2D Cell Lines

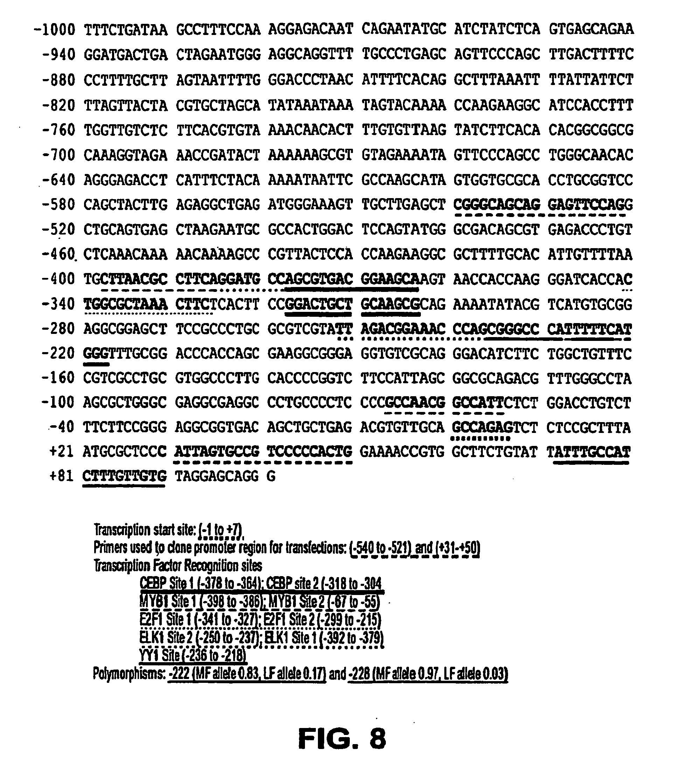

A 235 bp portion of the ERCC5 5' regulatory region (-450 to -235) containing a known polymorphic site was sequenced following isolation from the genomic DNA of 11 non-BC and 10 BC patients as well as the H23 cell line (derived from a BC individual) and the BEP2D cell line (immortalized from Non-BC individual) (**Table 13**). The region analyzed overlapped the region that was cloned into the luciferase reporter vector and used for analysis of ERCC5 transcription reuglation experiments. All 23 samples were from subjects from whom transcript abundance had been measured in NBEC for the antioxidant, DNA repair and transcription factor genes studied previously by Mullins et al. (2005). Individuals chosen for the study included the four BC individuals and the one Non-BC individual with ERCC5 level more than two standard deviations below the regression line for Non-BC individuals (**Figure 7**). The results are presented in **Table 13.** Two polymorphisms were identified (**Figure 8** and **Table 13**). A previously known polymorphism (G-222A) had a more frequent (MF) allele (G) with a prevalence among all 23 samples (83%) that was about the same as previously reported (80%). However, the more frequent MF allele prevalence was lower in the 12 Non-BC individuals (18/24 alleles, 75%) compared to the 11 BC individuals (20/22 alleles, 91%) (**Table 13**). A previously unknown polymorphism, T-228G, relative to the transcription start site of ERCC5 (**Figure 8** and **Table 13**) was present in one non-BC individual. The prevalence was one allele out of 46 evaluated (frequency of 0.02). This also was the only non-BC individual for whom the ERCC5 level was more than two standard deviations (SD) removed from the linear equation for the regression line defining the relationship between CEBPG and ERCC5 transcript abundance in non-BC individuals (**Figure 7****, Table 13**).

*Polymorphisms causing low ERCC5 relative to CEBPG*

Both polymorphic sites were within the YY1 recognition site in the 5' regulatory region of ERCC5. Certain allelotypes at each site were associated with low ERCC5 expression relative to CEBPG expression (defined as > 2 standard deviations below the regression line generated by bivariate comparison of ERCC5 to CEBPG among Non-BC individuals). OfBC individuals with homozygous GG at -222, 56% (5/9) had ERCC5 below regression line. In contrast, ofNon-BC individuals with homozygous GG at -222, none had ERCC5 below regression line (0/7). This suggests that there is another factor present in BC individuals that works in conjunction with -222 GG allelotype to cause reduced ERCC5.

In addition, there was one Non-BC individual with ERCC5 below the regression line. This individual was Non-GG at -222 but was the only individual among the 23 in this study to have a rare allele at -228. In addition to the association of particular alleles with low ERCC5/CEBPG in the 23 individuals sequenced, the slope of the
ERCC5/CEBPG relationship was markedly flattened among the six bronchogenic carcinoma cell lines studied compared to that observed in NBEC. The slope for the bronchogenic carcinoma cell lines was about 1, while the slope for NBEC was close to 100. It is likely that presence of GG allelotype will be observed in each cell line, as it was in H23.

### ERCC5 Promoter Sequence Polymorphisms at -228 and -222.

**Table 13.** ERCC5 promoter polymorphisms identified in 11 non-BC patients, 10 BC patients and the H23 and BEP2D cell lines. Genomic DNA was analyzed for polymorphisms in the above samples. Polymorphism position is relative to the transcription start site of ERCC5. Refer to **Figure 7** for the scatter plot of the CEBPG/ERCC5 transcript abundance bivariate correlation with the linear regression line of the correlation in non-BC individuals.

### CEBPG Regulates Key AO and DNAR Genes in NBEC

In one set of experiments, CMV-CEBPG expression vector was cotransfected with a Luciferase reporter construct containing the regulatory region of ERCC5 upstream. These experiments were conducted to determine whether CEBPG would upregulate luciferase from the ERCC5 regulatory region. In a second set of experiments Luciferase reporter constructs containing serially deleted ERCC5 regulatory region were transfected in an effort to confirm that the CEBPG recognition sites within the ERCC5 regulatory region are responsible for upregulation by CEBPG. In order to conduct these experiments, it was necessary first to a) identify a cell population with a low endogenous expression of CEBPG so that up-regulation of ERCC5 would be easily observed following exogenous upregulation of CEBPG, b) prepare the CMV-CEBPG expression vector, and c) prepare the ERCC5-Luciferase reporter constructs. In order to identify the most suitable cell line for the transfection experiments, StaRT-PCR was used to measure CEBPG and ERCC5 in six bronchogenic carcinoma cell lines, including H23, H209, H460, H1437, H1355 and BEP2D). The correlation between the two genes was strong (R2 > 0.99) as it was in the NBEC of non-BC individuals (Mullins et al, 2005), which is consistent with regulation of ERCC5 by CEBPG. However, the slope of regression line was much flatter among the cell lines, consistent with absence of one or more factors that work synergistically with CEBPG to upregulate ERCC5. Specifically, the slope was close to 1.0 among the cell lines and close to 100 among NBEC.

The H23 cell line was chosen because it had the lowest endogenous transcript abundance values for CEBPG (299 molecules/106 ACTB molecules) and ERCC5 (1,540 molecules/106 ACTB molecules).

### Plasmid Creation

### ERCC5-Luc Reporter Construct

ERCC5 regulatory region (**Figure 8**) was PCR-amplified from the genomic DNA of an individual without lung cancer (subject #260 from Mullins et al, 2005) and inserted immediately upstream of the promoter of a luciferase expression vector. Analysis of the 1101 bp ERCC5 upstream regulatory region for transcription factor recognition sites was previously reported (Mullins et al., 2005). Primers for amplifying a 589 bp segment of the regulatory region containing the intiation site and two CEBP recognition sites were designed using Oligo® Primer Analysis Software Version 6.0 (Cascade, CO), which identifies optimal primer sequences on the basis of annealing temperature and lack of duplex formation and non-specific binding. Platinum® Taq DNA Polymerase High Fidelity (Invitrogen Corporation, Carlsbad, CA) was used for PCR amplification. The amplicon was then ligated between the HindIII and XhoI restriction enzyme sites of the pGL2-Basic vector (Promega Corp., Madison, WI).

### CMV-CEBPG and CMV-CEBPB Expression Vectors

A full-length CEBPG expression vector was purchased from Open Biosystems, Inc. (Huntsville, AL). Restriction enzyme AvaI (Fisher Scientific International, Hampton, NH) was used to excise the CEBPG insert to obtain the negative control plasmid pCMV-SPORT6. A full-length CEBPB clone was purchased from Open Biosystems Inc., (Huntsville, AL), excised from the pOTB7 vector with EcoRI and XhoI enzymes (Invitrogen, Corporation, Carlsbad, CA and Fisher Scientific International, Hampton, NH, respectively) and ligated into pCMV-SPORT6.

The CEBPG or CEBPB coding regions were ligated immediately downstream of CMV promoter, to form expression vectors. All insert ligations were performed using 10X DNA ligase buffer and T4 DNA ligase from the Invitrogen Corporation (Carlsbad, CA). Plasmids and/or inserts were electrophoresed on 2% NuSieve® GTG® agarose (Cambrex Bio Science Rockland, Inc., East Rutherford, New Jersey). Plasmids were amplified by transformation into One Shot® Top10 Chemically Competent E. coli (Invitrogen Corporation, Carlsbad, CA) and isolated from cells grown on LB agar, Miller (Fisher Scientific International, Hampton, NH) with ampicillin (10 mg/ml) (Invitrogen Corporation, Carlsbad, CA). Plasmid purification was performed using QIAGEN Plasmid Mini, Midi or Maxi Kits (Qiagen, Inc., Valencia, CA). Gel extraction and purification of plasmids and inserts were performed using the QIAquick Gel Extraction Kit (Qiagen, Inc., Valencia, CA). Following extraction, CMV-CEBPG and CMV-CEBPB expression vectors were sequenced for confirmation of original sequence and proper orientation (University of Iowa DNA Facility, Iowa City, Iowa). Following sequence verification, a large amount of each plasmid was prepared through Maxiprep.

*Identification of Optimal Cell Lines for Transfections*

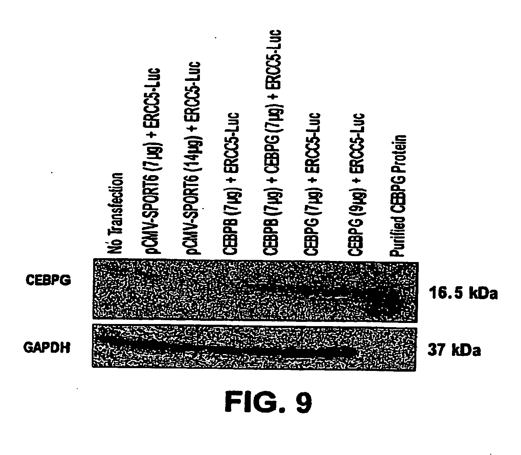

The CMV-CEBPG expression vector was transfected into three cell lines, including the bronchogenic carcinoma cell lines H460 and H23, and the immortalized human bronchial epithelial cell line BEP2D. Following transfection the highest exogenous CEBPG transcription was observed in H23 and increased protein expression in association with the exogenous CEBPG transcript was confirmed by Western blot (**Figure 9**). Thus, H23 cell line was used for subsequent cotransfection studies. For the co-transfection studies, ERCC5-LUC plasmid was co-transfected into H23 with either CEBPG or CEBPB alone, or the combination of CEBPG and CEBPB.

### Co-transfection of 7 µg CEBPG or CEBPB with ERCC5 Promoter-Luciferase Reporter Construct in H23 Cells

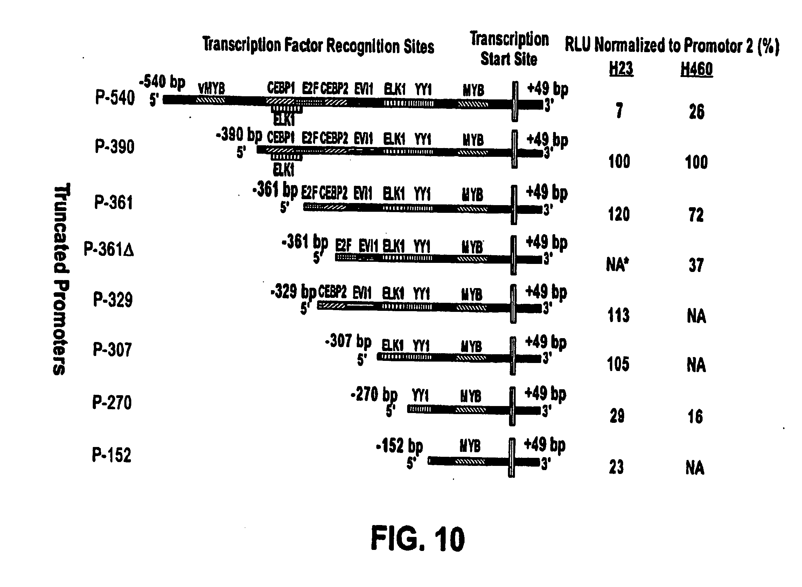

In the first set of transfection experiments, 7 µg of either the CEBPG or CEBPB expression vectors were co-transfected with 3 µg of the ERCC5 promoter-luciferase construct. Although the primary interest was to experimentally confirm whether CEBPG regulates ERCC5, because all CEBP family members, with the exception of CEBPZ, can bind to the same consensus binding site, transfections were performed with CEBPB to test for non-specific effects. Seven micrograms of the pCMV-SPORT6 plasmid served as the negative control for both expression vectors. CEBPG expression vector transfected at 7 µg was shown to reproducibly activate the exogenous ERCC5 promoter approximately 2-3 fold higher than the empty vector control, as seen by the increase in luciferase activity upon co-transfection (**Figure 10**). This is consistent with activation of the exogenous ERCC5 promoter by exogenous CEBPG. In subsequent experiments, with more efficient transfection, increases of 5-7 fold were observed. CEBPB, however, reproducibly caused less activation of the ERCC5 promoter than the empty vector control (62% of the control), and the difference between activation of the promoter by CEBPG and CEBPB was significant (p = 0.008).

### Transcript Abundance and Protein Expression Results

StaRT-PCRTM was performed with the cDNA from the first transfection in order to confirm CEBPG transcript was being produced by the expression vector. After DNase-treatment to remove contaminating plasmid
DNA a 130-fold increase in CEBPG transcript was observed with transfection of the CEBPG expression vector versus the empty vector control. A Western blot was also performed in order to confirm association between increased CEBPG transcript abundance and CEBPG protein (**Figure 9**). Endogenous CEBPG protein was observed (Lane 2), as well as increased amounts of CEBPG protein in the transfected cells (**Figure 9**).

### ERCC5 regulatory region deletion analysis to confirm role of CEBPG recognition site

In order to determine whether factors endogenous to H23 cells could activate the exogenous ERCC5 promoter, an experiment was performed in which 3 µg of only the 589 bp ERCC5 promoter-luciferase construct was transfected into H23 cells. The 5' end of this segment is 540 upstream of the start site, and this number is used as the reference point. Transfection of 3 µg of the pGL2-Basic vector was used as the empty vector control. There was a more than 16,000-fold higher luciferase activation from the P-540 ERCC5 promoter compared to the empty luciferase vector. This is consistent with activation of the exogenous ERCC5 P-540 by factors endogenous to H23 cells.

Sequentially longer deletions from the 5' end (with respect to the coding strand) of the regulatory region were made using appropriately designed PCR primers. Each of the deleted sequences was ligated into pGL2-Basic vector, verified for sequence and orientation, large scale prepared and extracted.

Luciferase activity results, shown in **Figure 10****,** are presented relative to the P-390 values. In P-390, deletion of the MYB1 site resulted in more than 10-fold increase in the luciferase expression from endogenous transcription factors in H23 compared to that observed with P-540, and nearly 4-fold increase in H460. No change was observed with additional deletion of the CEBP1 (the CEBP site most 3' to the start site) in P-361 or the more distal E2F1 site in P-329. However, a marked reduction was observed with deletion including the combination of the more proximal CEBP site (CEBP2), ELK1, and EVI1, in P-270.

In order to more closely define the role of the CEBP2 site, it was selectively deleted from P-361 using a combination of the Higuchi (1988) and Celi (1993) methods. When transfected into H460, P-361A yielded 50% of the luciferase activity observed with P-361. The remaining activity was likely due to ELK1, or YY1. This is because, further shortening of P-361 to P-307 left only the recognition sites for ELK1, and YY1, yet the luciferase activity was unchanged compared to P-361 in H23, and a promoter with only YY1 site, P-270, was associated with very little luciferase activity in either H23 (**Figure 10**) or H460.

The role of YY1 in down regulating ERCC5 expression was supported by transfection of P-361 containing both -222 G allele and -228 G allele into H460. This vector was associated with more than 50% reduced ERCC-Luc expression in H460 compared to P-361 which contained -222 A and -228 T.

Summary. The data indicates that CEBPG, MYB1, YY1, and/or ELK1 participate in ERCC5 regulation in NBEC ofNon-BC individuals. Further, the sequencing data support the conclusion that the reduced ERCC5 relative to CEBPG in BC individuals is explained by inheritance of particular alleles at the YY1 recognition site in at least some individuals. In these individuals, it is possible that YY1 functions by interacting with CEBPG, MYB1, and/or ELK1. The intereaction with ELK1 is due to proximity and ERCC5-Luc transfection results.

*DESIGNAND METHODS*

Sequence ERCC5 in 110 Non-BC and 110 BC individuals to confirm that the ERCC5 -222 G allele is represented at a significantly higher rate in BC Individuals.

Power analysis based on the data indicates that collecting sequence information for this number of individuals will enable demonstration of significant difference at p value of 0.05 and 80% power. Demonstration of significantly higher prevalence of the ERCC5 -222 G allele in BC individuals will be indicative of such individuals being of increased risk. Statistical criteria of 80% power with P < 0.05 suggests assessment of a total of 110
Non-BC and 110 BC individuals. This is based on data disclosed herein, in which the frequency of ERCC5-222 G allele was 75% among the 12 Non-BC individuals and 91% among the 11 BC individuals.

### Collection of Samples from Non-Bronchogenic Carcinoma (Non-BC) and Bronchogenic Carcinoma (BC) Individuals

Measurements are made using methods that enable quantitative ex vivo measurement of transcript abundance in NBEC biopsy samples yielding values closely representing the levels that exist *in vivo.* (Willey et al, 2000; Warner et al, 2003; Willey et al, 2004a; Willey et al, 2004b). In one example, NBEC is obtained through bronchoscopic brush biopsy. Bronchoscopic biopsy generally yields fewer than one million cells. PCR-based gene expression measurement methods are the only ones with sufficient signal amplification to enable hundreds of measurements in these small samples (Canales et al, in press).

### Peripheral Blood Sample Collection and Processing

From each patient, 10 ml of peripheral blood will be collected into a heparin tube for isolation of DNA, and 2.5 ml of blood will be collected into each of four PAX tubes (Qiagen, Inc.) for isolation of RNA. The 20 ml of peripheral blood will contain approximately 1-2 x 108 WBC. Ten per cent of cells (approximately 10 million cells) from heparin tube will be used for DNA extraction for sequencing studies. Ninety per cent of cells (approximately 90 million cells) from the heparin tube will be used to produce nuclear extract for the experiments planned under Specific Aim 3. All of the cells in the PAX tubes will be used for RNA extraction for StaRT-PCR transcript abundance measurement.

### DNA Sequencing Methods

The region sequences will span 235 bp, from -215 to -450 bp relative to the initiation site (See **Figure 8**). This region contains all of the recognition sites likely to be involved in ERCC5 transcription regulation, as described above. Based on the region desired, the necessary sequencing primers can be designed and synthesized using methods familiar to one of ordinary skill in the art. *Measure expression of CEBPG and ERCC5 in the normal bronchial epithelial cells and peripheral blood cells (PBC) of the 220 individuals*

Demonstration that alleles at the ERCC5-222, -228, and/or other polymorphic sites are associated with lower ERCC5 expression relative to CEBPG will be indicative that they are responsible for down regulation of ERCC5. If expression patterns are the same in PBC as in normal bronchial epithelial cells this will support their use as surrogate tissue for development of biomarkers of lung cancer risk.

*Collection of NBEC Samples*

Approximately 10 brush biopsies will be obtained from normal appearing mucosa at approximately the tertiary bronchi. If the patient has a local pathological condition, such as pneumonia, trauma, or BC, the brushes will be taken from the opposite lung. The patients uniformly tolerate this portion of the procedure extremely well, without complications or discomfort. The NBEC samples will be processed for use sequencing and measuring expression levels. After each bronchoscopic brush biopsy, the brush will be swirled in approximately 3 ml of ice cold saline to dislodge and retrieve the cells. Approximately 500,000 to 1 million cells are obtained with each brush. Thus, 10 brushes will yield 5-10 million cells. These cells in 3 ml of ice cold saline will be divided up for extraction of RNA and protein. Approximately 5 million cells will be used for nuclear protein extraction (Dignam et al, 1983). This should yield approximately 100 µg of nuclear extract for EMSA and Western hybridization analyes. Approximately 1 million cells will be used for RNA extraction for the StaRT-PCR transcript abundance measurements. This will yield 3-5 µg of RNA which is sufficient for the proposed studies based on past experience (Willey et al, 1997; Crawford et al, 20.00).

### Peripheral Blood Sample Processing

As described above, from each patient 2.5 ml of blood will be collected into each of four PAX tubes (Qiagen, Inc.) for isolation of RNA. This number of cells (approximately 108) should yield more than 10 µg of RNA which will be sufficient for the proposed studies.

### Transcript Abundance Measurement

StaRT-PCR will be used for transcript abundance measurement. The particular advantages of StaRT-PCR for the proposed studies is described herein above, and is supported by the pending Nature Biotechnology manuscript (Caneles et al, in press). Caneles clearly shows that StaRT-PCR generates data that meet the published FDA and CLIA recommended requirements for regulatory review in drug and diagnostic test development. Just as importantly, other methods reviewed for quantitative transcript abundance measurement did not meet FDA/CLIA criteria.

Triplicate measurements of CEBPG and ERCC5 in cDNA derived from each NBEC and blood RNA sample will be conducted. Standard methods for StaRT-PCR will be used, as previously published (Willey et al, 2004; Mullins et al, 2005; Caneles et al, in press). Briefly, RNA will be reverse transcribed to cDNA using MMLV reverse transcriptase and oligodT primers. Each cDNA sample will be calibrated through dilution so that 1 µl contains 600,000 ACTB cDNA molecules when measured relative to 1 µl of standardized mixture of internal standards (SMIS), which contains 600,000 molecules of ACTB internal standard. Two ul of each calibrated cDNA sample, 2 ul of SMIS, combined with polymerase, dNTPs, primers and buffer in a final PCR reaction volume of 20 µl will be used in each StaRT-PCR measurement. Following PCR amplification in an air thermocycler, the products will be size separated and quantified on an Agilent 2100 or Caliper AMS90 microfluidic electrophoresis device. The areas under the peak for native template and the known quantity of internal standard template (in molecules) will be quantified, and the ratio between the two will be automatically calculated by software to provide a value for initial native template in sample in molecules. This value will be compared to the number of ACTB molecules in each measurement to provide a final value in the form of molecules of target gene/106 ACTB molecules. The data will be combined with data already collected and presented in the Preliminary Data section.

*Bivariate Analysis of ERCC5 vs CEBPG Transcript Abundance Values*

As described above, in BC individuals the ERCC5-222 GG allelotype was associated with low ERCC5 transcript abundance in NBEC relative to the regression line formed from bivariate analysis of ERCC5 relative to CEBPG in NBEC among Non-BC individuals. The regression for Non-BC individuals was based on data from 24 samples (Mullins et al, 2005). By increasing the sample number to 110, confidence in differences from the regression line will be substantially increased. Based on results obtained above, more than 80% ofBC individuals will have ERCC5-222 allelotype, and approximately 50% of these individuals will have ERCC5 value more than two standard deviations below the Non-BC regression line. Thus, out of 110 BC individuals, approximately 90 will have ERCC5-222 allelotype and that 45 of these will have ERCC5 two standard deviations below the Non-BC regression line.

In addition to analysis in NBEC, transcript abundance of ERCC5 and CEBPG will be measured in RNA extracted from peripheral blood samples. The RNA in such samples is predominantly from neutrophils, with a smaller fraction from lymphocytes, macrophages, eosinophils, and basophils in descending order. Transcript abundance measurements from peripheral blood will simplify analysis of ERCC5 and CEBPG transcript abundance as biomarkers for BC risk, whether by themselves, or in conjunction with measurement of several other BC risk related genes. The suitability of StaRT-PCR for this application is illustrated (Peters et al, submitted for publication) in which StaRT-PCR was used to measure transcript abundance of 19 inflammation related genes in 15
individuals who were highly characterized as "normal". The key methods used by Peters et al (collection of blood samples into PAX tubes followed by StaRT-PCR analysis) will be used and is associated with very low analytical variation and low visit to visit biological variation. These results indicate that small biological differences in transcript abundance between groups of individuals can be identified with less than twenty subjects per group.

### Comparison of ERCC5 vs CEBPG Result to ERCC5-222 Allelotype

The results of ERCC5-222 allelotype analysis and bivariate transcript abundance bivarate analysis from above will be compared to determine whether there is a significant association of ERCC5 -222 GG allelotype with reduced ERCC5 relative to CEBPG among BC individuals and lack of such association among Non-BC individuals. In the data presented above, 5/9 BC individuals (56%) with GG allelotype had ERCC5 two standard deviations below regression line, but only 0/7 Non-BC individuals had this characteristic. There was one Non-BC individual below line with G/A, but this individual had rare polymorphism at ERCC-228. Under a reasonable power analysis, 1/7 (14%) Non-BC with GG allelotype have low ERCC5. Thus, 56% in BC and 14% in Non-BC, 95 samples in each group would provide detection of a significant difference at alpha of 0.05 and power of 80%.

### ERCC5 regulation by CEBPG and polymorphic sites affecting regulation

Mechanistic basis for correlation of ERCC5 with CEBPG in Non-BC individuals and absence of this correlation in BC individuals can be assayed. Results will indicate, a) ERCC5 transcription is regulated predominantly by CEBPG in Non-BC individuals and b) that inheritance of particular alleles at the ERCC5-222, ERCC5-228, and/or other polymorphic sites in the regulatory region of ERCC5 contribute to sub-optimal ERCC5 transcription regulation in BC individuals: Primary normal bronchial epithelial cells (NBEC) from Non-BC individuals and BC individuals as well as cultured normal, immortalized, or malignant bronchial epithelial cells will be used to carry out these studies.

The data indicate that the TA level of ERCC5 was significantly correlated with transcription factor CEBPG in the NBEC of Non-BC individuals. No such correlation was observed in NBEC ofBC individuals. The correlation exists because CEBPG regulates ERCC5 transcription in NBEC of Non-BC individuals, and that there is sub-optimal regulation in BC individuals, observed as a lack of correlation of ERCC5 and CEBPG transcript abundance. Further, no correlation existed between ERCC5 and the other transcription factors assessed, including CEBPB, E2F1, E2F3, E2F6 and EVI, indicating that CEBPG is the predominant determinant of ERCC5 regulation in NBEC ofNon-BC individuals. Notably, CEBPA, D, and E were expressed at very low, probably insignificant levels in NBEC.

Mechanisms underlying the lower correlation between CEBPG and ERCC5 in NBEC samples from BC individuals can classified into three general groups, schematically represented in **Figure 6**. Putative Mechanism 1: reduced CEBPG transcription is associated with low CEBPG protein, causing reduced ERCC5 transcription. As such, the bivariate relationship between CEBPG and ERCC5 would either decrease along the regression line of Non-BC individuals (NBCI), as exemplified by BCI2, or be above the regression line, as exemplified by BCI1. BCI1 could be above regression line due effects of other transcription factors putatively involved in regulation, including increase in ELK1, or decrease in MYB1 or YY1. Putative mechanism 2: involves reduced functional interaction between CEBPG and regulatory regions of ERCC5, as caused by a mutation in the coding region of CEBPG or the regulatory region of ERCC5. In this case, the bivariate relationship for the BC individual (e.g BCI 3-5) will fall below the Non-BC regression line. Putative mechanism 3: This involves polymorphisms that alter function of transcription factors other than CEBPG (e.g. ELK1, YY1, MYB1) that regulate ERCC5, causing ERCC5 value to fall below the regression line. The sequencing data indicate that polymorphisms in the YY1 recognition site within ERCC5 regulatory region are associated with altered YY1 function resulting in decrease in ERCC5 relative
to CEBPG and that this is the mechanism primarily responsible for causing ERCC5 to be below the regression line' in more than 50% ofBC individuals with ERCC5-222 GG allelotype.

### ERCC5-Luc experiments to investigate regulation of ERCC5 transcription

### CEBPG Analysis

CMV-CEBPG expression vector will be transfected into H23 cells (low CEBPG expressors) along with ERCC-5-LUC vectors containing specific deletions and/or site mutations. Cell lines suitable for this purpose have been identified (e.g. H23 and H460). Cultured cells will be transiently cotransfected with varying amounts of CEBPG expression plasmid (pCMV/CEBPG), and a constant amount of ERCC5-Luc reporter construct containing ERCC5 promoter upstream. In addition, cells will be transfected the RSV-β-galatosidase β-gal) plasmid to normalize transfection efficiency. In each transfection, Luc activity will be divided by β-gal activity to obtain normalized Luc activity. Plasmids without CEBPG cDNA insert (pCMV vector) and the PGL2-basic vector without the regulatory region of the target gene promoter construct will be used as negative controls. Forty-eight hours following transfection, these tranfectants will be analyzed for target gene promoter Luc reporter activity by the luciferase assay system.

### ELK1 Analysis

1) CEBP2 site deletion with preservation of ELK1 site (P-361Δ) does not decrease optimal ERCC5-Luc activity in the low CEBPG expressing line H23 (**Figure 10**). 2) Deletion of segment containing ELK1 and EVI recognition site causes maximum reduction in ERCC5-Luc activity in both H23 and H460. **3)** EVI1 is expressed at very low level in bronchial epithelial cells and this site likely is not relevant in this context. Additional experiments: **1)** Prepare P-361ERCC5-Luc with specific ELK1 deletion. **2)** Identify cell lines with high or low endogenous ELK1 expression. **3)** Prepare CMV-ELK1 expression vector. **4)** Assess luciferase activity from P-361ERCC5-Luc and ΔELK1P-361ERCC5-Luc in a) H23 and H460, b) in cell line with high endogenous ELK1 expression, c) in cell line with low endogenous ELK1 expression following exogenous ELK1 up-regulation (CMV-ELK1)

*YY1 Analysis*

Deletion of ERCC5 regulatory region through CEBP2 and ELK1 but not YY1 recognition site causes complete loss of ERCC5-Luc activity in H23 (low CEBPG) or H460 (high CEBPG). ERCC5-222 allele in YY1 site correlates with low transcript abundance of ERCC5 relative to CEBPG.

Luciferase activity can be assessed in H23 and H460 from ELK1delP-361ERCC5-Luc with: i. Intact CEBP2 and different YY1 alleles (ERCC5-222 G or A, and/or ERCC5-228 G or T); ii. Both CEBP2 and ELK1 deletion with different YY1 polymorphic alleles; iii. Conduct same experiments in H23 or H460 with siRNA for YY1. Furthermore, EMSAs can be utilized to assess binding of recombinant CEBPG or ELK1 to any of the promoters sequences. EMSA techniques are well known to one of ordinary skill in the art.

### CEBPG binding to ERCC5 in NBEC

As indicated above TA levels of ERCC5 and CEBPG show high correlation in non-BC individuals but not in BC individuals. In addition, site-specific mutations introduced into the CEBP2 site of ERCC5 regulatory region were associated with reduced luciferase activity following transfection into H23. These results indicate that CEBPG plays a key role in regulating ERCC5. Further, in order to determine whether NBEC ofBC individuals versus Non-BC individuals is due to lower CEBPG-DNA binding affinity, gel shift assay (EMPSA) experiments and immunohistochemistry assays can be conducted.

EMSA experiments will be performed with bronchial brush biopsy samples obtained from non-BC individuals and BC individuals. Acquisition of the bronchial brush samples as described above. After removing 1-2 million cells for RNA extraction, approximately 5 million cells, yielding approximately 100 µg of protein, will be
used for the nuclear extract preparation(Dignam et al. 1983). EMSA experiments will be performed using methods known in the art, such as (Periyasamy et al. 2000) using 32P-labeled CEBPG oligonucleotides spanning the CEBP2 recognition site and the nuclear extracts. The specific antibody for CEBPG will be included in the analysis to confirm specificity of CEBPG DNA binding activity. The purified CEBPG protein will serve as a positive control.

Immunofluorence studies using specific antibodies for ERCC5 and CEBPG will be performed to determine the correlation at the protein levels of ERCC5 and CEBPG in bronchial brush biopsies of non-BC individuals versus BC individuals. Specific antibodies to determine the expression levels of CEBPG and ERCC5 are readily available and will be obtained from commercial vendors (e.g., Santa Cruz Biotechnology). Briefly, the dislodged normal bronchial epithelial cells of bronchial brush biopsy samples from non-BC individuals and BC individuals will be grown on coverslips. The cells will be permeabilized and fixed with methanol/acetone. The methanol/acetone fixation has been shown to give reliable and reproducible staining with a broad scale of antibodies. After fixation, the cells will be incubated for 1 h at RT with the primary antibody in the appropriate dilution in PBS containing 1% bovine serum albumin (BSA). Subsequently, the cells will be washed with PBS, followed by 30 to 60 min of incubation with a secondary antibody conjugated to AlexaFlour 568 diluted in PBS containing 1% BSA. After this incubation step, the cells will be rinsed with PBS and mounted in TRIS-HCL buffered Mowiol containing 0.5 µg/ml of DAPI and 2% of the anti-fading reagent. The intensity of the staining will be determined under fluorescense microscopy. Reduced intensity in BC with low ERCC5 would be consistent with importance of CEBPG in ERCC5 regulation in lung cells.

Measurement of transcription rate and stability

Nuclear run-on analysis and StaRT-PCR will be conducted on the same samples to determine whether decreased target gene promoter activity and/or decreased ERCC5 DNA binding by CEBPG in presence of certain alleles in ERCC5 regulatory region affects rate of target gene transcription and steady-state target gene transcript abundance level, according to previously disclosed methods (Periyasamy et al, 1996, and Willey et al, 1998). To determine whether decreased transcript abundance level of the target gene is due to decreased stability, TA levels will be analyzed after treatment with actinomycin D at various time periods as previously described (Periyasamy et al, 1996).

**Table 1**

| Sequence for each primer used for StaRT-PCR (forward and reverse) MTA measurement or for preparation of internal standard (CT. | | | | | |
|---|---|---|---|---|---|
| Gene | Accession # | Primer | Sequence | Position | Product |
| ACTB | X00351 | Forward | 5' ATC CTC ACC CTG AAG TAC CC 3' -SEQ ID NO: 8 | 231 | |
| | | Reverse | 5' CCA TCT CTT GCT CGA AGT CC 3'-SEQ ID NO: 9 | 704 | 493 hp |
| | | CT | 5' CCA TCT CTT GCT CGA AGT CCG CCA GCC AGG TCC AGA CGC A 3' -SEQ ID NO: 10 | 568 | 377 hp |
| | | | | | |
| CAT | X04076 | Forward | 5' CCA GAA GAA AGC GGT CAA GA 3' -SEQ ID NO: 11 | 1492 | |
| | | Reverse | 5' AAC CTT CAT TTT CCC CTG GG 3' -SEQ ID NO: 12 | 1822 | 350 hp |
| | | CT | 5' AAC CTT CAT TTT CCC CTG GGC CAG TGA TGA GCG GGT TAC A 3' -SEQ ID NO: 13 | 1699 | 247 hp |
| | | | | | |
| CEBPB | NM_005194 | Forward | 5' TGT CCA AAC CAA CCG CAC AT 3'-SEQ ID NO:14 | 1412 | |
| | | Reverse | 5' AGC AAC AAG CCC GTA GGA AC 3' -SEQ **ID** NO: 15 | 1657 | 265 hp |
| | | CT | 5' AGC AAC AAG CCC GTA GGA ACA CGC GTT CAG CCA TGT TTA A 3' -SEQ ID NO: 16 | 1571 | 199 bp |
| | | | | | |
| CEBPG | U20240 | Forward | 5' CGG TTG AAA AGC AAG CAG AAA GCA 3' -SEQ ID NO: 17 | 488 | |
| | | Reverse | 5' GAT CCC AGA AAA TAG CCT CCA ATG 3' -SEQ ID NO: 18 | 814 | 350 bp |
| | | CT | 5' GAT CCC AGA AAA TAG CCT CCA ATG AAC ATT CAA GCC ACA AGC TC 3' –SEQ ID NO: 19 | 726 | 282 bp |
| | | | | | |
| E2F1 | M96577 | Forward | 5' TGA TAC CCC AAC TCC CTC TA 3'-SEQ ID NO: 20 | 2076 | |
| | | Reverse | 5' AAC GCA GGA GGG AAG AGA GC 3'-SEQ ID NO: 21 | 2452 | 396 bp |
| | | CT | 5' AAA GCA GGA GGG AAC AGA GCA CTG CAG GGA CCA CAG G 3' –SEQ ID NO: 22 | 2363 | 327 hp |
| | | | | | |
| E2F3 | Y10479 | Forward | 5'TGA AAG CCC CTC CAG AAA CAA G 3'-SEQ ID NO: 23 | 1019 | |
| | | Reverse | 5' GCA GCA GGG GAG GCA GTA AGT T 3'-SEQ ID NO: 24 | 1336 | 339 hp |
| | | CT | 5' GCA GCA GGG GAG GCA GTA AGT TGG GGA GGC CAG AGG AGA AAG GT 3' –SEQ ID NO: 25 | 1253 | 278 bp |
| | | | | | |
| E2F6 | AF059292 | Forward | 5' GGG CCT GCT GCC ATC AAA AAT A 3' -SEQ ID NO: 26 | 99 | |
| | | Reverse | 5' CCG CTT TCG GAC TCC CAG TTT 3' -SEO **ID** NO: 27 | 283 | 205 bp |
| | | CT | 5' CCG CTT TCG GAC TCC CAG TTA GCG ATA CAT CAA AAC GAG G 3' -SEQ ID NO: 28 | 184 | 125 bp |
| | | | | | |
| RRCCI | M13194 | Forward | 5' CTG GAG CCC CGA GGA AGC 3' -SEQ ID NO: 29 | 739 | |
| | | Reverse | 5' CAC TGG GGG TTT CCT TTG 3'-SEQ ID NO: 30 | 1049 | 328 bp |
| | | CT | 5' CAC TGG GGG TTT CCT TGG AAG GCC AGA TCT TCT CTT 3' - SEQ ID NO: 31 | 928 | 240 bp |
| | | | | | |
| ERCC2 | X52221 | Forward | 5' GGC CTT CTT CAC CAG CTA C 3' -SEQ ID NO: 32 | 1608 | |
| | | Reverse | 5' GTA GTC CGT CTT GCC CCT G 3' -SEQ ID NO: 33 | 2004 | 415 bp |
| | | CT | 5' GTA GTC CGT CTT GCC CCT GTG GAA CTG GTC CCG CAG GT 3' –SEQ ID NO: 34 | 2597 | 346 bp |
| | | | | | |
| ERCC4 | U64315 | Forward | 5' AGT GCA TCT CCA TGT CCC GCT ACT A 3'-SEQ ID NO: 35 | 2213 | |
| | | Reverse | 5' CGA TGT TCT TAA CGT GGT GCA TCA A 3' -SEQ ID NO: 36 | 2578 | 390 hp |
| | | CT | 5' CGA TGT TCT TAA CGT GGT GCA TCA ACA GGC TGT GGC TTG CTT TGT 3' -SEQ ID NO: 37 | 2433 | 265 bp |
| | | | | | |
| ERCC5 | D16305 | Forward | 5' AAG GAA AGA GAA AGA AGC AGC AGC CA 3' -SEQ ID NO: 38 | 3087 | |
| | | Reverse | 5' can ACA CAG ATC TGG CGG TCA CGA GG 3'-SFQ ID NO: 39 | 3501 | 440 bp |
| | | CT | 5' CAA ACA CAG ATC TGG CGG TCA CGA GGA GCT TCC TTC ACT GAG TTC TGC GAA T 3' –SEQ ID NO: 40 | 3401 | 366 hp |
| | | | | | |
| Evil | NM_005241 | Forward | 5' CGC CGG ATA TCC ACG AAG A 3' -SEQ ID NO: 41 | 302 | |
| | | Reverse | 5' ATG CTG AGA GCG AAT GTG C 3' -SEO ID NO: 42 | 711 | 428 bp |
| | | CT | 5' ATG CTG AGA GCG AAT GTG CTT AAA TGC CTT GGG ACA CT 3' -SEQ ID NO: 43 | 587 | -1 323 bp |

| Gene | Accession # | Primer | Sequence | Position | Product |
|---|---|---|---|---|---|
| GPX1 | Y00433 | Forward | 5' CCT GGT GGT GCT CGG CTT CC 3' SEQ ID NO: 44 | 522 | |
| | | Reverse | 5' CAA TGG TCT GGA AGC GGC GG 3' -SEQ ID NO: 45 | 852 | 350 bp |
| | | CT | 5' CAA TGG TCT GGA AGC GGC GGA CCG GAG ACC AGG TGA TGA G 3' –SEQ ID NO: 46 | 757 | 279 bp |
| | | | | | |
| GPX3 | D16360 | Forward | 5' GCA GAG CCG GGG ACA AGA GAA 3' -SEQ ID NO: 47 | 113 | |
| | | Reverse | 5' CTG CTC TTT CTC TCC ATT GAC 3' -SEQ ID NO: 48 | 471 | 379 bp |
| | | CT | 5' CTG CTC TTT CTC TCC ATT GAC GCT CTT CCT GTA GTG CAT TCA 3' -SEQ ID NO: 49 | 298 | 227 bp |
| | | | | | |
| GSTM1,2,4,5 | J03817 | Forward | 5' GGG ACG CTC CTG ATT ATG AC 3' -SEQ ID NO: 50 | 122 | |
| | | Reverse | 5' GCA AAC CAT GGC CGCTTC CC 3'-SEQ **ID** NO: 51 | 442 | 340 bp |
| | | CT | 5' GCA AAC CAT GGC CGC TTC CCT TCT CCA AAA TGT CCA CAC G 3' –SEQ ID NO: 52 | 301 | 219 bp |
| | | | | | |
| GSTM3 | J05459 | Forward | 5' GTG CGA GTC GTC TAT GGT TC 3'-SEQ ID NO: 53 | 23 | |
| | | Reverse | 5' AGT TGT GTG CGG AAA TCC AT 3' SEQ **ID** NO: 54 | 342 | 339 bp |
| | | CT | 5' AGT TGT GTG CGG AAA TCC ATT GCT CTG GGT GAT CTT GTT C 3'-SEQ ID NO: 55 | 230 | 247 bp |
| | | | | | |
| GSTP1 | X08058 | Forward | 5' TCC GCT GCA AAT ACA TCT CC 3'-SEQ ID NO: 56 | 305 | |
| | | Reverse | 5' TGT TTC CCG TTG CCA TTG AT 3 -SEQ ID NO: 57 | *616* | 331 bp |
| | | CT | 5' TGT TTC CCG TTG CCA TTG ATT AGG ACC TCA TGG ATC AGC A 3' –SEQ ID NO: 58 | 485 | 220 bp |
| | | | | | |
| GSTT1 | X79389 | Forward | 5' GCT CTA CCT GGA CCT GCT GT 3' -SEQ ID NO: 59 | 12 | |
| | | Reverse | 5' GGA ACA CAG GGA ACA TCA CC 3' -SEQ ID NO:60 | 351 | 359 bp |
| | | CT | 5' GGA ACA CAG GGA ACA TCA CCT AGA GCA GGA TGG CCA CAC T 3' –SEQ ID NO: 61 | 199 | 227 bp |
| | | | | | |
| GSTZ1 | U86529 | Forward | 5' TCA CCC CCT ACC CTA CCA TCA GC 3' -SEQ ID NO: 62 | 806 | |
| | | Reverse | 5' ATT TCA GCG CGG GCA TTC TIT 3' SEO ID NO: 63 | 1267 | 482 bp |
| | | CT | 5' ATT TCA GCG CGG GCA TTC TTT CCG CAT TCT CAT CTC AGC CTC AC 3' –SEQ ID NO: 64 | 1161 | 399 bp |
| | | | | | |
| mGST1 | J03746 | Forward | 5' GTC CGA GCA CGG ATC TAC CAC A 3' -SEQ ID NO: 65 | 404 | |
| | | Reverse | 5' TTC CTC TGC TCC CCT CCT ACC TA 3' -SEQ ID NO: 66 | 623 | 242 bp |
| | | CT | 5' TTC CTC TGC TCC CCT CCT ACC TAT TTT CAG CAA CCT GTA AGC C 3' –SEQ ID NO: 67 | 505 | 144 bp |
| | | | | | |
| SOD1 | X02317 | Forward | 5' TGA AGG TGT GGG GAA GCA TTA 3'-SEQ ID NO: 68 | 153 | |
| | | Reverse | 5' TTA CAC CAC AAG CCA AAC GAC 3' -SEQ ID NO: 69 | 492 | 360 bp |
| | | CT | 5' TTA CAC CAC AAG CCA AAC GAC TGA TGC AAT GGT CTC CTG AGA 3' -SEQ ID NO: 70 | 384 | 273 bp |
| | | | | | |
| XPA | D14533 | Forward | 5' CTC GGC GAC GGC GGC TGC GGC TAC TGG AG 3' -SEQ ID NO: 71 | 178 | |
| | | Reverse | 5' TGT CGG ACT TCC TTT GCT TCT TCT AAT GC 3' -SEQ ID NO: 72 | 629 | 480 bp |
| | | CT | 5' TGT CGG ACT TCC TTT GCT TCT TCT AAT GCT CTT TTT TCT AAA TCA CAG TCT 3' –SEQ ID NO: 73 | 487 | 360 bp |
| | | | | | |
| XRCC1 | M36089 | Forward | 5' CCC CTG AAG AGA CCA AAG CA 3' -SEQ ID NO: 74 | 1906 | |
| | | Reverse | 5' CCA TTC AAG GCT GTG ACG TA 3' -SEQ ID NO: 75 | 2241 | 355 bp |
| | | CT | [?] 5' CCA TTG AAG GCT GTG ACG TAT CAG GGA CTG GCA GAT G 3' -SEQ ID NO: 76 | 2142 | 276 bp |

**Table 2 : Demographic data of patients providing NBEC samples**

| Subject # | Group | Age | Gender | Histology | Smoking Hx² | Ethnicity |
|---|---|---|---|---|---|---|
| 63 | NBCI¹ | 77 | M | | 75 | W³ |
| 64 | NBCI | 47 | F | | 45 | W |
| 136 | NBCI | 38 | M | | 25 | A.A.⁴ |
| 139 | NBCI | 44 | F | | 17.5 | W |
| 150 | NBCI | 70 | F | | 45 | W |
| 156 | NBCI | 46 | F | | NS⁵ | A.A. |
| 157 | NBCI | 60 | M | | >100 | W |
| 194 | NBCI | 57 | M | | 3 | W |
| 210 | NBCI | 40 | M | | 34 | W |
| 257 | NBCI | 69 | F | | 20 | W |
| 261 | NBCI | 73 | F | | NS | W |
| 282 | NBCI | 83 | F | | 60 | W |
| 285 | NBCI | 69 | F | | NS | W |
| 296 | NBCI | 43 | F | | 20 | W |
| 305 | NBCI | 50 | F | | 40 | W |
| 315 | NBCI | 64 | M | | N/A⁶ | W |
| 330 | NBCI | 39 | M | | NS | W |
| 331 | NBCI | N/A | N/A | | N/A | N/A |
| 334 | NBCI | 51 | M | | >50 | W |
| 336 | NBCI | 31 | F | | NS | W |
| 337 | NBCI | 32 | M | | 22 | N/A |
| 339 | NBCI | 59 | M | | 50 | W |
| 361 | NBCI | 73 | F | | NS | H⁷ |
| 363 | NBCI | 50 | M | | 20 | A.A. |
| 34 | BCI⁸ | 80 | M | NSCLC⁹ | 40 | W |
| 71 | BCI | 63 | M | NSCLC | 100 | W |
| 85 | BCI | 73 | F | SQ¹⁰ | >100 | W |
| 88 | BCI | 85 | M | SQ | 75 | W |
| 99 | BCI | 63 | M | NSCLC | 45 | W |
| 118 | BCI | 72 | M | SQ | 30 | W |
| 146 | BCI | 64 | F | SULC¹¹ | 45 | W |
| 147 | BCI | 76 | M | SCLC | 75 | W |
| 158 | BCI | 88 | M | SCLC | 115.5 | W |
| 167 | BCI | 60 | F | NSCLC | 50 | W |
| 171 | BCI | 67 | M | SCLC | 100 | W |
| 191 | BCI | 75 | M | SQ | 54 | W |
| 211 | BCI | 71 | M | SQ | 50 | W |
| 212 | BCI | 65 | M | SQ | 67.5 | W |
| 247 | BCI | 75 | F | SQ | 50 | W |
| 255 | BCI | 60 | F | NSCLC | 30 | W |
| 259 | BCI | 68 | M | CS¹² | 137.5 | W |
| 271 | BCI | 58 | M | AC¹³ | 94.5 | W |
| 287 | BCI | 65 | F | NSCLC | 50 | W |
| 300 | BCI | 56 | M | SQ | 34 | W |
| 306 | BCI | 46 | M | SQ | 30 | W |
| 314 | BCI | 69 | F | BC¹⁴ | NS | W |
| 329 | BCI | 76 | F | PD¹⁵ | >37.5 | W |
| 335 | BCI | 75 | M | SCLC | 58 | A.A. |
| B3 | BCI | 63 | M | SQ | 60 | W |

| | | | | | | |
|---|---|---|---|---|---|---|
| ¹Non-bronchogenic carcinoma individual; ²Pack years; ³White; ⁴African-American; ⁵Non-smoker; ⁶Not available; hispanic; ⁸Bronchogenic carcinoma individual; ⁹Non-small cell lung cancer; ¹⁰Squamous carcinoma; ¹¹Small cell lung cancer; ¹²Carcinoma-in-situ; ¹³ Adenocarcinoma; ¹⁴Bronchogenic Cancer, histology not specified; ¹⁵Poorly differentiated carcinoma. | | | | | | |

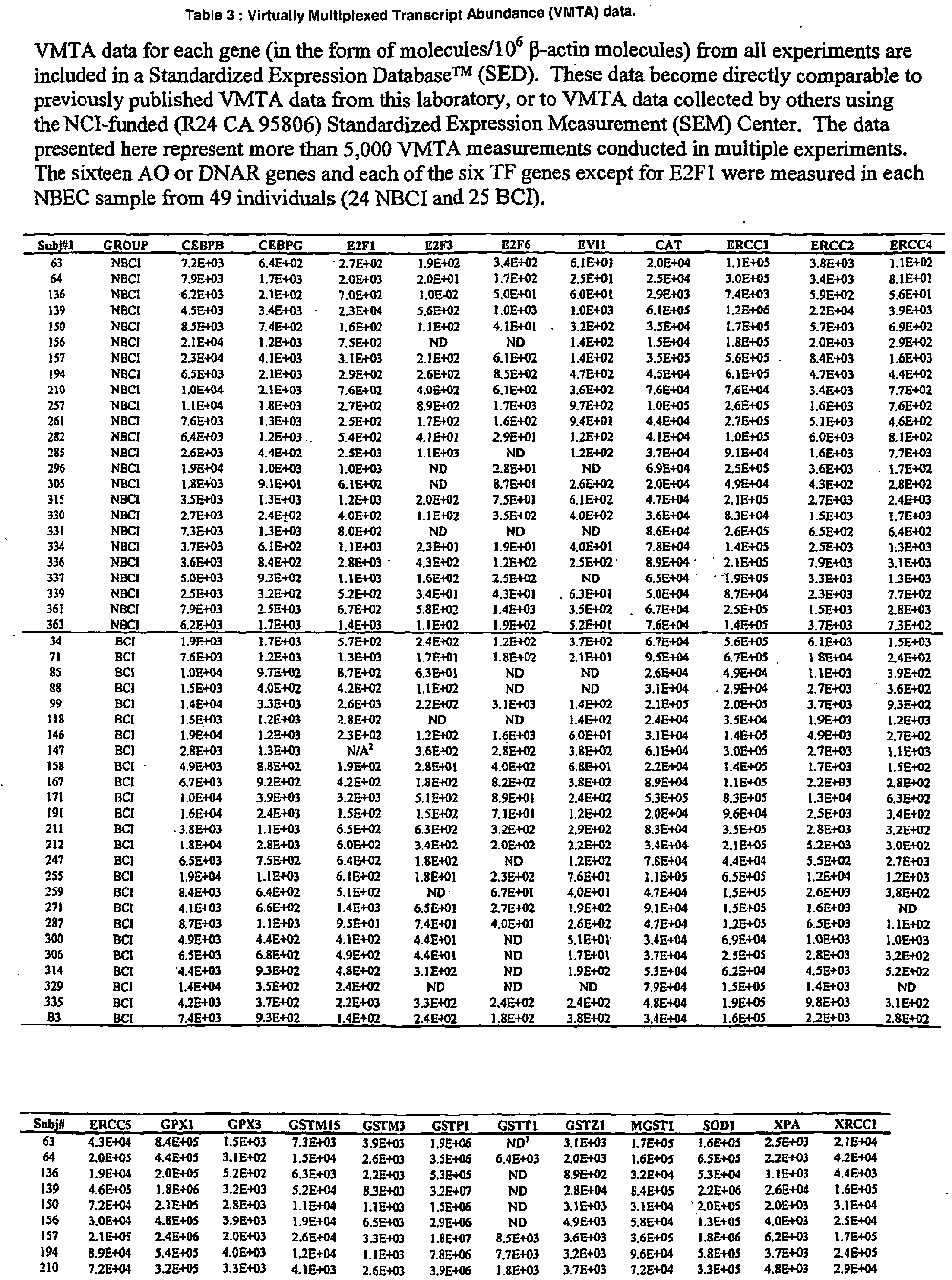

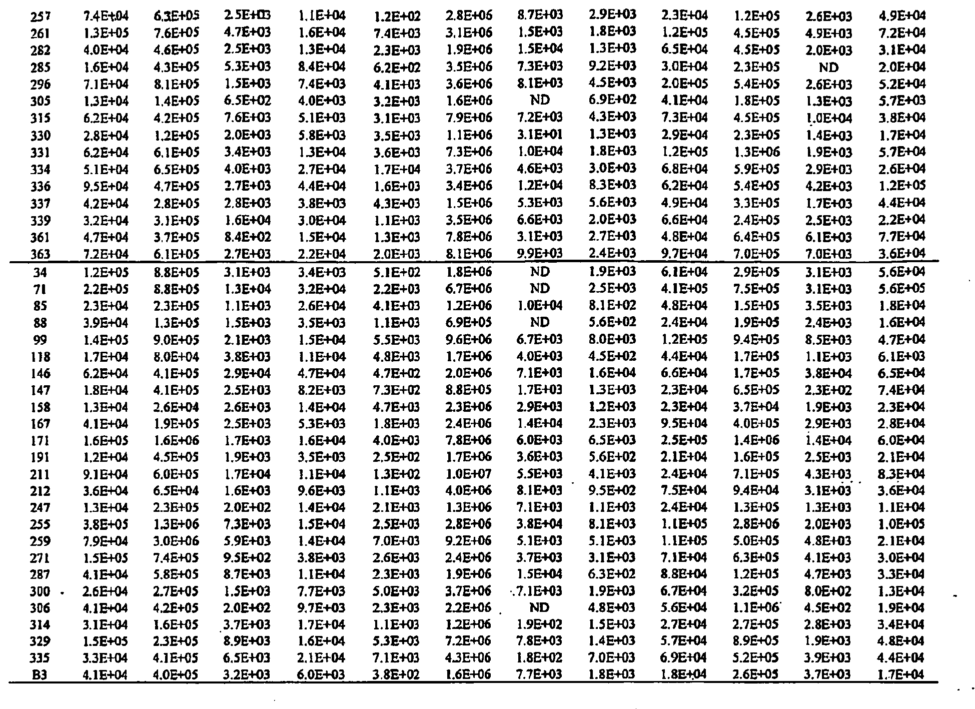

**Table 4: Pearson Analysis of Normalized VMTA Values for Antioxidant and DNA Repair Genes and Putative Regulatory Transcription Factors.**

| AO/DNAR Genes vs TFs | NBCI n=24 | | BCI n=25 | | ALL n=49 | |
|---|---|---|---|---|---|---|
| | r Value | p Value | r Value | p Value | r Value | p Value |
| CAT vs CEBPB | 0.13 | 1 | 0.18 | 1 | 0.15 | 1 |
| CAT vs CEBPG | 0.65 | **0.004** | 0.35 | 0.48 | 0.55 | **<0.0006** |
| CAT vs E2F1 * | 0.54 | 0.04 | 0.68 | **0.002** | 0.56 | **<0.0006** |
| CAT vs E2F3 | 0.48 | 0.12 | 0.18 | 1 | 0.37 | 0.06 |
| CAT vs E2F6 | 0.26 | 1 | 0.3 | 0.84 | 0.25 | 0.48 |
| CAT vs EVI1 | -0.01 | 1 | 0.21 | 1 | 0.08 | 1 |
| ERCC1 vs CEBPB | 0.32 | 0.78 | 0.27 | 1 | 0.29 | 0.24 |
| ERCC1 vs CEBPG | 0.77 | **<0.0006** | 0.42 | 0.24 | 0.62 | **<0.0006** |
| ERCC1 vs E2F1 | 0.35 | 0.54 | 0.39 | 0.36 | 0.37 | 0.06 |
| ERCC1 vs E2F3 | 0.39 | 0.36 | 0.21 | I | 0.31 | 0.18 |
| ERCC1 vs E2F6 | 0.17 | 1 | 0.63 | **0.005** | 0.42 | 0.02 |
| ERCC1 vs EVI1 | -0.02 | 1 | 0.38 | 0.36 | 0.17 | 1 |
| ERCC2 vs CEBPB | 0.25 | 1 | 0.19 | 1 | - 0.22 | 0.84 |
| ERCC2 vs CEBPG | 0.63 | **0.006** | 0.39 | 0.3 | 0.53 | **<0.0006** |
| ERCC2 vs E2F1 | 0.39 | 0.36 | 0.32 | 0.72 | 0.33 | 0.12 |
| ERCC2 vs E2F3 | 0.58 | 0.02 | 0.22 | 1 | 0.42 | 0.02 |
| ERCC2 vs E2F6 | 0.37 | 0.42 | 0.51 | 0.06 | 0.42 | 0.02 |
| ERCC2 vs EVI1 | 0.19 | 1 | 0.29 | 0.96 | 0.23 | 0.66 |
| ERCC4 vs CEBPB | -0.35 | 0.6 | -0.11 | 1 | -0.16 | 1 |
| ERCC4 vs CEBPG | 0.24 | 1 | 0.37 | 0.42 | 0.25 | 0.48 |
| ERCC4 vs E2F1 | 0.42 | 0.24 | 0.04 | 1 | 0.2 | 1 |
| ERCC4 vs E2F3 | 0.6 | 0.01 | 0.33 | 0.6 | 0.33 | 0.12 |
| ERCC4 vs E2F6 | -0.04 | 1 | 0.04 | 1 | 0.07 | 1 |
| ERCC4 vs EVI1 | 0.24 | 1 | 0.33 | 0.66 | 0.27 | 0.36 |
| ERCC5 vs CEBPB | 0.4 | 0.3 | 0.28 | 1 | 0.33 | 0.12 |
| ERCC5 vs CEBPG | 0.79 | **<0.0006** | 0.12 | 1 | 0.46 | **0.005** |
| ERCC5 vs E2F1 | 0.44 | 0.18 | 0.45 | 0.18 | 0.44 | **0.01** |
| ERCC5 vs E2F3 | 0.39 | 0.36 | -0.11 | 1 | 0.13 | 1 |
| ERCC5 vs E2F6 | 0.41 | 0.3 | 0.35 | 0.54 | 0.38 | 0.04 |
| ERCC5 vs EVI1 | 0.07 | 1 | -0.04 | 1 | 0.01 | 1 |
| GPX1 vs CEBPB | 0.49 | 0.06 | 0.24 | 1 | 0.32 | 0.12 |
| GPX1 vs CEBPG | 0.72 | **<0.0006** | 0.19 | 1 | 0.4 | 0.02 |
| GPX1 vs E2F1 | 0.48 | 0.12 | 0.38 | 0.36 | 0.43 | 0.02 |
| GPX1 vs E2F3 | 0.22 | 1 | -0.004 | 1 | 0.08 | 1 |
| GPX1 vs E2F6 | 0.06 | 1 | 0.36 | 0.48 | 0.28 | 0.3 |
| GPX1 vs EVI1 | -0.06 | 1 | 0.2 | 1 | 0.1 | 1 |
| GPX3 vs CEBPB | -0.18 | 1 | 0.14 | 1 | 0.02 | 1 |
| GPX3 vs CEBPG | 0.13 | 1 | 0.01 | 1 | 0.07 | 1 |
| GPX3 vs E2F1 | -0.03 | 1 | -0.17 | 1 | -0.12 | 1 |
| GPX3 vs E2F3 | 0.32 | 0.78 | -0.2 | 1 | 0.05 | 1 |
| GPX3 vs E2F6 | -0.26 | 1 | 0.44 | 0.18 | 0.19 | 1 |
| GPX3 vs EVI1 | 0.06 | 1 | 0.08 | 1 | 0.06 | 1 |
| GSTMI-5 vs CEBPB | -0.08 | 1 | 0.43 | 0.18 | 0.17 | 1 |
| GSTM1-5 vs CEBPG | 0.25 | 1 | 0.02 | 1 | 0.16 | 1 |
| GSTM1-5 vs E2F1 | 0.51 | 0.06 | 0.23 | 1 | 0.41 | 0.02 |
| GSTM1-5 vs E2F3 | 0.29 | 0.96 | -0.16 | 1 | 0.1 | 1 |
| GSTM1-5 vs E2F6 | -0.3 | 0.9 | 0.006 | 1 | -0.1 | 1 |
| GSTM1-5 vs EVI1 | 0.22 | 1 | -0.12 | 1 | 0.07 | 1 |
| GSTM3 vs CEBPB | 0.01 | 1 | 0.06 | 1 | 0.04 | 1 |
| GSTM3 vs CEBPG | -0.007 | 1 | -0.28 | 1 | 0.01 | 1 |
| GSTM3 vs E2F1 | 0.29 | 1 | 0.35 | 0.54 | 0.34 | 0.12 |
| GSTM3 vs E2F3 | -0.31 | 0.84 | -0.49 | 0.06 | -0.4 | 0.03 |
| GSTM3 vs E2F6 | -0.11 | 1 | -0.25 | I | -0.16 | 1 |
| GSTM3 vs EVI1 | -0.27 | 1 | -0.25 | 1 | -0.25 | 0.54 |
| GSTP1 vs CEBPB | 0.19 | 1 | 0.38 | 0.36 | 0.28 | 0.36 |
| GSTP1 vs CEBPG | 0.74 | **<0.0006** | 0.18 | 1 | 0.51 | **0.001** |
| GSTP1 vs E2F1 | 0.6 | **0.01** | 0.46 | 0.12 | 0.56 | **<0.0006** |
| GSTP1 vs E2F3 | 0.32 | 0.78 | -0.25 | 1 | 0.07 | 1 |
| GSTP1 vs E2F6 | 0.1 | 1 | 0.35 | 0.48 | 0.26 | 0.42 |
| GSTP1 vs EVI1 | 0.11 | 1 | 0.13 | 1 | 0.12 | 1 |
| GSTT1 vs CEBPB | 0.03 | 1 | 0.45 | 0.12 | 0.24 | 0.6 |
| GSTT1 vs CEBPG | 0.39 | 0.36 | 0.16 | 1 | 0.3 | 0.24 |
| GSTT1 vs E2F1 | 0.07 | 1 | -0.15 | 1 | -0.05 | 1 |
| GSTT1 vs E2F3 | 0.35 | 0.54 | -0.1 | 1 | 0.17 | 1 |
| GSTT1 vs E2F6 | 0.05 | 1 | 0.21 | 1 | 0.11 | 1 |
| GSTT1 vs EVI1 | -0.26 | 1 | 0.22 | 1 | -0.04 | 1 |
| GSTZ1 vs CEBPB | 0.11 | 1 | 0.36 | 0.42 | 0.25 | 0.54 |
| GSTZ1 vs CEBPG | 0.51 | 0.06 | 0.08 | 1 | 0.28 | 0.3 |
| GSTZ1 vs E2F1 | 0.64 | **0.004** | 0.5 | 0.06 | 0.58 | **<0.0006** |
| GSTZ1 vs E2F3 | 0.42 | 0.24 | 0.14 | 1 | 0.25 | 0.54 |
| GSTZ1 vs E2F6 | -0.05 | 1 | 0.48 | 0.12 | 0.32 | 0.18 |
| GSTZ1 vs EVI1 | 0.02 | 1 | 0.27 | 1 | 0.16 | 1 |
| mGST vs CEBPB | 0.31 | 0.78 | 0.35 | 0.48 | 0.32 | 0.12 |
| mGST vs CEBPG | 0.56 | 0.02 | 0.25 | 1 | 0.42 | 0.02 |
| mGST vs E2F1 | 0.58 | 0.02 | 0.54 | 0.04 | 0.58 | **<0.0006** |
| mGST vs E2F3 | 0.03 | 1 | -0.15 | 1 | -0.06 | 1 |
| mGST vs E2F6 | 0.17 | 1 | 0.29 | 0.96 | 0.27 | 0.36 |
| mGST vs EVI1 | -0.16 | 1 | 0.07 | 1 | -0.04 | 1 |
| SOD1 vs CEBPB | 0.13 | 1 | 0.15 | 1 | 0.14 | 1 |
| SOD1 vs CEBPG | 0.66 | **0.002** | 0.009 | 1 | 0.36 | 0.06 |
| SOD1 vs E2F1 | 0.59 | 0.02 | 0.55 | 0.04 | 0.56 | **<0.0006** |
| SOD1 vs E2F3 | 0.25 | 1 | -0.07 | 1 | 0.09 | 1 |
| SOD1 vs E2F6 | 0.12 | 1 | 0.14 | 1 | 0.14 | 1 |
| SOD1 vs EVI1 | -0.17 | 1 | 0.03 | 1 | -0.06 | 1 |
| XPA vs CEBPB | 0.31 | 0.84 | 0.42 | 024 | 0.31 | 0.18 |
| XPA vs CEBPG | 0.36 | 0.54 | 0.33 | 0.66 | 0.34 | 0.12 |
| XPA vs E2F1 | -0.05 | 1 | 0.22 | 1 | -0.02 | 1 |
| XPA vs E2F3 | -0.07 | 1 | 0.14 | 1 | -0.01 | 1 |
| XPA vs E2F6 | 0.55 | 0.04 | 0.46 | 0.12 | 0.4 | 0.02 |
| XPA vs EVI1 | 0.04 | 1 | 0.07 | 1 | 0.04 | 1 |
| XRCC1 vs CEBPB | 0.36 | 0.48 | 0.28 | 1 | 0.32 | 0.18 |
| XRCC1 vs CEBPG | 0.83 | **<0.0006** | 0.27 | 1 | 0.591 | **<0.0006** |
| XRCC1 vs E2F1 | 0.32 | 0.78 | 0.37 | 0.48 | 0.35 | 0.12 |
| XRCC1 vs E2F3 | 0.47 | 0.12 | 0.22 | 1 | 0.35 | 0.06 |
| XRCC1 vs E2F6 | 0.26 | 1 | 0.54 | 0.04 | 0.41 | 0.02 |
| XRCC1 vs EVI1 | -0.009 | 1 | 0.12 | 1 | 0.06 | 1 |

| | | | | | | |
|---|---|---|---|---|---|---|
| Table 4 presents correlation coefficient (r value) and level of significance (p value) for each correlation derived from Pearson's correlation analysis of normalized VMTA data in Table 2. The correlation of each of the six TFs and each of the sixteen AO or DNAR genes is determined by Pearson's correlation following logarithmic transformation, necessary due to the wide range of expression of each gene among the samples. Significance (p < 0.01) is determined using a two- tailed test following Bonferroni adjustment for multiple comparison (comparison of each of six TFs to each of the AO or DNAR genes). *values not obtained in one BCI (see Examples). | | | | | | |

| **Table 5** | | | | |
|---|---|---|---|---|
| **Correlation of CEBPG with Each of Ten Antioxidant or DNA Repair Genes in Non-Bronchogenic Carcinoma Individuals (NBCI) or Bronchogenic Carcinoma Individuals (BCI)** | | | | |
| **Individuals Combined from Studies 2 and 3** | **NBCI (N=24)** | | **BCI (N=25)** | |
| | **Correlation Coefficient** | **P value** | **Correlation Coefficient** | **P value** |
| **CEBPG** | | | | |
| CAT | 0.65 | 0.0006 | 0.35 | 0.08 |
| ERCC1 | 0.77 | <0.0001 | 0.42 | 0.04 |
| ERCC2 | 0.63 | 0.001 | 0.39 | 0.05 |
| ERCC5 | 0.79 | <0.0001 | 0.12 | 0.57 |
| GPX1 | 0.72 | <0.0001 | 0.19 | 0.37 |
| GSTP1 | 0.74 | <0.0001 | 0.18 | 0.4 |
| GSTZ1 | 0.51 | 0.01 | 0.08 | 0.71 |
| mGST1 | 0.56 | 0.004 | 0.25 | 0.22 |
| SOD1 | 0.66 | 0.0004 | 0.009 | 0.97 |
| XRCC1 | 0.83 | <0.0001 | 0.27 | 0.2 |
| All genes | 0.69 +/-0.10 | 0.003 +/-0.004 | 0.23 +/-0.13 | 0.36 +/-0.29 |
| Antioxidant Genes | 0.64+/-0.09 | 0.004 +/-0.004 | 10.18 +/-0.12 | 0.46 +/-0.33 |
| DNA Repair Genes | 0.76/-0.09 | 0.001 | 0.3 +/-0.14 | 0.22 +/-0.25 |

| **Table 6** | | | | |
|---|---|---|---|---|
| **Correlation of CEBPG with Each of She Antioxidant or DNA Repair Genes in Non-Bronchogenic Carcinoma Individuals (NBCI) or Bronchogenic Carcinoma Individuals (BCI)** | | | | |
| **Individuals Combined from Studies 2 and 3** | **NBCI (N = 24)** | | **BCI (N=25)** | |
| | **Correlation Coefficient** | **P value** | **Correlation Coefficient** | **P value** |
| **CEBPG** | | | | |
| ERCC4 | 0.23 | 0.31 | 0.18 | 0.42 |
| GPX3 | 0.13 | 0.55 | 0.01 | 0.96 |
| GSTM3 | -0.007 | 0.98 | -0.28 | 0.17 |
| GSTM 1-5 | 0.25 | 0.23 | 0.02 | 0.92 |
| GSTT1 | 0.23 | 0.3 | -0.01 | 0.96 |
| XPA | 0.36 | 0.09 | 0.33 | 0.11 |
| All genes | 0.20 +/-0.12 | 0.41 +/-0.32 | 0.04 +/-0.20 | 0.59 +/-0.40 |
| Antioxidant Genes | 0.15 +/-0.12 | 0.52 +/-0.34 | -0.07 +/-0.14 | 0.75 +/-0.39 |
| DNA Repair Genes | 0.30 +/-0.09 | 0.2 +/-0.16 | 0.26 +/-0.11 | 0.27 +/-0.22 |

| **Table 7** | | | | |
|---|---|---|---|---|
| **Correlation of CEBPB with Each of Ten Antioxidant or DNA Repair Genes in Non-Bronchogenic Carcinoma Individuals (NBCI) or Bronchogenic Carcinoma Individuals (BCI)** | | | | |
| **Individuals Combined from Studies 2 and 3** | **NBCI (N=24)** | | **BCI (N=25)** | |
| | **Correlation Coefficient** | **P value** | **Correlation Coefficient** | **P value** |
| **CEBPB** | | | | |
| CAT | 0.13 | 0.54 | 0.18 | 0.4 |
| ERCC1 | 0.32 | 0.13 | 0.27 | 0.19 |
| ERCC2 | 0.25 | 0.24 | 0.19 | 0.37 |
| ERCC5 | 0.4 | 0.05 | 0.28 | 0.18 |
| GPX1 | 0.49 | 0.01 | 0.24 | 0.24 |
| GSTP1 | 0.19 | 0.37 | 0.38 | 0.06 |
| GSTZ1 | 0.11 | 0.62 | 0.36 | 0.07 |
| mGST1 | 0.31 | 0.13 | 0.35 | 0.08 |
| SOD1 | 0.13 | 0.56 | 0.15 | 0.48 |
| XRCC1 | 0.36 | 0.08 | 0.28 | 0.18 |
| All genes | 0.27 +/-0.13 | 0.27 +/-0.23 | 0.27 +/-0.08 | 0.23 +/-0.15 |
| Antioxidant Genes | 0.23 +/-0.15 | 0.37 +/-0.25 | 0.28 +/-0.10 | 0.22+/-0.18 |
| DNA Repair Genes | 0.33 +/-0.06 | 0.13 +/-0.08 | 0.26 +/-0.04 | 0.23 +/-0.09 |

| **Table 8** | | | | |
|---|---|---|---|---|
| **Correlation of E2F1 with Each of Ten Antioxidant or DNA Repair Genes in Non-Bronchogenic Carcinoma Individuals (NBCI) or Bronchogenic Carcinoma Individuals (BCI)** | | | | |
| **Individuals Combined from Studies 2 and 3** | **NBCI (N=24)** | | **BCI (N=25)** | |
| | **Correlation Coefficient** | **P value** | **Correlation Coefficient** | **P value** |
| **E2F1** | | | | |
| CAT | 0.54 | 0.007 | 0.68 | 0.0003 |
| ERCC1 | 0.35 | 0.09 | 0.39 | 0.06 |
| ERCC2 | 0.39 | 0.06 | 0.32 | 0.12 |
| ERCC5 | 0.44 | 0.03 | 0.45 | 0.03 |
| GPX1 | 0.48 | 0.02 | 0.38 | 0.06 |
| GSTP1 | 0.6 | 0.002 | 0.46 | 0.02 |
| GSTZ1 | 0.64 | 0.0007 | 0.5 | 0.01 |
| mGST1 | 0.58 | 0.003 | 0.54 | 0.006 |
| SOD1 | 0.59 | 0.003 | 0.55 | 0.006 |
| XRCC1 | 0.32 | 0.13 | 0.37 | 0.08 |
| All genes | 0.49 +/-0.11 | 0.03 +/-0.04 | 0.46 +/-0.11 | 0.04 +/-0.04 |
| Antioxidant Genes | 0.57+/-0.06 | 0.006 +/-0.007 | 0.52 +/-0.10 | 0.02 +/-0.02 |
| DNA Repair Genes | 0.38/-0.05 | 0.08+/-0.04 | 0.38 +/-0.05 | 0.07 +/-0.04 |

| **Table 9** | | **SNP Data for CEBPG, A, B** | | | | | | |
|---|---|---|---|---|---|---|---|---|
| | | | Assay | Pop Data | Tot # Pop | Tot # indlv | | |
| | | | Sample | Sample | w/Freq | w/ Geno | Ave | Ave Allele |
| Gene | Position | SNP | Size | Size | Data | Data | Hetero | Freq |
| | | | | | | | | G=0.577 |
| CEBPG | Intron 1 | A/G | 63 | N/A | 0 | 331 | 0.488 | A=0.423 |
| | Overlap | | | | | | | |
| | w/ 3' UTR | G/T | 4 | N/A | 0 | 0 | N/A | N/A |
| | Overlap | | | | | | | G=0.946 |
| | w/ 3' UTR | A/G | 94 | 1494 | 1 | 331 | 0.102 | A=0.054 |
| | Overlap | | | | | | | T=0.980 |
| | w/ 3' UTR | G/T | 10 | 184 | 1 | 0 | 0.039 | G=0.020 |
| | Overlap | | | | | | | |
| | w/ 3' UTR | A/G | 8 | N/A | 0 | 0 | 0 | N/A |
| | Overlep | | | | | | | A=0.896 |
| | w/ 3' UTR | A/C | 97 | 184 | 1 | 71 | 0.187 | G=0.104 |
| | Overlap | | | | | | | C=0.853 |
| CEBPA | w/ 3' UTR | C/T | 24 | 372 | 2 | 269 | 0.251 | T=0.147 |
| | Overlap | | | | | | | C=0.850 |
| | w/ 3' UTR | C/G | 18 | 184 | 1 | 0 | 0.255 | G=0.150 |
| | Overlap | | | | | | | |
| | w/ 3' UTR | C/T | 10 | N/A | 0 | 0 | 0 | N/A |
| | Overlap | | | | | | | |
| | w/ 3' UTR | A/G | 15 | N/A | 0 | 0 | 0 | N/A |
| | Overlap | | | | | | | |
| | w/ 3' UTR | C/T | 2 | N/A | 0 | 0 | 0 | N/A |
| | Overlap | | | | | | | |
| | w/ Exon 1 | G/T | 10 | N/A | 0 | 0 | 0 | N/A |
| | Overlap | | | | | | | |
| | w/ Exon 1 | A/C | 48 | N/A | 0 | 0 | 0 | N/A |
| | Overlap | | | | | | | |
| CEBPB | w/ Exon 1 | A/G | 2 | N/A | 0 | 0 | 0 | N/A |
| | Overlap | | | | | | | C=0.691 |
| | w/ Exon 1 | C/T | 50 | 94 | 2 | 47 | 0.427 | T=0.309 |
| | Overlap | | | | | | | G=0.979 |
| | w/ Exon 1 | A/G | 48 | 48 | 1 | 24 | 0.041 | A=0.021 |
| | Overlap | | | | | | | G=1.000 |
| | w/ 3' UTR | A/G | 3 | 184 | 1 | 0 | 0 | A=0.000 |

| **Table 10** | | | **Target Gene SNPS** | | | | | |
|---|---|---|---|---|---|---|---|---|
| | | | | | | | | C= 0.993 |
| XRCC1 | 59 | C/T | 152 | 152 | 1 | 90 | 0.013 | T= 0.007 |
| | 59 | A/G(Genomic Reverse) | N/A | N/A | N/A | N/A | N/A | N/A |
| | | | | | | | | T= 0.776 |
| | 72 | C/T | 207 | 152 | 1 | 90 | 0.347 | C= 0.224 |
| | 72 | A/G(Genomic Reverse) | N/A | N/A | N/A | N/A | N/A | N/A |
| | | | | | | | | C= 0.987 |
| | 313 | A/C | 152 | 152 | 1 | 90 | 0.026 | A= 0.013 |
| | 313 | T/G(Genomic Reverse) | N/A | N/A | N/A | N/A | N/A | N/A |
| | | | | | | | | G= 0.994 |
| | 697 | A/G | 172 | 172 | 1 | 90 | 0.012 | A= 0.006 |
| | 697 | C/T(Genomie Reverse) | N/A | N/A | N/A | N/A | N/A | N/A |

| **Table 11** | **Measurable Biological Correlate** | | | | | | |
|---|---|---|---|---|---|---|---|
| Function affected by SNP | TG TA/ CEBPG TA | CEBPG TA level | Total CEBPG protein | TG TA level | *In vitro* Free/ Bound CEBPG | *Unimeric* /*heterodimeric CEBPG in NBEC* | *Free CEBPG*/ *Bound CEBPG in NBEC* |
| Low CEBPG transcription | High or no change | low | Low | Low | Low | low | Low |
| Low TF Heterodimer formation | Low | High | High | Low | High | High | High |
| Unstable TF transcript | High or no change | Low | Low | Low | Low | low | low |
| Low binding of TF to TG Recognition site | low | High | High | Low | High | low | High |
| Poor sub-cellular localization ofTF protein | High or no change | High | High | Low | No effect | low | low |
| Poor processing of TF for translation | Low | High | Low | Low | No effect | low | low |

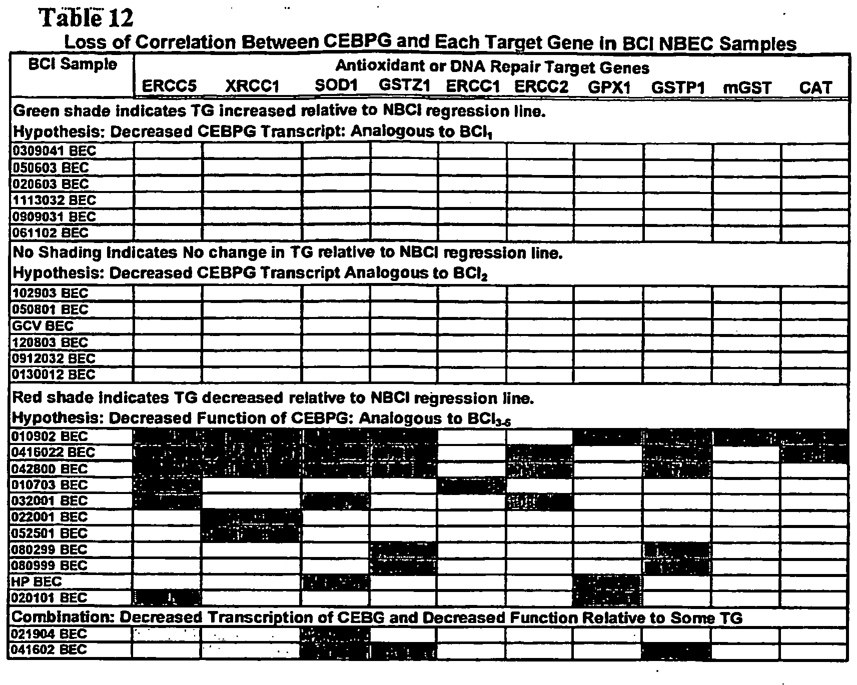

**Table 13 : ERCC5 Promoter Sequence Polymporphisms at -228 and -222**

| **Subject #** | **Group** | **Position Relative to CEBPG vs ERCC5 Regression Line** | **T-228G Polymorphism** | **G-222A Polymorphism** |
|---|---|---|---|---|
| 315 | non-BC | On | T | G |
| 305 | non-BC | On | T | G |
| 194 | non-BC | On | T | G |
| 157 | non-BC | On | T | G |
| 261 | non-BC | On | T | Hetero G/A |
| 156 | non-BC | On | T | G |
| 334 | non-BC | On | T | G |
| 210 | non-BC | On | T | A |
| 337 | non-BC | On | T | Hetero G/A |
| 339 | non-BC | On | T | Hetero G/A |
| 285 | non-BC | Below | Hetero G/T | Hetero G/A |
| BEP2D | immortalized | On | T | G |
| 211 | BC | On | T | G |
| 287 | BC | On | T | G |
| 99 | BC | On | T | Hetero G/A |
| 271 | BC | Above | T | G |
| 329 | BC | Above | T | G |
| 255 | BC | Above | T | Hetero G/A |
| 147 | BC | Below | T | G |
| 247 | BC | Below | T | G |
| 191 | BC | Below | T | G |
| 212 | BC | Below | T | G |
| H23 | BC | Below | T | G |

### Sequence Listings

5' GAT CCC AGA AAA TAG CCT CCA ATG AAC ATT CAA GCC ACA AGC TC 3' (SEQ ID. NO. 1)
5' AAA GCA GGA GGG AAC AGA GCA CTG CAG GGA CCA CAG G 3' (SEQ ID. NO. 2)
5' CAA ACA CAG ATC TGG CGG TCA CGA GGA GCT TCC TTC ACT GAG TTC TGC GAA T 3' (SEQ ID. NO. 3)
5' CAA TGG TCT GGA AGC GGC GGA CCG GAG ACC AGG TGA TGA G 3' (SEQ ID. NO. 4)
5' TGT TTC CCG TTG CCA TTG ATT AGG ACC TCA TGG ATC AGC A 3' (SEQ ID. NO. 5)
5' TTA CAC CAC AAG CCA AAC GAC TGA TGC AAT GGT CTC CTG AGA 3' (SEQ. ID. NO. 6)
5' CCA TTG AAG GCT GTG ACG TAT CAG GGA CTG GCA GAT G 3' (SEQ ID. NO. 7)

## Claims

1. A method of identifying a biomarker that indicates a biological state, comprising:
assaying in a plurality of control samples expression levels of a gene and of a first transcription factor;
assaying in a plurality of case samples expression levels of said gene and of said first transcription factor; and
deducing whether said expression levels of said gene are correlated with said expression levels of said first transcription factor in said control samples but
not correlated in said case samples, thereby identifying a biomarker for said biological state,
wherein at least one of said expression levels is assayed by assaying abundance of an mRNA transcript and wherein assaying said mRNA transcript abundance comprises:
mixing a nucleic acid corresponding to said gene with a competitive template for said gene;
measuring a nucleic acid corresponding to said first transcription factor in relation to a competitive template for said first transcription factor; and
obtaining a relation comparing said transcription factor to said competitive template; and
(a) wherein said biological state is bronchogenic carcinoma or risk thereof and said first transcription factor is CCAAT/enhancer-binding protein gamma (CEBPG), and wherein said gene is X-ray repair complementing defective repair in Chinese hamster cells 1 (XRCC1), catalase (CAT), excision repair cross-complementing rodent repair deficiency, complementation group 1 (ERCC1), excision repair cross-complementing rodent repair deficiency, complementation group 2 (ERCC2), excision repair cross-complementing rodent repair deficiency, complementation group 5 (ERCC5), glutathione transferase zeta 1 (GSTZ1), microsomal glutathione S-transferase 1 (mGST1), glutathione S-transferase pi (GSTP1), superoxide dismutase 1 (SOD1) or glutathione peroxidase 1 (GPX1); or
(b) wherein said biological state is bronchogenic carcinoma or risk thereof and said first transcription factor is E2F1, and wherein said gene is ERCC5, GSTP1 or SOD1; or
(c) wherein said biological state is COPD or risk thereof and said first transcription factor is CEBPG, and wherein said gene is XRCC1, ERCC1, ERCC2, CAT, ERCC5, GSTZ1, mGST1, GSTP1, SOD1 or GPX1; or
(d) wherein said biological state is COPD or risk thereof and said first transcription factor is E2F1, and wherein said gene is ERCC5, GSTP1 or SOD1.

2. The method as recited in claim 1, further comprising assaying expression levels of one or more additional transcription factors associated with said biological state.

3. The method as recited in claim 2, wherein said gene is regulated by said first transcription factor and by said one or more additional transcription factors in said control samples.

4. The method as recited in any one of claims 1 to 3, wherein said competitive templates are provided in a standardized mixture.

5. The method as recited in any one of claims 1 to 4, wherein assaying said mRNA transcript abundance comprises:
co-amplifying a nucleic acid corresponding to said gene with a competitive template for said gene;
co-amplifying a nucleic acid corresponding to said first transcription factor with
a competitive template for said first transcription factor; and
obtaining a relation comparing amplified products obtained from said co-amplifications.

6. The method as recited in any one of claims 1 to 5, wherein
(i) more than 10 case samples and more than 10 control samples are used, or
(ii) more than 30 case samples and more than 30 control samples are used, or
(iii) more than 50 case samples and more than 50 control samples are used, or
(iv) more than 100 case samples and more than 100 control samples are used.

7. The method as recited in any one of claims 1 to 6, further comprising making a diagnostic decision based on identifying said biomarker.

## Patentansprüche

1. Verfahren zur Identifizierung eines Biomarkers, der einen biologischen Zustand anzeigt, umfassend:
Testen der Expressionsniveaus eines Gens und eines ersten Transkriptionsfaktors in einer Vielzahl von Kontrollproben;
Testen der Expressionsniveaus des Gens und des ersten Transkriptionsfaktors in einer Vielzahl von Proben von Krankheitsfällen; und
Rückschließen, ob die Expressionsniveaus des Gens mit den Expressionsniveaus des ersten Transkriptionsfaktors in den Kontrollproben korrelieren, aber nicht in den Proben der Krankheitsfälle korrelieren, wodurch ein Biomarker für den biologischen Zustand identifiziert wird,
wobei mindestens eines der Expressionsniveaus durch Testen des Überflusses eines mRNA-Transkripts getestet wird und wobei das Testen des Überflusses des mRNA-Transkripts umfasst:
Mischen einer dem Gen entsprechenden Nucleinsäure mit einer für das Gen kompetitiven Matrize;
Messen einer dem ersten Transkriptionsfaktor entsprechenden Nucleinsäure im Verhältnis zu einer für den ersten Transkriptionsfaktor kompetitiven Matrize; und
Erhalten eines Verhältnisses, das den Transkriptionsfaktor mit der kompetitiven Matrize vergleicht; und
(a) wobei der biologische Zustand ein bronchogenes Karzinom oder das Risiko dafür ist und der erste Transkriptionsfaktor CCAAT/Enhancerbindendes Protein gamma (CEBPG) ist, und wobei das Gen das X-ray repair complementing defective repair in Chinese hamster cells 1 (XRCC1), Katalase (CAT), excision repair cross-complementing rodent repair deficiency, complementation group 1 (ERCC1), excision repair cross-complementing rodent repair deficiency, complementation group 2 (ERCC2), excision repair cross-complementing rodent repair deficiency, complementation group 5 (ERCC5), Glutathion-Transferase zeta 1 (GSTZ1), microsomale Glutathion-S-Transferase 1 (mGST1), Glutathion-S-Transferase pi (GSTP1), Superoxiddismutase (SOD1) oder Glutathionperoxidase 1 (GPX1) ist; oder
(b) wobei der biologische Zustand ein bronchogenes Karzinom oder das Risiko dafür ist und der erste Transkriptionsfaktor E2F1 ist, und wobei das Gen ERCC5, GSTP1 oder SOD1 ist; oder
(c) wobei der biologische Zustand COPD oder das Risiko dafür ist und der erste Transkriptionsfaktor CEBPG ist, und wobei das Gen XRCC1, ERCC1, ERCC2, CAT, ERCC5, GSTZ1, mGST1, GSTP1, SOD1 oder GPX1 ist; oder
(d) wobei der biologische Zustand COPD oder das Risiko dafür ist und der erste Transkriptionsfaktor E2F1 ist, und wobei das Gen ERCC5, GSTP1 oder SOD1 ist.

2. Verfahren nach Anspruch 1, weiter umfassend das Testen der Expressionsniveaus von einem oder mehreren zusätzlichen Transkriptionsfaktoren, die mit dem biologischen Zustand in Zusammenhang stehen.

3. Verfahren nach Anspruch 2, wobei das Gen durch den ersten Transkriptionsfaktor und durch den einen oder die mehreren zusätzlichen Transkriptionsfaktoren in den Kontrollproben reguliert wird.

4. Verfahren nach einem der Ansprüche 1 bis 3, wobei die kompetitiven Matrizen in einer standardisierten Mischung bereitgestellt werden.

5. Verfahren nach einem der Ansprüche 1 bis 4, wobei das Testen des Überflusses des mRNA-Transkripts umfasst:
Co-Amplifizieren einer dem Gen entsprechenden Nucleinsäure mit einer für das Gen kompetitiven Matrize;
Co-Amplifizieren einer dem ersten Transkriptionsfaktor entsprechenden Nucleinsäure mit einer für den ersten Transkriptionsfaktor kompetitiven Matrize; und
Erhalten eines Verhältnisses, das die amplifizierten Produkte, die durch die Co-Amplifizierungen erhalten wurden, vergleicht.

6. Verfahren nach einem der Ansprüche 1 bis 5, wobei
(i) mehr als 10 Proben von Krankheitsfällen und mehr als 10 Kontrollproben verwendet werden, oder
(ii) mehr als 30 Proben von Krankheitsfällen und mehr als 30 Kontrollproben verwendet werden, oder
(iii) mehr als 50 Proben von Krankheitsfällen und mehr als 50 Kontrollproben verwendet werden, oder
(iv) mehr als 100 Proben von Krankheitsfällen und mehr als 100 Kontrollproben verwendet werden.

7. Verfahren nach einem der Ansprüche 1 bis 6, weiter umfassend das Treffen einer diagnostischen Entscheidung basierend auf der Identifizierung des Biomarkers.

## Revendications

1. Procédé d'identification d'un biomarqueur qui indique un état biologique, comprenant les étapes consistant à :
doser dans une pluralité d'échantillons contrôles des taux d'expression d'un gène et d'un premier facteur de transcription ;
doser dans une pluralité d'échantillons de cas des taux d'expression dudit gène et dudit premier facteur de transcription ; et
déduire si lesdits taux d'expression dudit gène sont en corrélation avec lesdits taux d'expression dudit premier facteur de transcription dans lesdits échantillons contrôles mais ne sont pas en corrélation dans lesdits échantillons de cas, identifiant de cette manière un biomarqueur pour ledit état biologique,
dans lequel au moins l'un desdits taux d'expression est dosé en dosant l'abondance d'un transcrit d'ARNm et dans lequel le dosage de ladite abondance du transcrit d'ARNm comprend les étapes consistant à :
mélanger un acide nucléique correspondant audit gène avec une matrice compétitive pour ledit gène ;
mesurer un acide nucléique correspondant audit premier facteur de transcription par rapport à une matrice compétitive pour ledit premier facteur de transcription ; et
obtenir une relation comparant ledit facteur de transcription à ladite matrice compétitive ; et
(a) dans lequel ledit état biologique est un carcinome bronchogénique ou le risque de celui-ci et ledit premier facteur de transcription est CEBPG (protéine de liaison gamma à un amplificateur/CCAAT), et où ledit gène est XRCC1 (réparation de rayons X complétant la réparation défectueuse dans des cellules de hamster chinois 1), CAT (catalase), ERCC1 (réparation par excision pour une complémentation croisée de la déficience en réparation d'un rongeur, groupe de complémentation 1), ERCC2 (réparation par excision pour une complémentation croisée de la déficience en réparation d'un rongeur, groupe de complémentation 2), ERCC5 (réparation par excision pour une complémentation croisée de la déficience en réparation d'un rongeur, groupe de complémentation 5), GSTZ1 (glutathion transférase zêta 1), mGST1 (glutathion S-transférase 1 microsomique), GSTP1 (glutathion S-transférase pi), SOD1 (superoxyde dismutase 1) ou GPX1 (glutathion peroxydase 1) ; ou
(b) dans lequel ledit état biologique est un carcinome bronchogénique ou le risque de celui-ci et ledit premier facteur de transcription est E2F1, et où ledit gène est ERCC5, GSTP1 ou SOD1 ; ou
(c) dans lequel ledit état biologique est une BPCO ou le risque de celle-ci et ledit premier facteur de transcription est CEBPG, et où ledit gène est XRCC1, ERCC1, ERCC2, CAT, ERCC5, GSTZ1, mGST1, GSTP1, SOD1 ou GPX1 ; ou
(d) dans lequel l'état biologique est une BPCO ou le risque de celle-ci et ledit premier facteur de transcription est E2F1, et où ledit gène est ERCC5, GSTP1 ou SOD1.

2. Procédé selon la revendication 1, comprenant en outre le dosage des taux d'expression d'un ou plusieurs facteurs de transcription supplémentaires associés audit état biologique.

3. Procédé selon la revendication 2, dans lequel ledit gène est régulé par ledit premier facteur de transcription et par lesdits un ou plusieurs facteurs de transcription supplémentaires dans lesdits échantillons contrôles.

4. Procédé selon l'une quelconque des revendications 1 à 3, dans lequel lesdites matrices compétitives sont fournies dans un mélange normalisé.

5. Procédé selon l'une quelconque des revendications 1 à 4, dans lequel le dosage de ladite abondance de transcrit d'ARNm comprend les étapes consistant à :
coamplifier un acide nucléique correspondant audit gène avec une matrice compétitive pour ledit gène ;
coamplifier un acide nucléique correspondant audit premier facteur de transcription avec une matrice compétitive pour ledit premier facteur de transcription ; et
obtenir une relation comparant les produits amplifiés obtenus à partir desdites coamplifications.

6. Procédé selon l'une quelconque des revendications 1 à 5, dans lequel
(i) plus de 10 échantillons de cas et plus de 10 échantillons contrôles sont utilisés, ou
(ii) plus de 30 échantillons de cas et plus de 30 échantillons contrôles sont utilisés, ou
(iii) plus de 50 échantillons de cas et plus de 50 échantillons contrôles sont utilisés, ou
(iv) plus de 100 échantillons de cas et plus de 100 échantillons contrôles sont utilisés.

7. Procédé selon l'une quelconque des revendications 1 à 6, comprenant en outre l'étape consistant à établir une décision diagnostic basée sur l'identification dudit biomarqueur.
